# EUROPEAN PATENT APPLICATION

(11) **EP 3 550 021 A1**
(43) Date of publication of application: **09.10.2019**
(21) Application number: 18165917.8
(22) Date of filing: 05.04.2018
(51) Int. Cl.: C12N 15/113, C12N 15/11

(54) **PRODUCTS AND COMPOSITIONS FOR INHIBITING EXPRESSION OF A TARGET GENE**

(71) Applicant: Silence Therapeutics GmbH, 13125 Berlin (DE)
(72) Inventor: The designation of the inventor has not yet been filed
(74) Representative: CMS Cameron McKenna Nabarro Olswang LLP

(57) **Abstract**

The present invention relates to products and compositions and their uses. In particular the invention relates to nucleic acid products that interfere with gene expression or inhibits its expression and therapeutic uses such as for the treatment of disease and disorders.

## Description

The present invention relates to products and compositions and their uses. In particular the invention relates to nucleic acid products that interfere with gene expression or inhibits its expression and therapeutic uses such as for the treatment of diseases and disorders.

### Background

Double-stranded RNA (dsRNA) has been shown to block gene expression (Fire *et al*, 1998 and Elbashir *et al*, 2001) and this has been termed RNA interference (RNAi). Short dsRNAs direct gene-specific, post-transcriptional silencing in many organisms, including vertebrates, and has provided a new tool for studying gene function. RNAi is mediated by RNA-induced silencing complex (RISC), a sequence-specific, multi-component nuclease that destroys messenger RNAs homologous to the silencing trigger. Interfering RNA (iRNA) such as nucleic acid, antisense RNA, and micro-RNA are oligonucleotides that prevent the formation of proteins by gene-silencing i.e. inhibiting gene translation of the protein. Gene-silencing agents are becoming increasingly important for therapeutic applications in medicine.

However, delivery of nucleic acids, such as RNA, to cells avoiding degradation by cellular nucleases, whilst maintaining efficacy and target specificity has proved challenging to those in the field of developing nucleic acid molecules for therapeutic use.

According to Watts and Corey in the Journal of Pathology (2012; Vol 226, p 365-379) there are algorithms that can be used to design nucleic acid but none is perfect. It may take various experimental methods to identify potent nucleic acid, as algorithms do not take into account factors such as tertiary structure or the involvement of RNA binding proteins. Therefore the discovery of a potent nucleic acid with minimal off-target effects is a complex process but necessary for the pharmaceutical development of these highly charged molecules to be synthesised economically, distributed to target tissues, enter cells and function within acceptable limits of toxicity. Thus, means for efficient delivery of oligonucleotides, in particular double stranded siRNAs, to cells in vivo is becoming increasingly important and requires specific targeting and substantial protection from the extracellular environment, particularly serum proteins. One method of achieving specific targeting is to conjugate a targeting moiety to the iRNA duplex agent. The targeting moiety helps in targeting the iRNA duplex agent to the required target site and there is a need to design appropriate targeting moieties for the desired receptor sites for the conjugated molecules to be taken up by the cells such as by endocytosis.

However, targeting ligands developed so far do not always translate to in vivo setting and there is a clear need for more efficacous receptor specific ligand conjugated iRNA duplex agents and methods for their preparation for the in vivo delivery of oligonucleotide therapeutics, nucleic acids and double stranded siRNAs.

Rather than a lipid delivery system alone, the present invention addresses the structure of the nucleic acid itself.

Accordingly, the present invention provides a nucleic acid for inhibiting expression of a target gene in a cell, comprising at least one duplex region that comprises at least a portion of a first strand and at least a portion of a second strand that is at least partially complementary to the first strand, wherein said first strand is at least partially complementary to at least a portion of a RNA transcribed from said target gene, wherein said first strand includes a modified nucleotides or non-modified nucleotides at a plurality of positions in order to facilitate processing of the nucleic acid by RISC.

The present invention also relates to a nucleic acid capable of inhibiting the expression of a target gene, the nucleic acid comprising a first strand and a second strand, wherein the first stand and second strand are at least partially complementary with one another, wherein said first strand is at least partially complementary to RNA transcribed from a portion of said target gene; and wherein said first strand and/or said second strand include modified nucleotides.

The nucleotides at positions 2 and 14 from the 5' end of the first strand may be modified.

The nucleotides at positions 2 and 14 from the 5' end of the first strand may not be modified with a 2' O-methyl modification.

The nucleotides at positions 2 and 14 from the 5' end of the first strand may not be modified with a modification selected from the group consisting of 2'-O-(2-Methoxyethyl), 2'-O-allyl, 2'-O-DNP, 2'-CE, 2'-EA, 2'-AEM, 2'-APM and 2'-GE.

The nucleotides at positions 2 and 14 from the 5' end of the first strand may be modified with a modification selected from the group consisting of 2'F, 4'-S, 2'-FANA and UNA.

The nucleotides at positions 2 and 14 from the 5' end of the first strand may be unmodified.

The nucleotides at position 2 and 14 from the 5' end of the second strand may be modified with a 2' O-methyl modification or with a '-O-(2-Methoxyethyl) modification.

The first strand and the second strand may be separate strands

The nucleic acid may comprise a single strand that comprises the first strand and the second strand.

The first strand and/or said second strand may each be from 17-35 nucleotides in length and the at least one duplex region may be from 10-25 nucleotides in length. The duplex may comprise two separate strands or it may comprise a single strand which comprises the first strand and the second strand.

In one aspect the second strand may be as short as 11 nucleotides in length such as 11, 12, 13, 14, 15, 16, 17, 18 , 19 nucleotides or more.

The nucleic acid may: a) be blunt ended at both ends; b) have an overhang at one end and a blunt end at the other; or c) have an overhang at both ends.

One or more nucleotides on the first and/or second strand may be modified, to form modified nucleotides. One or more of the odd numbered nucleotides of the first strand may be modified. One or more of the even numbered nucleotides of the first strand may be modified by at least a second modification, wherein the at least second modification is different from the modification on the one or more add nucleotides. At least one of the one or more modified even numbered nucleotides may be adjacent to at least one of the one or more modified odd numbered nucleotides.
A plurality of odd numbered nucleotides in the first strand may be modified in the nucleic acid of the invention. A plurality of even numbered nucleotides in the first strand may be modified by a second modification. The first strand may comprise adjacent nucleotides that are modified by a common modification. The first strand may also comprise adjacent nucleotides that are modified by a second different modification.

One or more of the odd numbered nucleotides of the second strand may be modified by a modification that is different to the modification of the odd numbered nucleotides on the first strand and/or one or more of the even numbered nucleotides of the second strand may be by the same modification of the odd numbered nucleotides of the first strand. At least one of the one or more modified even numbered nucleotides of the second strand may be adjacent to the one or more modified odd numbered nucleotides. A plurality of odd numbered nucleotides of the second strand may be modified by a common modification and/or a plurality of even numbered nucleotides may be modified by the same modification that is present on the first strand odd numbered nucleotides. A plurality of odd numbered nucleotides on the second strand may be modified by a second modification, wherein the second modification is different from the modification of the first strand odd numbered nucleotides.

The second strand comprises adjacent nucleotides that are modified by a common modification, which may be a second modification that is different from the modification of the odd numbered nucleotides of the first strand.

In the nucleic acid of the invention, each of the odd numbered nucleotides in the first strand and each of the even numbered nucleotides in the second strand may be modified with a common modification and, each of the even numbered nucleotides may be modified in the first strand with a second modification and each of the odd numbered nucleotides may be modified in the second strand with a second different modification.

The nucleic acid of the invention may have the modified nucleotides of the first strand shifted by at least one nucleotide relative to the unmodified or differently modified nucleotides of the second strand.

The modification and / or modifications may each and individually be selected from the group consisting of 3'-terminal deoxy-thymine, 2'-O-methyl, a 2'-deoxy-modification, a 2'-amino-modification, a 2'-alkyl-modification, a morpholino modification, a phosphoramidate modification, 5'-phosphorothioate group modification, a 5' phosphate or 5' phosphate mimic modification and a cholesteryl derivative or a dodecanoic acid bisdecylamide group modification and/or the modified nucleotide may be any one of a locked nucleotide, an abasic nucleotide or a non-natural base comprising nucleotide.

At least one modification may be 2'-O-methyl and/or at least one modification may be 2'-F.

A nucleic acid of the invention may comprise a phosphorothioate linkage between the terminal one, two or three 3' nucleotides and/or one two or three 5' nucleotides of the first and/or the second strand. It may comprise two phosphorothioate linkages between each of the three terminal 3' and between each of the three terminal 5' nucleotides on the first strand, and two phosphorothioate linkages between the three terminal nucleotides of the 3' end of the second strand.

Such a nucleic acid may be conjugated to a ligand.

The ligand may comprise a serinol-derived linker moiety.

The present invention relates to a conjugate for inhibiting expression of a target gene in a cell, said conjugate comprising a nucleic acid portion and ligand portions, said nucleic acid portion comprising at least one duplex region that comprises at least a portion of a first RNA strand and at least a portion of a second RNA strand that is at least partially complementary to the first strand, wherein said first strand is at least partially complementary to at least a portion of RNA transcribed from said target gene, said ligand portions comprising a serinol-derived linker moiety and a targeting ligand for in vivo targeting of cells and being conjugated exclusively to the 3' and/or 5' ends of one or both RNA strands, wherein the 5' end of the first RNA strand is not conjugated, wherein:
(i) the second RNA strand is conjugated at the 5' end to the ligand, and wherein (a) the second RNA strand is also conjugated at the 3' end to the ligand and the 3' end of the first RNA strand is not conjugated; or (b) the first RNA strand is conjugated at the 3' end to the ligand and the 3' end of the second RNA strand is not conjugated; or (c) both the second RNA strand and the first RNA strand are also conjugated at the 3' ends; or
(ii) both the second RNA strand and the first RNA strand are conjugated at the 3' ends and the 5' end of the second RNA strand is not conjugated;
(iii) and whererin the first strand of the nucleic acid includes modified nucleotides at a plurality of positions, and wherein the nucleotides at positions 2 and 14 from the 5' end of the first strand are not modified with a 2' O-methyl modification.

The invention further provides a nucleic acid for inhibiting expression of a target gene in a cell, comprising at least one duplex region that comprises at least a portion of a first strand and at least a portion of a second strand that is at least partially complementary to the first strand, wherein said first strand is at least partially complementary to at least a portion of a RNA transcribed from said target gene wherein said first strand includes modified nucleotides or unmodified nucleotides at a plurality of positions in order to facilitate processing of the nucleic acid by RISC, and wherein the nucleotide sequence is conjugated to a ligand.

The ligand may comprise (i) one or more N-acetyl galactosamine (GalNac) moieties and derivatives thereof, and (ii) a linker, wherein the linker conjugates the GalNac moieties to a sequence as defined in any preceding aspects. The linker may be a bivalent or trivalent or tetravalent branched structure. The nucleotides may be modified as defined herein.

The ligand may comprise the formula I:

[S-X¹-p-X²]₃-A-X³- (I)

wherein:
S represents a saccharide, wherein the saccharide is N-acetyl galactosamine;
X¹ represents C₃-C₆ alkylene or (-CH₂-CH₂-O)ₘ(-CH₂)₂- wherein m is 1, 2, or 3;
P is a phosphate or modified phosphate (preferably a thiophosphate);
X² is alkylene or an alkylene ether of the formula (-CH₂)ₙ-O-CH₂- where n = 1- 6;
A is a branching unit;
X³ represents a bridging unit;
wherein a nucleic acid according to the present invention is conjugated to X³ via a phosphate or modified phosphate (preferably a thiophosphate).

The present invention therefore additionally provides a conjugated nucleic acid having one of the following structures 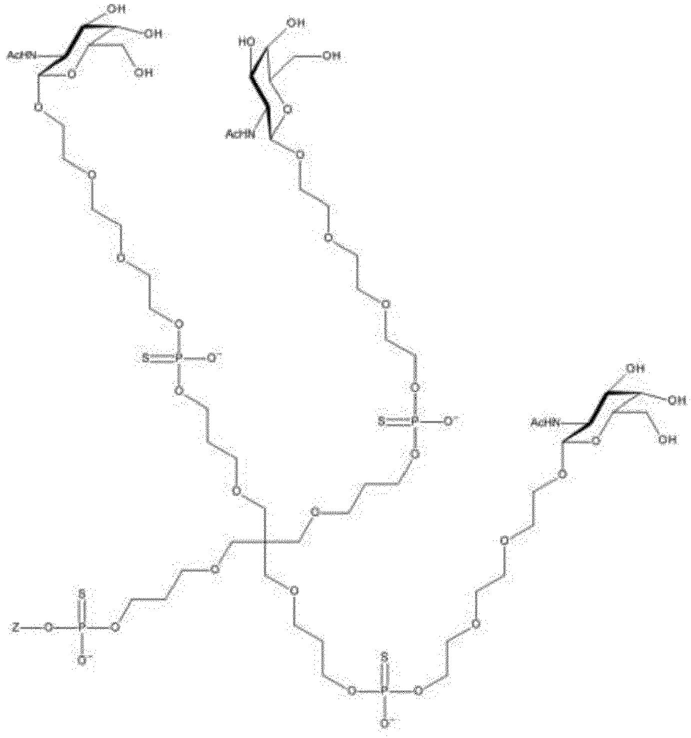 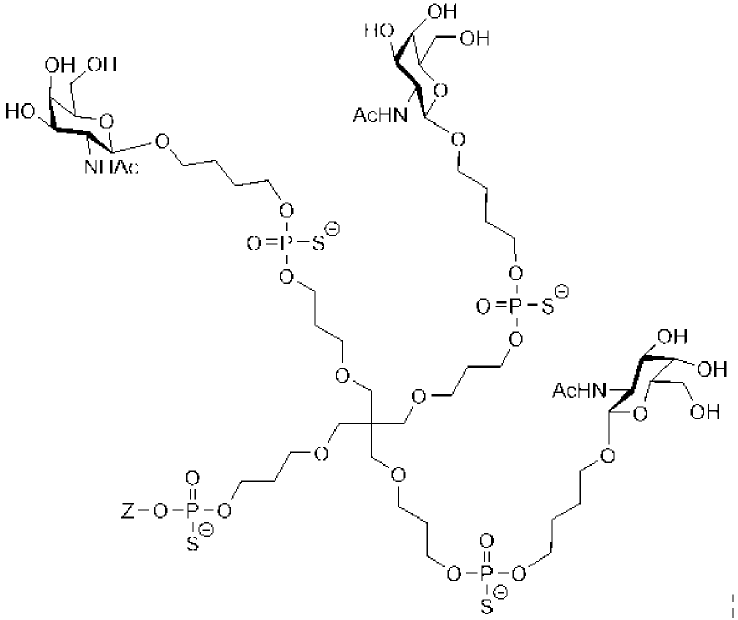 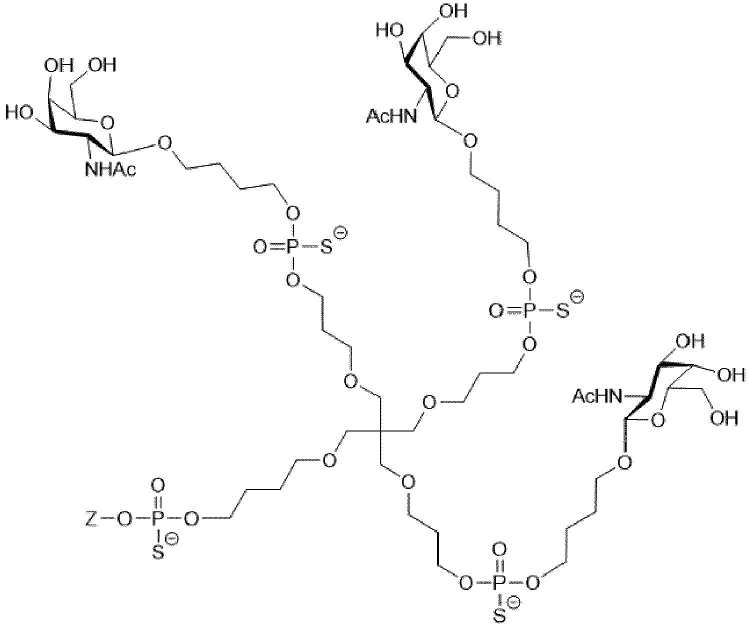 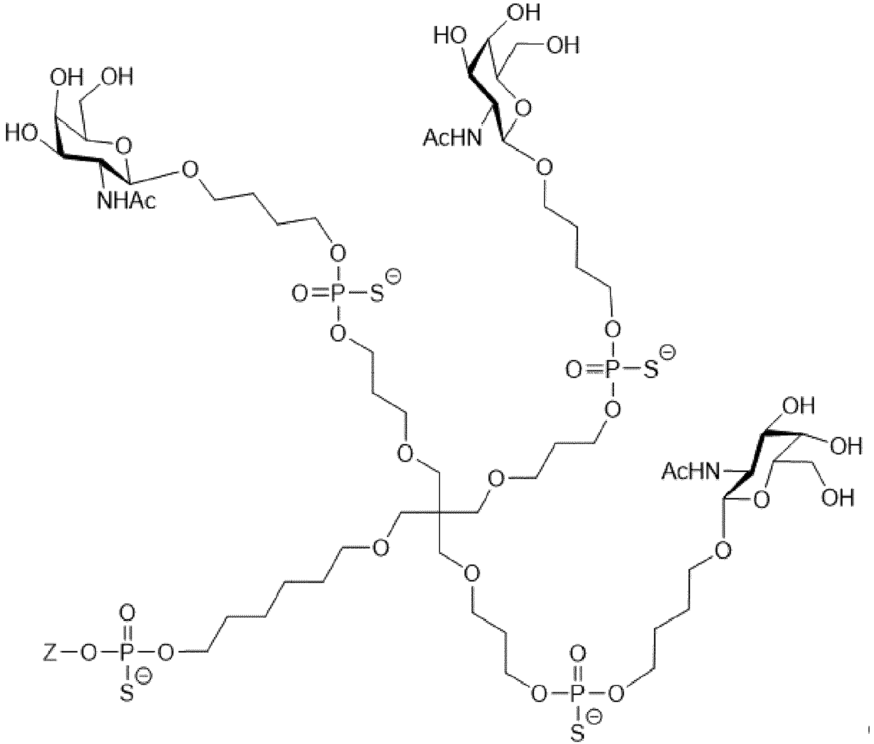 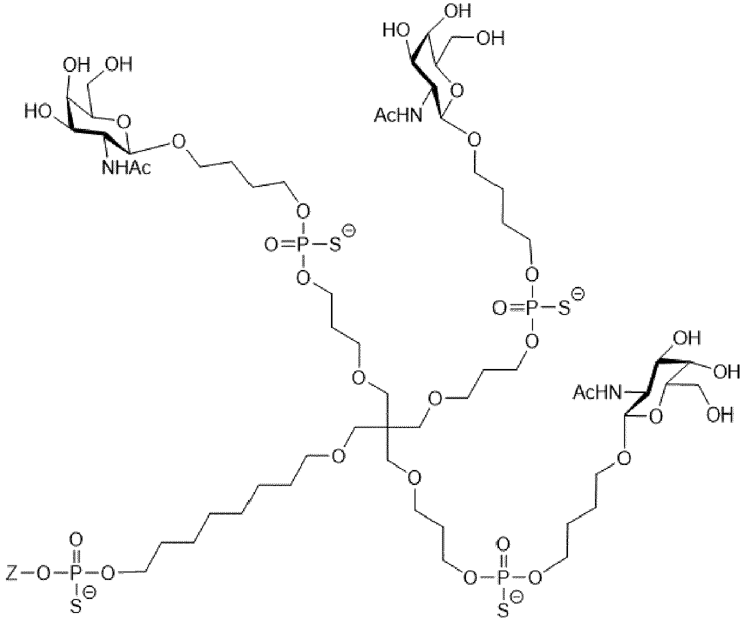 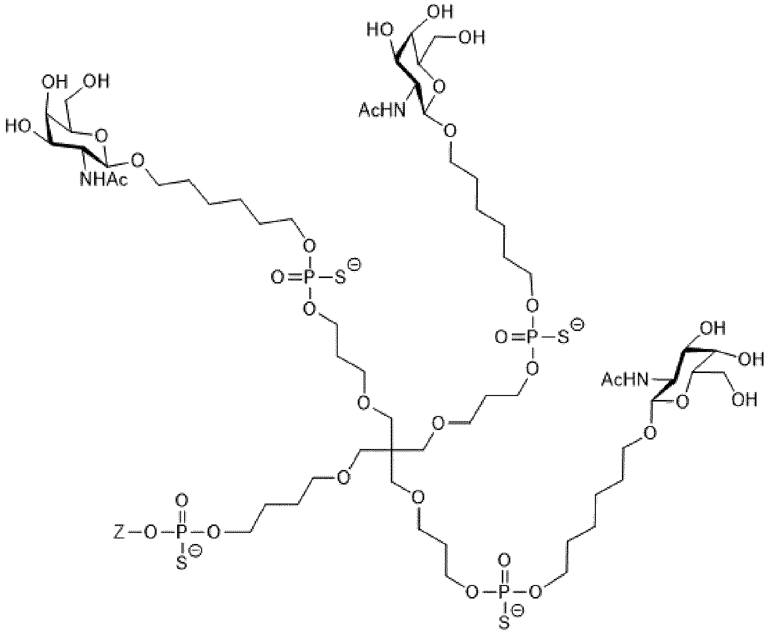 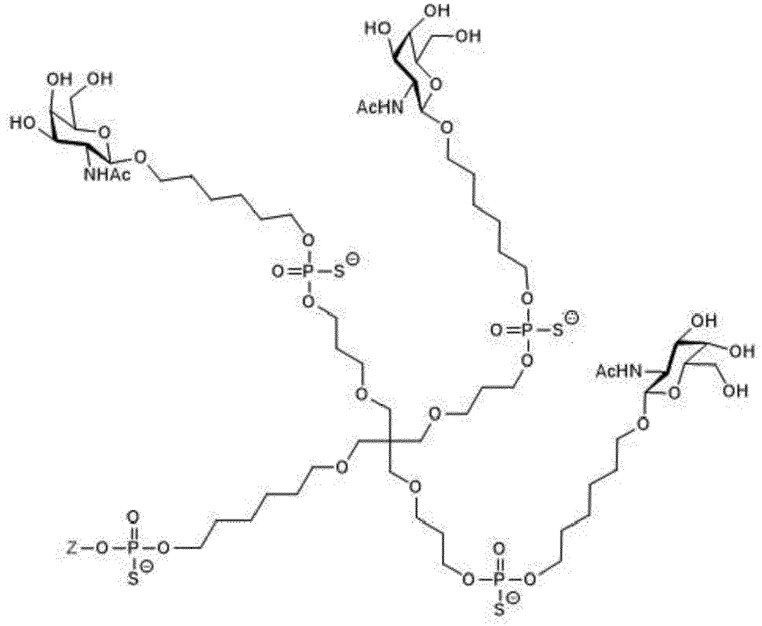 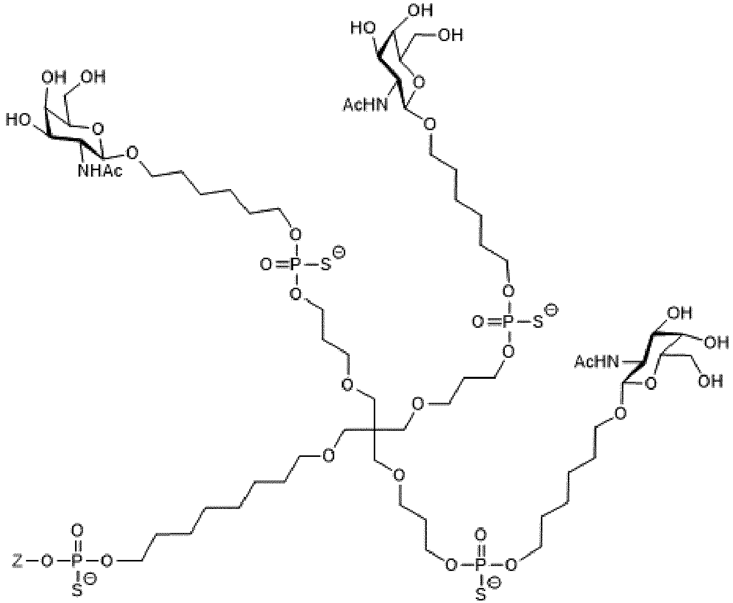 wherein Z represents a nucleic acid as defined herein before.

The ligand may comprise

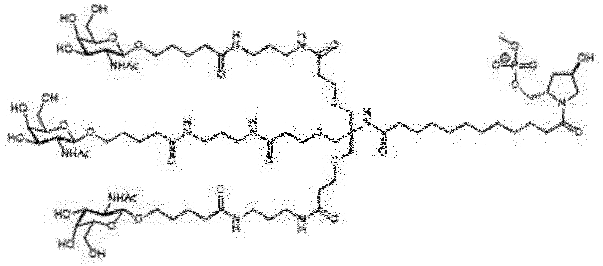

The invention also provides a composition comprising a nucleic acid or conjugated nucleic acid as defined herein and a physiologically acceptable excipient. The composition can comprise the following excipients:
i) a cationic lipid, or a pharmaceutically acceptable salt thereof;
ii) a steroid;
iii) a phosphatidylethanolamine phospholipid;
iv) a PEGylated lipid.

The content of the cationic lipid component in the composition may be from about 55 mol% to about 65 mol% of the overall lipid content of the lipid formulation, preferably about 59 mol% of the overall lipid content of the lipid composition.

The composition may comprise a cationic lipid having the structure 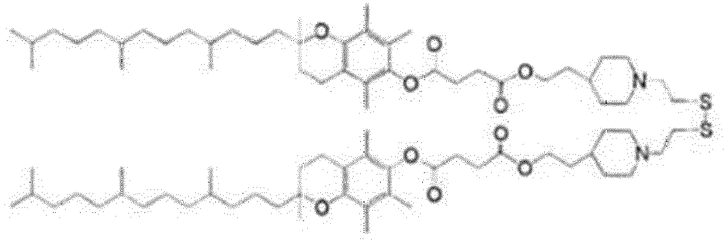 a steroid having the structure 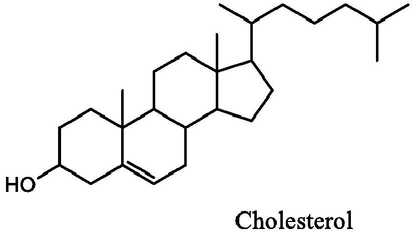 a phosphatidylethanolamine phospholipid having the structure

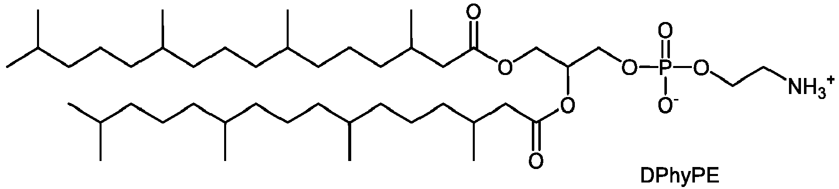

And a PEGylated lipid having the structure

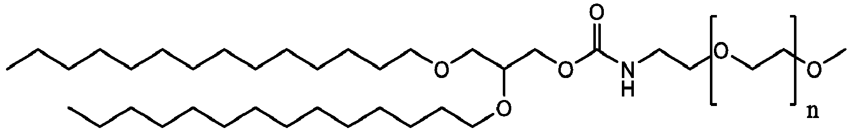

Also provided is a nucleic acid or conjugated nucleic acid according to any aspect of the invention for use in the treatment of a disease or disorder and/or in the manufacture of a medicament for treating a disease or disorder.

The invention provides a method of treating a disease or disorder comprising administration of a composition comprising a nucleic acid or conjugated nucleic acid according to any aspect of the invention to an individual in need of treatment. The nucleic acid may be administered to the subject subcutaneously, intravenously or using any other application routes such as oral, rectal or intraperitoneal.

A method of making a nucleic acid or conjugated nucleic acid according to the invention is also included.

### Detailed Description of Invention

The present invention relates to a nucleic acid which is double stranded and directed to an expressed RNA transcript of a target gene and compositions thereof. These nucleic acids can be used in the treatment of a variety of diseases and disorders where reduced expression of the target gene product is desirable.

A first aspect of the invention relates to an nucleic acid for inhibiting expression of a target gene in a cell, comprising at least one duplex region that comprises at least a portion of a first strand and at least a portion of a second strand that is at least partially complementary to the first strand, wherein said first strand is at least partially complementary to at least a portion of a RNA transcribed from the target gene, wherein said first strand comprises a modified nucleotide at a selected position in order to facilitate processing of the nucleic acid by RISC. The first strand may comprise an unmodified nucleotide.

Another aspect of the invention relates to a nucleic acid capable of inhibiting the expression of a target gene, the nucleic acid comprising a first strand and a second strand, wherein the first stand and second strand are at least partially complementary with one another, wherein said first strand is at least partially complementary to RNA transcribed from a portion of said target gene; and wherein said first strand and/or said second strand include modified nucleotides.

By nucleic acid it is meant a nucleic acid comprising two strands comprising nucleotides, that is able to interfere with gene expression. Inhibition may be complete or partial and results in down regulation of gene expression in a targeted manner. The nucleic acid comprises two separate polynucleotide strands; the first strand, which may also be a guide strand; and a second strand, which may also be a passenger strand. The first strand and the second strand may be part of the same polynucleotide strand that is self complementary which 'folds' to form a double stranded molecule. The nucleic acid may be an siRNA molecule.

The nucleic acid may comprise ribonucleotides, modified ribonucleotides, deoxynucleotides, deoxyribonucleotides, or nucleotide analogous. The nucleic acid may further comprise a double-stranded nucleic acid portion or duplex region formed by all or a portion of the first strand (also known in the art as a guide strand) and all or a portion of the second strand (also known in the art as a passenger strand). The duplex region is defined as beginning with the first base pair formed between the first strand and the second strand and ending with the last base pair formed between the first strand and the second strand, inclusive.

By duplex region refers it is meant the region in two complementary or substantially complementary oligonucleotides that form base pairs with one another, either by Watson-Crick base pairing or any other manner that allows for a duplex between oligonucleotide strands that are complementary or substantially complementary. For example, an oligonucleotide strand having 21 nucleotide units can base pair with another oligonucleotide of 21 nucleotide units, yet only 19 nucleotides on each strand are complementary or substantially complementary, such that the "duplex region" consists of 19 base pairs. The remaining base pairs may exist as 5' and 3' overhangs, or as single stranded regions. Further, within the duplex region, 100% complementarity is not required; substantial complementarity is allowable within a duplex region. Substantial complementarity refers to complementarity between the strands such that they are capable of annealing under biological conditions. Techniques to empirically determine if two strands are capable of annealing under biological conditions are well known in the art. Alternatively, two strands can be synthesised and added together under biological conditions to determine if they anneal to one another.

The portion of the first strand and second strand that form at least one duplex region may be fully complementary and are at least partially complementary to each other.

Depending on the length of an nucleic acid, a perfect match in terms of base complementarity between the first strand and second strand is not necessarily required. However, the first and second strands must be able to hybridise under physiological conditions.

The complementarity between the first strand and second strand in the at least one duplex region may be perfect in that there are no nucleotide mismatches or additional/deleted nucleotides in either strand. Alternatively, the complementarity may not be perfect. The complementarity may be at least 70%, 75%, 80%, 85%, 90% or 95%.

The first strand and the second strand may each comprise a region of complementarity which comprises at least 15 contiguous nucleotides.

The nucleic acid may comprise a second sequence comprising a nucleotide sequence of SEQ ID NO:2, 4, 6, 8, 10, 12, 14, 16, 18, 20, 22, 24, 26, 28 or 30.

The nucleic acid involves the formation of a duplex region between all or a portion of the first strand and a portion of the target nucleic acid. The portion of the target nucleic acid that forms a duplex region with the first strand, defined as beginning with the first base pair formed between the first strand and the target sequence and ending with the last base pair formed between the first strand and the target sequence, inclusive, is the target nucleic acid sequence or simply, target sequence. The duplex region formed between the first strand and the second strand need not be the same as the duplex region formed between the first strand and the target sequence. That is, the second strand may have a sequence different from the target sequence however, the first strand must be able to form a duplex structure with both the second strand and the target sequence.

The complementarity between the first strand and the target sequence may be perfect (no nucleotide mismatches or additional/deleted nucleotides in either nucleic acid).

The complementarity between the first strand and the target sequence may not be perfect. The complementarity may be at least 70%, 80%, 85%, 90% or 95%.

The identity between the first strand and the complementary sequence of the target sequence may be at least 75%, 80%, 85%, 90% or 95%, provided an nucleic acid is capable of reducing or inhibiting the expression of the target gene.

The nucleic acid may be able to reduce expression of the target gene by at least 25%, 50% or 75% of a comparative nucleic acid with perfect identity to the first strand and target sequence.

The nucleic acid may comprise a first strand and a second strand that are each from 17-35 or 19-25 nucleotides in length. The first strand and the second strand may be of different lengths.

The nucleic acid may be 15-25 nucleotide pairs in length. The nucleic acid may be 17-23 nucleotide pairs in length. The nucleic acid may be 17-25 nucleotide pairs in length. The nucleic acid may be 23-24 nucleotide pairs in length. The nucleic acid may be 19-21 nucleotide pairs in length. The nucleic acid may be 21-23 nucleotide pairs in length.

The nucleic acid may comprise a duplex region that consists of 19-25 nucleotide base pairs. The duplex region may consist of 17, 18, 19, 20, 21, 22, 23, 24 or 25 base pairs which may be contiguous.

The nucleic acid may be blunt ended at both ends; have an overhang at one end and a blunt end at the other end; or have an overhang at both ends.

An "overhang" as used herein has its normal and customary meaning in the art, i.e. a single stranded portion of a nucleic acid that extends beyond the terminal nucleotide of a complementary strand in a double strand nucleic acid. The term "blunt end" includes double stranded nucleic acid whereby both strands terminate at the same position, regardless of whether the terminal nucleotide(s) are base paired. The terminal nucleotide of a first strand and a second strand at a blunt end may be base paired. The terminal nucleotide of a first strand and a second strand at a blunt end may not be paired. The terminal two nucleotides of an first strand and a second strand at a blunt end may be base paired. The terminal two nucleotides of an first strand and a second strand at a blunt end may not be paired.

The nucleic acid may have an overhang at one end and a blunt end at the other. The nucleic acid may have an overhang at both ends. The nucleic acid may be blunt ended at both ends. The nucleic acid may be blunt ended at the end with the 5'-end of the first strand and the 3'-end of the second strand or at the 3'-end of the first strand and the 5'-end of the second strand.

The nucleic acid may comprise an overhang at a 3'- or 5'-end. The nucleic acid may have a 3'-overhang on the first strand. The nucleic acid may have a 3'-overhang on the second strand. The nucleic acid may have a 5'-overhang on the first strand. The nucleic acid may have a 5'-overhang on the second strand. The nucleic acid may have an overhang at both the 5'-end and 3'-end of the first strand. The nucleic acid may have an overhang at both the 5'-end and 3'-end of the second strand. The nucleic acid may have a 5' overhang on the first strand and a 3' overhang on the second strand. The nucleic acid may have a 3' overhang on the first strand and a 5' overhang on the second strand. The nucleic acid may have a 3' overhang on the first strand and a 3' overhang on the second strand. The nucleic acid may have a 5' overhang on the first strand and a 5' overhang on the second strand.

An overhang at the 3'-end or 5' end of the second strand or the first strand may be selected from consisting of 1, 2, 3, 4 and 5 nucleotides in length. Optionally, an overhang may consist of 1 or 2 nucleotides, which may or may not be modified.

Unmodified polynucleotides, particularly ribonucleotides, may be prone to degradation by cellular nucleases, and, as such, modification/ modified nucleotides may be included in the nucleic acid of the invention.

One or more nucleotides on the second and/or first strand of the nucleic acid of the invention may be modified.

Modifications of the nucleic acid of the present invention generally provide a powerful tool in overcoming potential limitations including, but not limited to, in vitro and in vivo stability and bioavailability inherent to native RNA molecules. The nucleic acid according to the invention may be modified by chemical modifications. Modified nucleic acid can also minimise the possibility of inducing interferon activity in humans. Modification can further enhance the functional delivery of a nucleic acid to a target cell. The modified nucleic acid of the present invention may comprise one or more chemically modified ribonucleotides of either or both of the first strand or the second strand. A ribonucleotide may comprise a chemical modification of the base, sugar or phosphate moieties. The ribonucleic acid may be modified by substitution or insertion with analogues of nucleic acids or bases.

One or more nucleotides of a nucleic acid of the present invention may be modified. The nucleic acid may comprise at least one modified nucleotide. The modified nucleotide may be on the first strand. The modified nucleotide may be in the second strand. The modified nucleotide may be in the duplex region. The modified nucleotide may be outside the duplex region, i.e., in a single stranded region. The modified nucleotide may be on the first strand and may be outside the duplex region. The modified nucleotide may be on the second strand and may be outside the duplex region. The 3'-terminal nucleotide of the first strand may be a modified nucleotide. The 3'-terminal nucleotide of the second strand may be a modified nucleotide. The 5'-terminal nucleotide of the first strand may be a modified nucleotide. The 5'-terminal nucleotide of the second strand may be a modified nucleotide.

An nucleic acid of the invention may have 1 modified nucleotide or a nucleic acid of the invention may have about 2-4 modified nucleotides, or a nucleic acid may have about 4-6 modified nucleotides, about 6-8 modified nucleotides, about 8-10 modified nucleotides, about 10-12 modified nucleotides, about 12-14 modified nucleotides, about 14-16 modified nucleotides about 16-18 modified nucleotides, about 18-20 modified nucleotides, about 20-22 modified nucleotides, about 22-24 modified nucleotides, 24-26 modified nucleotides or about 26-28 modified nucleotides. In each case the nucleic acid comprising said modified nucleotides retains at least 50% of its activity as compared to the same nucleic acid but without said modified nucleotides. The nucleic acid may retain 55%, 60%, 65%, 70%, 75%, 80%, 85%, 90%, 95% or 100% or above of its activity as compared to the same nucleic acid but without said modified nucleotides

The modified nucleotide may be a purine or a pyrimidine. At least half of the purines may be modified. At least half of the pyrimidines may be modified. All of the purines may be modified. All of the pyrimidines may be modified. The modified nucleotides may be selected from the group consisting of a 3'-terminal deoxy-thymine (dT) nucleotide, a 2'-O-methyl modified nucleotide, a 2' modified nucleotide, a 2'-deoxy-modified nucleotide, a locked nucleotide, an abasic nucleotide, a 2'-amino-modified nucleotide, a 2'-alkyl-modified nucleotide, a morpholino nucleotide, a phosphoramidate, a non-natural base comprising nucleotide, a nucleotide comprising a 5'-phosphorothioate group, a nucleotide comprising a 5' phosphate or 5' phosphate mimic and a terminal nucleotide linked to a cholesteryl derivative or a dodecanoic acid bisdecylamide group.

The nucleic acid may comprise a nucleotide comprising a modified nucleotide, wherein the base is selected from 2-aminoadenosine, 2,6-diaminopurine,inosine, pyridin-4-one, pyridin-2-one, phenyl, pseudouracil, 2, 4, 6-trimethoxy benzene, 3-methyl uracil, dihydrouridine, naphthyl, aminophenyl, 5-alkylcytidine (e.g., 5-methylcytidine), 5-alkyluridine (e.g., ribothymidine), 5-halouridine (e.g., 5-bromouridine), 6-azapyrimidine, 6-alkylpyrimidine (e.g. 6-methyluridine), propyne, quesosine, 2-thiouridine, 4-thiouridine, wybutosine, wybutoxosine, 4-acetylcytidine, 5-(carboxyhydroxymethyl)uridine, 5'-carboxymethylaminomethyl-2-thiouridine, 5-carboxymethylaminomethyluridine, beta-D-galactosylqueosine, 1-methyladenosine, 1-methylinosine, 2,2-dimethylguanosine, 3-methylcytidine, 2-methyladenosine, 2-methylguanosine, N6-methyladenosine, 7-methylguanosine, 5-methoxyaminomethyl-2-thiouridine, 5-methylaminomethyluridine, 5-methylcarbonylmethyluridine, 5-methyloxyuridine, 5-methyl-2-thiouridine, 2-methylthio-N6-isopentenyladenosine, beta-D-mannosylqueosine, uridine-5-oxyacetic acid and 2-thiocytidine.

PNucleic acids discussed herein include unmodified RNA as well as RNA which have been modified, e.g., to improve efficacy, and polymers of nucleoside surrogates. Unmodified RNA refers to a molecule in which the components of the nucleic acid, namely sugars, bases, and phosphate moieties, are the same or essentially the same as that which occur in nature, for example as occur naturally in the human body. Modified nucleotide as used herein refers to a nucleotide in which one or more of the components of the nucleic acid, namely sugars, bases, and phosphate moieties, are different from that which occur in nature. While they are referred to as modified nucleotides they will of course, because of the modification, include molecules which are not nucleotides, for example a polynucleotide molecules in which the ribophosphate backbone is replaced with a non-ribophosphate construct that allows hybridisation between strands i.e. the modified nucleotides mimic the ribophosphate backbone.

Many of the modifications described below that occur within a nucleic acid will be repeated within a polynucleotide molecule, such as a modification of a base, or a phosphate moiety, or the a non-linking O of a phosphate moiety. In some cases the modification will occur at all of the possible positions/nucleotides in the polynucleotide but in many cases it will not. A modification may only occur at a 3' or 5' terminal position, may only occur in a terminal regions, such as at a position on a terminal nucleotide or in the last 2, 3, 4, 5, or 10 nucleotides of a strand. A modification may occur in a double strand region, a single strand region, or in both. A modification may occur only in the double strand region of an nucleic acid of the invention or may only occur in a single strand region of an nucleic acid of the invention. A phosphorothioate modification at a non-linking O position may only occur at one or both termini, may only occur in a terminal region, e.g., at a position on a terminal nucleotide or in the last 2, 3, 4 or 5 nucleotides of a strand, or may occur in duplex and/or in single strand regions, particularly at termini. The 5' end or 3' ends may be phosphorylated.

Stability of an nucleic acid of the invention may be increased by including particular bases in overhangs, or to include modified nucleotides, in single strand overhangs, e.g., in a 5' or 3' overhang, or in both. Purine nucleotides may be included in overhangs. All or some of the bases in a 3' or 5' overhang may be modified. Modifications can include the use of modifications at the 2' OH group of the ribose sugar, the use of deoxyribonucleotides, instead of ribonucleotides, and modifications in the phosphate group, such as phosphothioate modifications. Overhangs need not be homologous with the target sequence.

The 5'- or 3'- overhangs at the first strand, second strand or both strands of the dsRNA agent of the invention may be phosphorylated. In some embodiments, the overhang region contains two nucleotides having a phosphorothioate between the two nucleotides, where the two nucleotides can be the same or different. In one embodiment, the overhang is present at the 3 '-end of the first strand, second strand or both strands. In one embodiment, this 3 '-overhang is present in the first strand. In one embodiment, this 3 '-overhang is present in the second strand.

Nucleases can hydrolyze nucleic acid phosphodiester bonds. However, chemical modifications to nucleic acids can confer improved properties, and, can render oligoribonucleotides more stable to nucleases.

Modified nucleic acids, as used herein, can include one or more of:
(i) alteration, e.g., replacement, of one or both of the non-linking phosphate oxygens and/or of one or more of the linking phosphate oxygens (referred to as linking even if at the 5' and 3' terminus of the nucleic acid of the invention);
(ii) alteration, e.g., replacement, of a constituent of the ribose sugar, e.g., of the 2' hydroxyl on the ribose sugar;
(iii) replacement of the phosphate moiety with "dephospho" linkers;
(iv) modification or replacement of a naturally occurring base;
(v) replacement or modification of the ribose-phosphate backbone;
(vi) modification of the 3' end or 5' end of the RNA, e.g., removal, modification or replacement of a terminal phosphate group or conjugation of a moiety, e.g., a fluorescently labeled moiety, to either the 3' or 5' end of RNA.

The terms replacement, modification, alteration, indicates a difference from a naturally occurring molecule.

Specific modifications are discussed in more detail below.

Examples of modified phosphate groups include phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulphur. One, each or both non-linking oxygens in the phosphate group can be independently any one of S, Se, B, C, H, N, or OR (R is alkyl or aryl).

The phosphate linker can also be modified by replacement of a linking oxygen with nitrogen (bridged phosphoroamidates), sulfur (bridged phosphorothioates) and carbon (bridged methylenephosphonates). The replacement can occur at a terminal oxygen. Replacement of the non-linking oxygens with nitrogen is possible.

A modified nucleotide can include modification of the sugar groups. The 2' hydroxyl group (OH) can be modified or replaced with a number of different "oxy" or "deoxy" substituents.

Examples of "oxy"-2' hydroxyl group modifications include alkoxy or aryloxy (OR, e.g., R=H, alkyl, cycloalkyl, aryl, aralkyl, heteroaryl or sugar); polyethyleneglycols (PEG), O(CH2CH2O)nCH2CH2OR; "locked" nucleic acids (LNA) in which the 2' hydroxyl is connected, e.g., by a methylene bridge, to the 4' carbon of the same ribose sugar; O-AMINE (AMINE=NH2; alkylamino, dialkylamino, heterocyclyl, arylamino, diaryl amino, heteroaryl amino, or diheteroaryl amino, ethylene diamine, polyamino) and aminoalkoxy, O(CH2)nAMINE, (e.g., AMINE=NH2; alkylamino, dialkylamino, heterocyclyl, arylamino, diaryl amino, heteroaryl amino, or diheteroaryl amino, ethylene diamine, polyamino).

"Deoxy" modifications include hydrogen halo; amino (e.g., NH2; alkylamino, dialkylamino, heterocyclyl, arylamino, diaryl amino, heteroaryl amino, diheteroaryl amino, or amino acid); NH(CH2CH2NH)nCH2CH2-AMINE (AMINE=NH2; alkylamino, dialkylamino, heterocyclyl, arylamino, diaryl amino, heteroaryl amino, or diheteroaryl amino), -NHC(O)R (R=alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with e.g., an amino functionality. Other substitutents of certain embodiments include 2'-methoxyethyl, 2'-OCH3, 2'-O-allyl, 2'-C-allyl, and 2'-fluoro.

The sugar group can also contain one or more carbons that possess the opposite stereochemical configuration than that of the corresponding carbon in ribose. Thus, a modified nucleotides may contain a sugar such as arabinose.

Modified nucleotides can also include "abasic" sugars, which lack a nucleobase at C-I'. These abasic sugars can further contain modifications at one or more of the constituent sugar atoms.

The 2' modifications may be used in combination with one or more phosphate linker modifications (e.g., phosphorothioate).

The phosphate group can be replaced by non-phosphorus containing connectors.

Examples of moieties which can replace the phosphate group include siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino. In certain embodiments, replacements may include the methylenecarbonylamino and methylenemethylimino groups.

The phosphate linker and ribose sugar may be replaced by nuclease resistant nucleotides.

Examples include the mophilino, cyclobutyl, pyrrolidine and peptide nucleic acid (PNA) nucleoside surrogates. In certain embodiments, PNA surrogates may be used.

The 3' and 5' ends of an oligonucleotide can be modified. Such modifications can be at the 3' end or the 5' end or both ends of the molecule. They can include modification or replacement of an entire terminal phosphate or of one or more of the atoms of the phosphate group. For example, the 3' and 5' ends of an oligonucleotide can be conjugated to other functional molecular entities such as labeling moieties, e.g., fluorophores (e.g., pyrene, TAMRA, fluorescein, Cy3 or Cy5 dyes) or protecting groups (based e.g., on sulfur, silicon, boron or ester). The functional molecular entities can be attached to the sugar through a phosphate group and/or a linker. The terminal atom of the linker can connect to or replace the linking atom of the phosphate group or the C-3' or C-5' O, N, S or C group of the sugar. Alternatively, the linker can connect to or replace the terminal atom of a nucleotide surrogate (e.g., PNAs). These spacers or linkers can include e.g., -(CH₂)ₙ₋, -(CH₂)ₙN-, -(CH₂)ₙO-, - (CH₂)ₙS-, O(CH₂CH₂O)nCH₂CH₂OH (e.g., n=3 or 6), abasic sugars, amide, carboxy, amine, oxyamine, oxyimine, thioether, disulfide, thiourea, sulfonamide, or morpholino, or biotin and fluorescein reagents. The 3' end can be an -OH group.

Other examples of terminal modifications include dyes, intercalating agents (e.g., acridines), cross-linkers (e.g., psoralene, mitomycin C), porphyrins (TPPC4, texaphyrin, Sapphyrin), polycyclic aromatic hydrocarbons (e.g., phenazine, dihydrophenazine), artificial endonucleases (e.g., EDTA), lipophilic carriers (e.g., cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine) and peptide conjugates (e.g., antennapedia peptide, Tat peptide), alkylating agents, phosphate, amino, mercapto, PEG (e.g., PEG-40K), MPEG, [MPEG]2, polyamino, alkyl, substituted alkyl, radiolabeled markers, enzymes, haptens (e.g., biotin), transport/absorption facilitators (e.g., aspirin, vitamin E, folic acid), synthetic ribonucleases (e.g., imidazole, bisimidazole, histamine, imidazole clusters, acridine-imidazole conjugates, Eu³⁺ complexes of tetraazamacrocycles).

Terminal modifications can be added for a number of reasons, including to modulate activity or to modulate resistance to degradation. Terminal modifications useful for modulating activity include modification of the 5' end with phosphate or phosphate analogs. Nucleic acids of the invention, on the first or second strand, may be 5' phosphorylated or include a phosphoryl analog at the 5' prime terminus. 5'-phosphate modifications include those which are compatible with RISC mediated gene silencing. Suitable modifications include: 5'-monophosphate ((HO)₂(O)P-O-5'); 5'-diphosphate ((HO)₂(O)P-O-P(HO)(O)-O-5'); 5'-triphosphate ((HO)₂(O)P-O-(HO)(O)P-O-P(HO)(O)-O-5'); 5'-guanosine cap (7-methylated or non-methylated) (7m-G-O-5'-(HO)(O)P-O-(HO)(O)P-O-P-(HO)(O)-O-5'); 5'-adenosine cap (Appp), and any modified or unmodified nucleotide cap structure (N-O-5'-(HO)(O)P-O-(HO)(O)P-O-P(HO)(O)-O-5'); 5'-monothiophosphate (phosphorothioate; (HO)₂(S)P-O-5'); 5'-monodithiophosphate (phosphorodithioate; (HO)(HS)(S)P-O-5'), 5'-phosphorothiolate ((HO)₂(O)P-S-5'); any additional combination of oxygen/sulfur replaced monophosphate, diphosphate and triphosphates (e.g., 5'-alpha-thiotriphosphate, 5'-gamma-thiotriphosphate, etc.), 5'-phosphoramidates ((HO)₂(O)P-NH-5', (HO)(NH2)(O)P-O-5'), 5'-alkylphosphonates (R=alkyl=methyl, ethyl, isopropyl, propyl, etc., e.g., RP(OH)(O)-O-5'-, (OH)₂(O)P-5'-CH₂-), 5'vinylphosphonate, 5'-alkyletherphosphonates (R=alkylether=methoxymethyl (MeOCH₂-), ethoxymethyl, etc., e.g., RP(OH)(O)-O-5'-).

The nucleic acid of the present invention may include one or more phosphorothioate modifications on one or more of the terminal ends of the first and/or the second strand. Optionally, each or either end of the first strand may comprise one or two or three phosphorothioate modified nucleotides. Optionally, each or either end of the second strand may comprise one or two or three phosphorothioate modified nucleotides. Optionally, both ends of the first strand and the 5' end of the second strand may comprise two phosphorothioate modified nucleotides. By phosphorothioate modified nucleotide it is meant that the linkage between the nucleotide and the adjacent nucleotide comprises a phosphorothioate group instead of a standard phosphate group.

Terminal modifications can also be useful for monitoring distribution, and in such cases the groups to be added may include fluorophores, e.g., fluorscein or an Alexa dye. Terminal modifications can also be useful for enhancing uptake, useful modifications for this include cholesterol. Terminal modifications can also be useful for cross-linking an RNA agent to another moiety.

Adenine, guanine, cytosine and uracil are the most common bases found in RNA. These bases can be modified or replaced to provide RNA's having improved properties. E.g., nuclease resistant oligoribonucleotides can be prepared with these bases or with synthetic and natural nucleobases (e.g., inosine, thymine, xanthine, hypoxanthine, nubularine, isoguanisine, or tubercidine) and any one of the above modifications. Alternatively, substituted or modified analogs of any of the above bases and "universal bases" can be employed. Examples include 2-aminoadenine, 6-methyl and other alkyl derivatives of adenine and guanine, 2-propyl and other alkyl derivatives of adenine and guanine, 5-halouracil and cytosine, 5-propynyl uracil and cytosine, 6-azo uracil, cytosine and thymine, 5-uracil (pseudouracil), 4-thiouracil, 5-halouracil, 5-(2-aminopropyl)uracil, 5-amino allyl uracil, 8-halo, amino, thiol, thioalkyl, hydroxyl and other 8-substituted adenines and guanines, 5-trifluoromethyl and other 5-substituted uracils and cytosines, 7-methylguanine, 5-substituted pyrimidines, 6-azapyrimidines and N-2, N-6 and O-6 substituted purines, including 2-aminopropyladenine, 5-propynyluracil and 5-propynylcytosine, dihydrouracil, 3-deaza-5-azacytosine, 2-aminopurine, 5-alkyluracil, 7-alkylguanine, 5-alkyl cytosine, 7-deazaadenine, N6,N6-dimethyladenine, 2,6-diaminopurine, 5-amino-allyl-uracil, N3-methyluracil, substituted 1,2,4-triazoles, 2-pyridinone, 5-nitroindole, 3-nitropyrrole, 5-methoxyuracil, uracil-5-oxyacetic acid, 5-methoxycarbonylmethyluracil, 5-methyl-2-thiouracil, 5-methoxycarbonylmethyl-2-thiouracil, 5-methylaminomethyl-2-thiouracil, 3-(3-amino-3-carboxypropyl)uracil, 3-methylcytosine, 5-methylcytosine, N<4>-acetyl cytosine, 2-thiocytosine, N6-methyladenine, N6-isopentyladenine, 2-methylthio-N6-isopentenyladenine, N-methylguanines, or O-alkylated bases.

As used herein, the terms "non-pairing nucleotide analog" means a nucleotide analog which includes a non-base pairing moiety including but not limited to: 6 des amino adenosine (Nebularine), 4-Me-indole, 3-nitropyrrole, 5-nitroindole, Ds, Pa, N3-Me ribo U, N3-Me riboT, N3-Me dC, N3-Me-dT, N1-Me-dG, N1-Me-dA, N3-ethyl-dC, N3-Me dC. In some embodiments the non-base pairing nucleotide analog is a ribonucleotide. In other embodiments it is a deoxyribonucleotide.

As used herein, the term, "terminal functional group" includes without limitation a halogen, alcohol, amine, carboxylic, ester, amide, aldehyde, ketone, ether groups.

Certain moieties may be linked to the 5' terminus of the first strand or the second strand and includes abasic ribose moiety, abasic deoxyribose moiety, modifications abasic ribose and abasic deoxyribose moieties including 2' O alkyl modifications; inverted abasic ribose and abasic deoxyribose moieties and modifications thereof, C6-imino-Pi; a mirror nucleotide including L-DNA and L-RNA; 5'OMe nucleotide; and nucleotide analogs including 4',5'-methylene nucleotide; 1-(β-D-erythrofuranosyl)nucleotide; 4'-thio nucleotide, carbocyclic nucleotide; 5'-amino-alkyl phosphate; 1,3-diamino-2-propyl phosphate, 3-aminopropyl phosphate; 6-aminohexyl phosphate; 12-aminododecyl phosphate; hydroxypropyl phosphate; 1,5-anhydrohexitol nucleotide; alpha-nucleotide; threo-pentofuranosyl nucleotide; acyclic 3',4'-seco nucleotide; 3,4-dihydroxybutyl nucleotide; 3,5-dihydroxypentyl nucleotide, 5'-5'-inverted abasic moiety; 1,4-butanediol phosphate; 5'-amino; and bridging or non bridging methylphosphonate and 5'-mercapto moieties.

The nucleic acids of the invention may be included one or more inverted nucleotides, for example inverted thymidine or inverted adenine (for example see Takei, et al., 2002. JBC 277 (26):23800-06).

As used herein, the term "inhibit", "down-regulate", or "reduce" with respect to gene expression means the expression of the gene, or level of RNA molecules or equivalent RNA molecules encoding one or more proteins or protein subunits (e.g., mRNA), or activity of one or more proteins or protein subunits, is reduced below that observed in the absence of a nucleic acid of the invention; for example the expression may be reduced to 90%, 80%, 70%, 60%, 50%, 40%, 30%, 20%, 15% or less than that observed in the absence of an inhibitor.

The nucleic acid of the present invention may comprise an abasic nucleotide. The term "abasic" as used herein, refers to moieties lacking a base or having other chemical groups in place of a base at the 1' position, for example a 3',3'-linked or 5',5'-linked deoxyabasic ribose derivative.

The nucleic acid may comprise one or more nucleotides on the second and/or first strands that are modified. Alternating nucleotides may be modified, to form modified nucleotides.

Alternating as described herein means to occur one after another in a regular way. In other words, alternating means to occur in turn repeatedly. For example if one nucleotide is modified, the next contiguous nucleotide is not modified and the following contiguous nucleotide is modified and so on. One nucleotide may be modified with a first modification, the next contiguous nucleotide may be modified with a second modification and the following contiguous nucleotide is modified with the first modification and so on, where the first and second modifications are different.

One or more of the odd numbered nucleotides of the first strand of the nucleic acid of the invention may be modified wherein the first strand is numbered 5' to 3'. The term "odd numbered" as described herein means a number not divisible by two. Examples of odd numbers are 1, 3, 5, 7, 9, 11 and so on. One or more of the even numbered nucleotides of the first strand of the nucleic acid of the invention may be modified, wherein the first strand is numbered 5' to 3'. The term "even numbered" as described herein means a number which is evenly divisible by two. Examples of even numbers are 2, 4, 6, 8, 10, 12, 14 and so on. One or more of the odd numbered nucleotides of the second strand of the nucleic acid of the invention may be modified wherein the second strand is numbered 3' to 5'. One or more of the even numbered nucleotides of the second strand of the nucleic acid of the invention may be modified, wherein the second strand is numbered 3' to 5'.

One or more nucleotides on the first and/or second strand may be modified, to form modified nucleotides. One or more of the odd numbered nucleotides of the first strand may be modified. One or more of the even numbered nucleotides of the first strand may be modified by at least a second modification, wherein the at least second modification is different from the modification on the one or more add nucleotides. At least one of the one or more modified even numbered nucleotides may be adjacent to at least one of the one or more modified odd numbered nucleotides.

A plurality of odd numbered nucleotides in the first strand may be modified in the nucleic acid of the invention. A plurality of even numbered nucleotides in the first strand may be modified by a second modification. The first strand may comprise adjacent nucleotides that are modified by a common modification. The first strand may also comprise adjacent nucleotides that are modified by a second different modification.

One or more of the odd numbered nucleotides of the second strand may be modified by a modification that is different to the modification of the odd numbered nucleotides on the first strand and/or one or more of the even numbered nucleotides of the second strand may be by the same modification of the odd numbered nucleotides of the first strand. At least one of the one or more modified even numbered nucleotides of the second strand may be adjacent to the one or more modified odd numbered nucleotides. A plurality of odd numbered nucleotides of the second strand may be modified by a common modification and/or a plurality of even numbered nucleotides may be modified by the same modification that is present on the first stand odd numbered nucleotides. A plurality of odd numbered nucleotides on the second strand may be modified by a second modification, wherein the second modification is different from the modification of the first strand odd numbered nucleotides.

The second strand may comprise adjacent nucleotides that are modified by a common modification, which may be a second modification that is different from the modification of the odd numbered nucleotides of the first strand.

In the nucleic acid of the invention, each of the odd numbered nucleotides in the first strand and each of the even numbered nucleotides in the second strand may be modified with a common modification and, each of the even numbered nucleotides may be modified in the first strand with a second modification and each of the odd numbered nucleotides may be modified in the second strand with the second modification.

The nucleic acid of the invention may have the modified nucleotides of the first strand shifted by at least one nucleotide relative to the unmodified or differently modified nucleotides of the second strand.

One or more or each of the odd numbered nucleotides may be modified in the first strand and one or more or each of the even numbered nucleotides may be modified in the second strand. One or more or each of the alternating nucleotides on either or both strands may be modified by a second modification. One or more or each of the even numbered nucleotides may be modified in the first strand and one or more or each of the even numbered nucleotides may be modified in the second strand. One or more or each of the alternating nucleotides on either or both strands may be modified by a second modification. One or more or each of the odd numbered nucleotides may be modified in the first strand and one or more of the odd numbered nucleotides may be modified in the second strand by a common modification. One or more or each of the alternating nucleotides on either or both strands may be modified by a second modification. One or more or each of the even numbered nucleotides may be modified in the first strand and one or more or each of the odd numbered nucleotides may be modified in the second strand by a common modification. One or more or each of the alternating nucleotides on either or both strands may be modified by a second modification.

The nucleic acid of the invention may comprise single or double stranded constructs that comprise at least two regions of alternating modifications in one or both of the strands. These alternating regions can comprise up to about 12 nucleotides but preferably comprise from about 3 to about 10 nucleotides. The regions of alternating nucleotides may be located at the termini of one or both strands of the nucleic acid of the invention. The nucleic acid may comprise from 4 to about 10 nucleotides of alternating nucleotides at each termini (3' and 5') and these regions may be separated by from about 5 to about 12 contiguous unmodified or differently or commonly modified nucleotides.

The odd numbered nucleotides of the first strand may be modified and the even numbered nucleotides may be modified with a second modification. The second strand may comprise adjacent nucleotides that are modified with a common modification, which may be the same as the modification of the odd numbered nucleotides of the first strand. One or more nucleotides of second strand may also be modified with the second modification. One or more nucleotides with the second modification may be adjacent to each other and to nucleotides having a modification that is the same as the modification of the odd numbered nucleotides of the first strand. The first strand may also comprise phosphorothioate linkages between the two nucleotides at the 3' end and at the 5' end. The second strand may comprise a phosphorothioate linkage between the two nucleotides at 5' end. The second strand may also be conjugated to a ligand at the 5' end.

The nucleic acid of the invention may comprise a first strand comprising adjacent nucleotides that are modified with a common modification. One or more of such nucleotides may be adjacent to one or more nucleotides which may be modified with a second modification. One or more nucleotides with the second modification may be adjacent. The second strand may comprise adjacent nucleotides that are modified with a common modification, which may be the same as one of the modifications of one or more nucleotides of the first strand. One or more nucleotides of second strand may also be modified with the second modification. One or more nucleotides with the second modification may be adjacent. The first strand may also comprise phosphorothioate linkages between the two nucleotides at the 5' end and at the 3' end. The second strand may comprise a phosphorothioate linkage between the two nucleotides at 3' end. The second strand may also be conjugated to a ligand at the 5' end.

The nucleotides for the purposes of modification as described herein (unless otherwise indicated) are numbered from 5' to 3' on the first strand and 3' and 5' on the second strand. Nucleotides numbered 1, 3, 5, 7, 9, 11, 13, 15, 17, 19, 21, 23 and 25 may be modified by a modification on the first strand. The nucleotides numbered 2, 4, 6, 8, 10, 12, 14, 16, 18, 20, 22 and 24 may be modified by a second modification on the first strand. The nucleotides numbered 1, 3, 5, 7, 9, 11, 13, 15, 17, 19, 21, 23 may be modified by a modification on the second strand. The nucleotides numbered 2, 4, 6, 8, 10, 12, 14, 16, 18, 20, 22 and 24 may be modified by a second modification on the second strand. Nucleotides are numbered for the sake of the nucleic acid of the present invention from 5' to 3' on the first strand and 3' and 5' on the second strand, unless otherwise indicated.

The nucleotides numbered 2, 4, 6, 8, 10, 12, 14, 16, 18, 20, 22 and 24 may be modified on the first strand. The nucleotides numbered 1, 3, 5, 7, 9, 11, 13, 15, 17, 19, 21, 23 may be modified by a second modification on the first strand. The nucleotides numbered 1, 3, 5, 7, 9, 11, 13, 15, 17, 19, 21, 23 may be modified by a modification on the second strand. The nucleotides numbered 2, 4, 6, 8, 10, 12, 14, 16, 18, 20, 22 and 24 may be modified by a second modification on the second strand.

Clearly, if the first and/or the second strand are shorter or longer than 25 nucleotides in length, such as 19 nucleotides in length, there are no nucleotides numbered 20, 21, 22, 23, 24 and 25 to be modified. The skilled person understands the description above to apply to shorter or longer strands, accordingly.

One or more modified nucleotides on the first strand may be paired with modified nucleotides on the second strand having a common modification. One or more modified nucleotides on the first strand may be paired with modified nucleotides on the second strand having a different modification. One or more modified nucleotides on the first strand may be paired with unmodified nucleotides on the second strand. One or more modified nucleotides on the second strand may be paired with unmodified nucleotides on the first strand. In other words, the alternating nucleotides can be aligned on the two strands such as, for example, all the modifications in the alternating regions of the second strand are paired with identical modifications in the first strand or alternatively the modifications can be offset by one nucleotide with the common modifications in the alternating regions of one strand pairing with dissimilar modifications (i.e. a second or further modification) in the other strand. Another option is to have dissimilar modifications in each of the strands.

The modifications on the first strand may be shifted by one nucleotide relative to the modified nucleotides on the second strand, such that common modified nucleotides are not paired with each other.

The modification and/or modifications may each and individually be selected from the group consisting of 3'-terminal deoxy-thymine, 2'-O-methyl, a 2'-deoxy-modification, a 2'-amino-modification, a 2'-alkyl-modification, a morpholino modification, a phosphoramidate modification, 5'-phosphorothioate group modification, a 5' phosphate or 5' phosphate mimic modification and a cholesteryl derivative or a dodecanoic acid bisdecylamide group modification and/or the modified nucleotide may be any one of a locked nucleotide, an abasic nucleotide or a non-natural base comprising nucleotide.
At least one modification may be 2'-O-methyl and/or at least one modification may be 2'-F. Further modifications as described herein may be present on the first and/or second strand.

Throughout the description of the invention, "same or common modification" means the same modification to any nucleotide, be that A, G, C or U modified with a group such as such as a methyl group or a fluoro group. Is it not taken to mean the same addition on the same nucleotide. For example, 2'F-dU, 2'F-dA, 2'F-dC, 2'F-dG are all considered to be the same or common modification, as are 2'-OMe-rU, 2'-OMe-rA; 2'-OMe-rC; 2'-OMe-rG. A 2'F modification is a different modification to a 2'OMe modification.

Some representative modified nucleic acid sequences of the present invention are shown in the examples. These examples are meant to be representative and not limiting.

Preferably, the nucleic acid may comprise a modification and the second or further modification which are each and individually selected from the group comprising 2'-O-methyl modification and 2'-F modification. The nucleic acid may comprise a modification that is 2'-O-methyl (2'OMe) that may be a first modification, and a second modification that is 2'-F. The nucleic acid of the invention may also include a phosphorothioate modification and/or a deoxy modification which may be present in or between the terminal 1, 2 or 3 nucleotides of each or any end of each or both strands.

The nucleic acid of the invention may be conjugated to a ligand.

For example, the Asialoglycoprotein receptor (ASGP-R) is a high capacity receptor, which is highly abundant on hepatocytes. One of the first disclosures of triantennary cluster glycosides was in US patent number US 5,885,968. Conjugates having three GalNAc ligands and comprising phosphate groups are known and are described in Dubber et al. (2003). The ASGP-R shows a 50-fold higher affinity for N-Acetyl-D-Galactosylamine (GalNAc) than D-Gal.

Hepatocytes expressing the lectin (asialoglycoprotein receptor; ASGPR), which recognizes specifically terminal β-galactosyl subunits of glycosylated proteins or other oligosaccharides (P. H. Weigel et. al., 2002,) can be used for targeting a drug to the liver by covalent coupling of galactose or galactoseamine to the drug substance (S.Ishibashi, et. al. 1994). Furthermore the binding affinity can be significantly increased by the multi-valency effect, which is achieved by the repetition of the targeting unit (E. A. L. Biessen et. al., 1995).

The ASGPR is a mediator for an active endosomal transport of terminal β-galactosyl containing glycoproteins, thus ASGPR is highly suitable for targeted delivery of drug candidates like nucleic acid, which have to be delivered into a cell (Akinc et al.).

The saccharide, which can also be referred to as the ligand, may be selected to have an affinity for at least one type of receptor on a target cell. In particular, the receptor is on the surface of a mammalian liver cell, for example, the hepatic asialoglycoprotein receptor (ASGP-R).

The saccharide may be selected from N-acetyl galactoseamine, mannose, galactose, glucose, glucosamone and fucose. The saccharide may be N-acetyl galactoseamine (GalNAc).

"GalNAc" refers to 2-(Acetylamino)-2-deoxy-D- galactopyranose, commonly referred to in the literature as N-acetyl galactosamine. Reference to "GalNAc" or "N-acetyl galactoseamine" includes both the β- form: 2-(Acetylamino)-2-deoxy-β -D-galactopyranose and the α-form: 2-(Acetylamino)-2-deoxy-α-D-galactopyranose. Both the β-form: 2-(Acetylarnino)-2-deoxy-β-D-galactopyranose and α-form: 2-(Acetylamino)-2-deoxy-α-D-galactopyranose may be used interchangeably. Preferably, the compounds of the invention comprise the β-form, 2-(Acetylarnino)-2-deoxy-β-D-galactopyranose.

The ligand may comprise GalNAc.

The ligand may comprise a compound of formula I:

[S-X¹-P-X²]₃-A-X³- (I)

wherein:
S represents a saccharide, wherein the saccharide is N-acetyl galactosamine;
X¹ represents C₃-C₆ alkylene or (-CH₂-CH₂-O)ₘ(-CH₂)₂- wherein m is 1, 2, or 3;
P is a phosphate or modified phosphate (preferably a thiophosphate);
X² is alkylene or an alkylene ether of the formula (-CH₂)ₙ-O-CH₂- where n = 1- 6;
A is a branching unit;
X³ represents a bridging unit;
wherein a nucleic acid according to the present invention is conjugated to X³ via a phosphate or modified phosphate (preferably a thiophosphate).

In formula I, branching unit "A" branches into three in order to accommodate the three saccharide ligands. The branching unit is covalently attached to the ligands and the nucleic acid. The branching unit may comprise a branched aliphatic group comprising groups selected from alkyl, amide, disulphide, polyethylene glycol, ether, thioether and hydroxyamino groups. The branching unit may comprise groups selected from alkyl and ether groups.

The branching unit A may have a structure selected from: 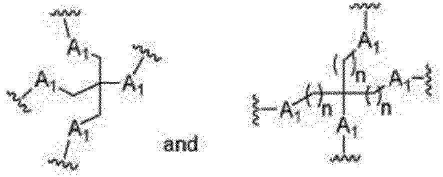 wherein each A₁ independently represents O, S, C=O or NH; and
each n independently represents an integer from 1 to 20.

The branching unit may have a structure selected from: 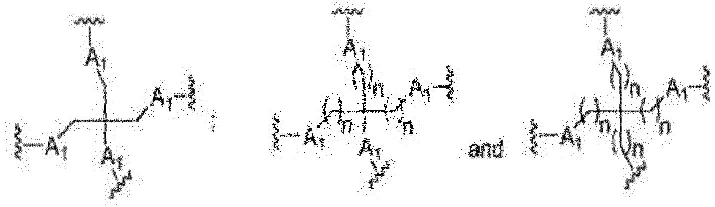 wherein each A₁ independently represents O, S, C=O or NH; and
each n independently represents an integer from 1 to 20.

The branching unit may have a structure selected from: 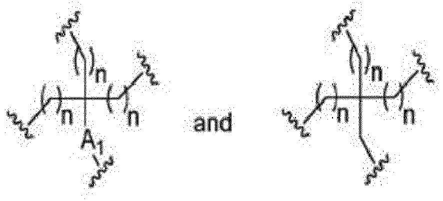 wherein A₁ is O, S, C=O or NH; and
each n independently represents an integer from 1 to 20.

The branching unit may have the structure:

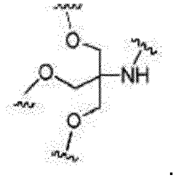

The branching unit may have the structure:

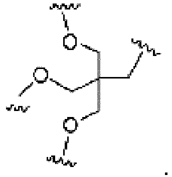

The branching unit may have the structure:

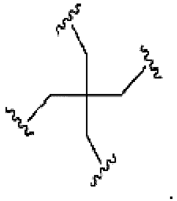

Optionally, the branching unit consists of only a carbon atom.

X³ may be selected from -C₁-C₂₀ alkylene-, -C₂-C₂₀ alkenylene-, an alkylene ether of formula - (C₁-C₂₀ alkylene)-O-(C₁-C₂₀ alkylene)-, -C(O)-C₁-C₂₀ alkylene-, -C₀-C₄ alkylene(Cy)C₀-C₄ alkylene- wherein Cy represents a substituted or unsubstituted 5 or 6 membered cycloalkylene, arylene, heterocyclylene or heteroarylene ring, -C₁-C₄ alkylene-NHC(O)-CrC₄ alkylene-, -C₁-C₄ alkylene-C(O)NH-C₁-C₄ alkylene-, -C₁-C₄ alkylene-SC(O)-C₁C₄ alkylene-,-C₁-C₄ alkylene-C(O)S-C₁-C₄ alkylene-, -C₁-C₄ alkylene-OC(O)-C₁-C₄ alkylene-, -C₁-C₄ alkylene-C(O)O-C₁-C₄ alkylene-, and -C₁-C₆ alkylene-S-S-C₁-C₆ alkylene-.

X³ may be an alkylene ether of formula -(C₁-C₂₀ alkylene)-O-(C₁-C₂₀ alkylene)-. X³ may be an alkylene ether of formula -(C₁-C₂₀ alkylene)-O-(C₄-C₂₀ alkylene)-, wherein said (C₄-C₂₀ alkylene) is linked to Z. X³ may be selected from the group consisting of -CH₂-O-C₃H₆-, -CH₂-O-C₄H₈-, -CH₂-O-C₆H₁₂- and -CH₂-O-C₈H₁₆, especially -CH₂-O-C₄H₆-, -CH₂-O-C₆H₁₂- and - CH₂-O-C₈H₁₆-, wherein in each case the -CH₂- group is linked to A.

The ligand may comprise a compound of formula (II):

[S-X¹-P-X²]₃-A-X³- (II)

wherein:
S represents a saccharide;
X¹ represents C₃-C₆ alkylene or (-CH₂-CH₂-O)ₘ(-CH₂)₂- wherein m is 1, 2, or 3;
P is a phosphate or modified phosphate (preferably a thiophosphate);
X² is C₁-C₈ alkylene;
A is a branching unit selected from: 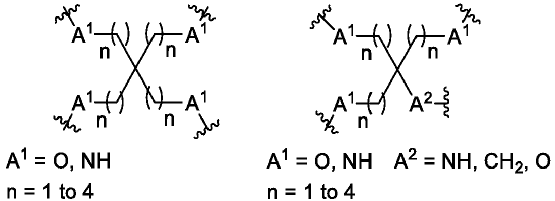
X³ is a bridging unit;
wherein a nucleic acid according to the present invention is conjugated to X³ via a phosphate or modified phosphate (preferably a thiophosphate).

Branching unit A may have the structure:

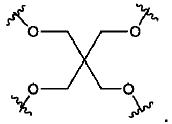

Branching unit A may have the structure: 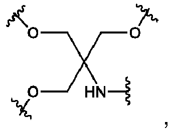 wherein X³ is attached to the nitrogen atom.

X³ may be C₁-C₂₀ alkylene. Preferably, X³ is selected from the group consisting of -C₃H₆-, - C₄H₈-,-C₆H₁₂- and -C₈H₁₆-, especially -C₄H₈-, -C₆H₁₂- and -C₈H₁₆-.

The ligand may comprise a compound of formula (III):

[S-X¹-P-X²]₃-A-X³- (III)

wherein:
S represents a saccharide;
X¹ represents C₃-C₆ alkylene or (-CH₂-CH₂-O)ₘ(-CH₂)₂- wherein m is 1, 2, or 3;
P is a phosphate or modified phosphate (preferably a thiophosphate);
X² is an alkylene ether of formula -C₃H₆-O-CH₂-;
A is a branching unit;
X³ is an alkylene ether of formula selected from the group consisting of -CH₂-O-CH₂-, - CH₂-O-C₂H₄-, -CH₂-O-C₃H₆-, -CH₂-O-C₄H₈-, -CH₂-O-C₅H₁₀-, -CH₂-O-C₆H₁₂-, -CH₂-O-C₇H₁₄-, and -CH₂-O-C₈H₁₆-, wherein in each case the -CH₂- group is linked to A,
wherein a nucleic acid according to the present invention is conjugated to X³ via a phosphate or modified phosphate (preferably a thiophosphate).

The branching unit may comprise carbon. Preferably, the carbon unit is carbon.

X³ may be selected from the group consisting of -CH₂-O-C₄H₈-, -CH₂-O-C₅H₁₀-, -CH₂-O-C₆H₁₂-, -CH₂-O-C₇H₁₄-, and -CH₂-O-C₈H₁₆-. Preferably, X³ is selected from the group consisting of - CH₂-O-C₄H₈-, -CH₂-O-C₆H₁₂- and -CH₂-O-C₈H₁₆.

For any of the above aspects, P represents a modified phosphate group. P can be represented by: 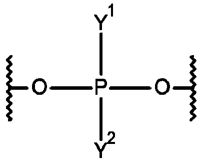 wherein Y¹ and Y² each independently represent =O, =S, -O⁻, -OH, -SH, -BH₃, -OCH₂CO₂, - OCH₂CO₂R^{x}, -OCH₂C(S)OR^{x}, and -OR^{x}, wherein R^{x} represents C₁-C₆ alkyl and wherein 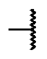 indicates attachment to the remainder of the compound.

For example, Y¹ may represent -OH and Y² may represent =O or =S; or
Y¹ may represent -O⁻ and Y² may represent =O or =S;
Y¹ may represent =O and Y² may represent -CH₃, -SH, -OR^{x}, or -BH₃
Y¹ may represent =S and Y² may represent -CH₃, OR^{x} or -SH.

It will be understood by the skilled person that in certain instances there will be delocalisation between Y¹ and Y².

Preferably, the modified phosphate group is a thiophosphate group. Thiophosphate groups include bithiophosphate (i.e. where Y¹ represents =S and Y² represents -S⁻) and monothiophosphate (i.e. where Y¹ represents -O⁻ and Y² represents =S, or where Y¹ represents =O and Y² represents -S⁻). Preferably, P is a monothiophosphate. The inventors have found that conjugates having thiophosphate groups in replacement of phosphate groups have improved potency and duration of action *in vivo.*
P may also be an ethylphosphate (i.e. where Y¹ represents =O and Y² represents OCH₂CH₃).

The saccharide, which can also be referred to as the ligand, may be selected to have an affinity for at least one type of receptor on a target cell. In particular, the receptor is on the surface of a mammalian liver cell, for example, the hepatic asialoglycoprotein receptor (ASGP-R).

For any of the above aspects, the saccharide may be selected from N-acetyl with one or more of galactosamine, mannose, galactose, glucose, glucosamine and fructose. Preferably, the saccharide is two molecules of N-acetyl galactosamine (GalNAc). The compounds of the invention may have 3 ligands which are each preferably N-acetyl galactosamine.

"GalNAc" refers to 2-(Acetylamino)-2-deoxy-D- galactopyranose, commonly referred to in the literature as N-acetyl galactosamine. Reference to "GalNAc" or "N-acetyl galactosamine" includes both the β- form: 2-(Acetylamino)-2-deoxy-β -D-galactopyranose and the α-form: 2-(Acetylamino)-2-deoxy-α-D- galactopyranose. In certain embodiments, both the β-form: 2-(Acetylarnino)-2-deoxy-β-D-galactopyranose and α-form: 2-(Acetylamino)-2-deoxy-α-D-galactopyranose may be used interchangeably. Preferably, the compounds of the invention comprise the β-form, 2-(Acetylarnino)-2-deoxy-β-D-galactopyranose. 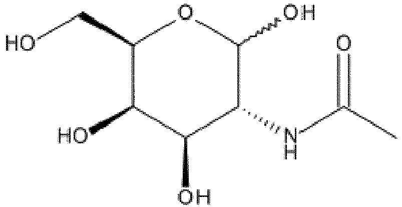 2-(Acetylamino)-2-deoxy-D-galactopyranose 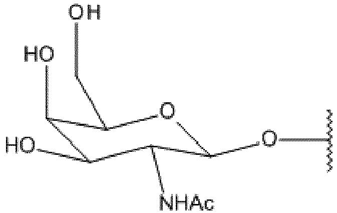 2-(Acetylamino)-2-deoxy-β-D-galactopyranose 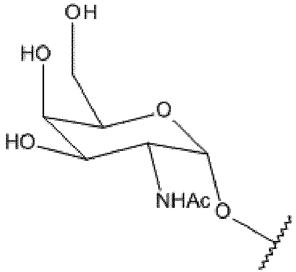 2-(Acetylamino)-2-deoxy-α-D-galactopyranose

For any of the above compounds of formula (III), X¹ may be (-CH₂-CH₂-O)ₘ(-CH₂)₂- wherein m is 1, 2, or 3. X¹ may be (-CH₂-CH₂-O)(-CH₂)₂-. X¹ may be (-CH₂-CH₂-O)₂(-CH₂)₂-. X¹ may be (-CH₂-CH₂-O)₃(-CH₂)₂-. Preferably, X¹ is (-CH₂-CH₂-O)₂(-CH₂)₂-. Alternatively, X¹ represents C₃-C₆ alkylene. X¹ may be propylene. X¹ may be butylene. X¹ may be pentylene. X¹ may be hexylene. Preferably the alkyl is a linear alkylene. In particular, X¹ may be butylene.

For compounds of formula (III), X² represents an alkylene ether of formula -C₃H₆-O-CH₂- i.e. C₃ alkoxy methylene, or -CH₂CH₂CH₂OCH₂-.

The present invention therefore additionally provides a conjugated nucleic acid having one of the following structures 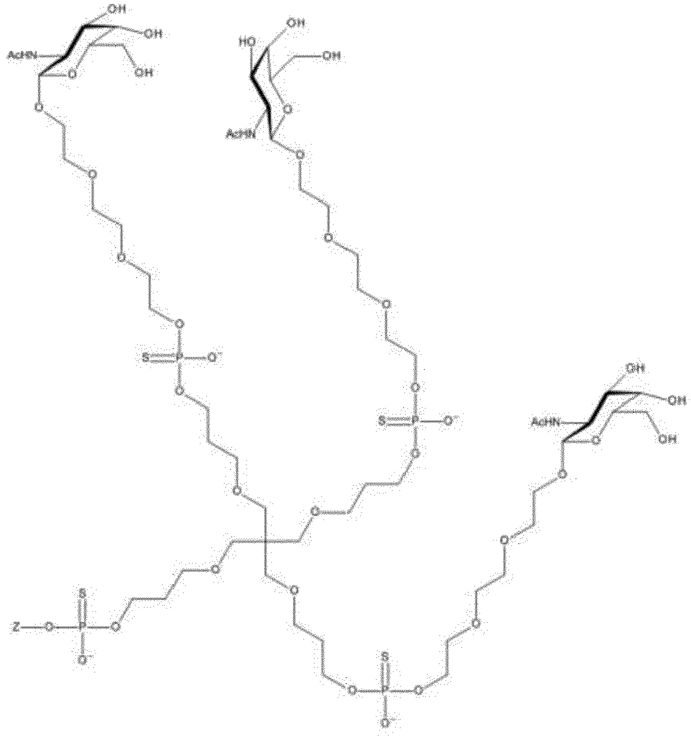 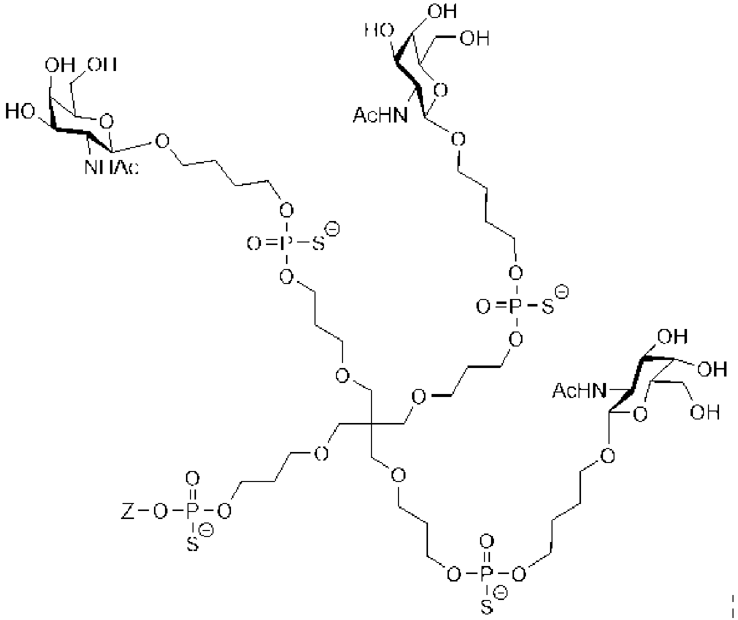 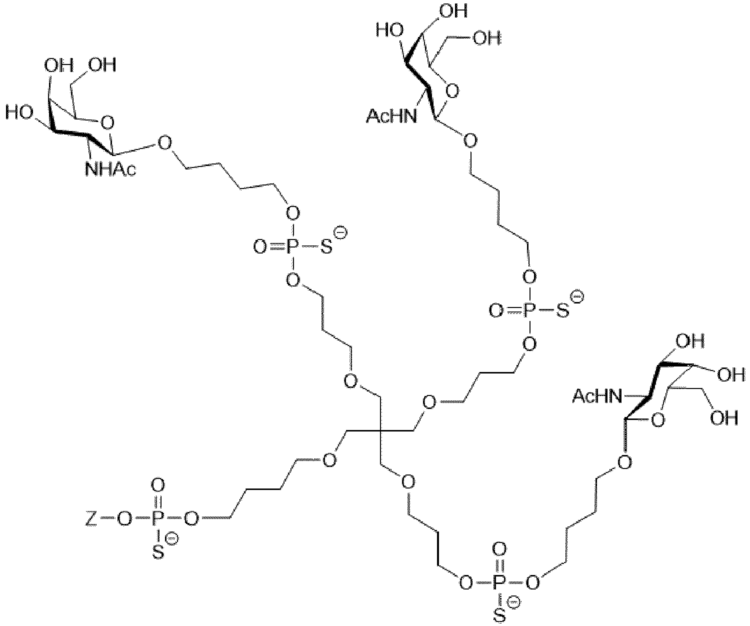 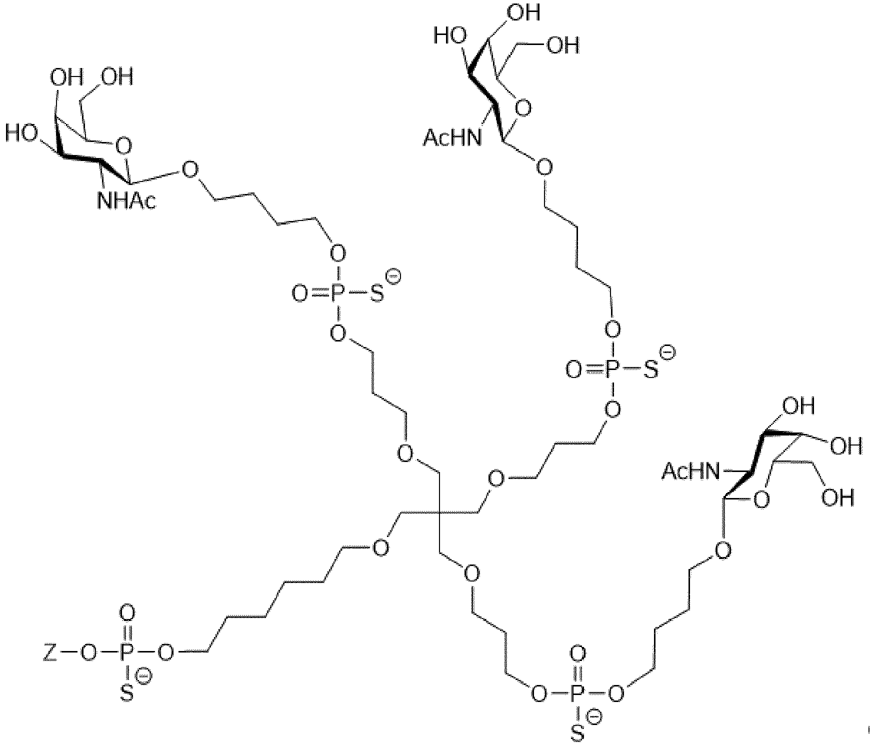 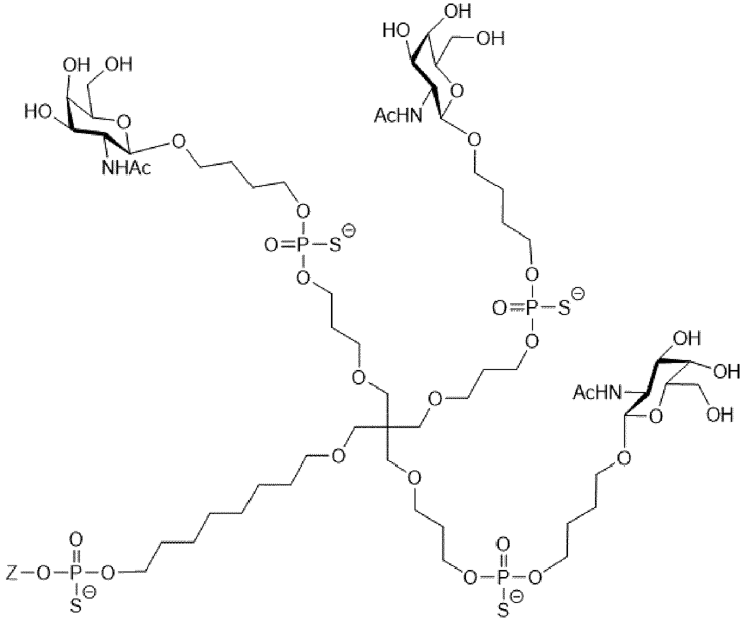 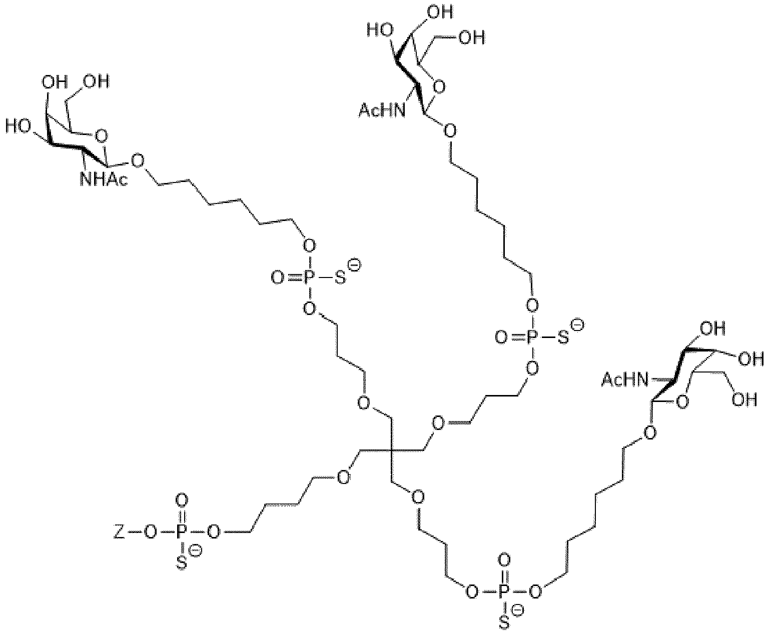 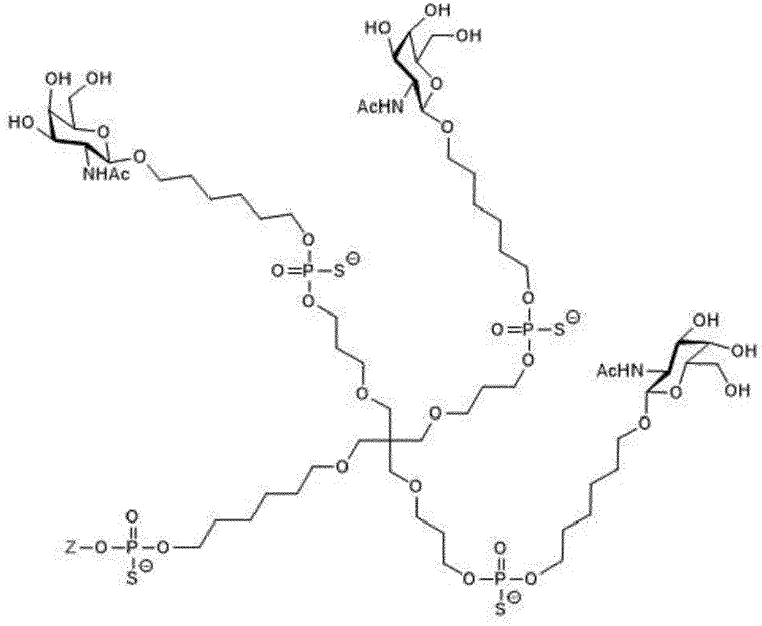 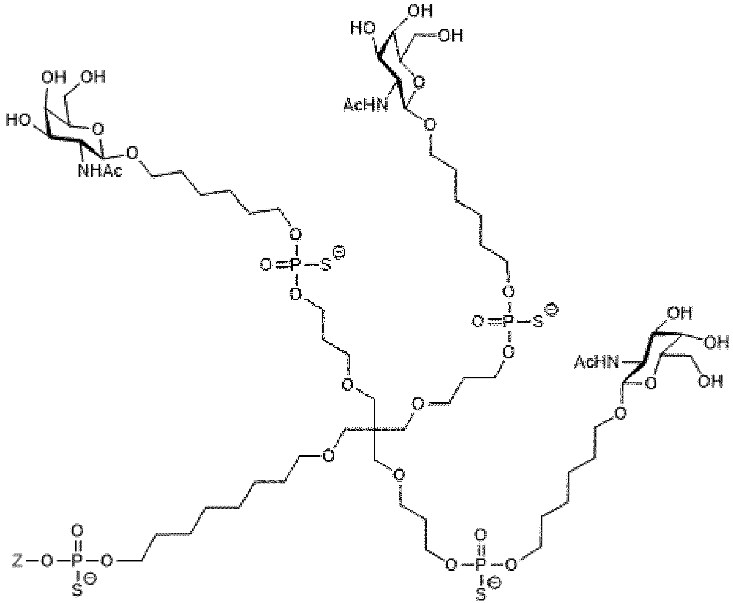 wherein Z represents a nucleic acid as defined herein before.

The invention provides, as another aspect, a nucleic acid or conjugated nucleic acid for inhibiting expression of a target gene in a cell, comprising at least one duplex region that comprises at least a portion of a first strand and at least a portion of a second strand that is at least partially complementary to the first strand, wherein said first strand is at least partially complementary to at least a portion of a RNA transcribed from the target gene, wherein said first strand comprises a modified nucleotide at selected position in order to facilitate processing of the nucleic acid by RISC, wherein the nucleic acid is conjugated indirectly or directly to a ligand via a linker. The nucleic acid may be conjugated to a ligand as herein described. The nucleotides of the first and/or second strand may be modified, as herein described.

The ligand may be conjugated to the nucleic acid via a linker as set out in formula I and wherein the first strand is modified with a 2'OMe modification on the odd numbered nucleotides, and modified with a 2'F on the even numbered nucleotides, and the second strand is modified with a 2'OMe on the even numbered nucleotides and modified with a 2'F on the odd numbered nucleotides.

The ligand may GalNac and be attached via a linker.

In another aspect the invention relates to a nucleic acid as described herein, conjugated to a targeting ligand via a linker, wherein the linker is a serinol-derived linker moiety.

The term **"serinol-derived linker moiety"** means the linker moiety comprises the following structure:

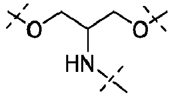

The moiety may comprise other groups such as methyl groups, such as a methyl group, for example a methyl group in the alpha-position. The moiety may comprise a further linker group such as group L defined below, interposed between the N atom of the serinol-derived linker moiety and the targeting ligand.
Preferably the ligand is a moiety (or several moieties) such as a saccharide, such as a galactosamine derivative e.g. GalNAc. It preferably has an affinity for at least one type of receptor on a target cell. In particular, the receptor is on the surface of a mammalian liver cell, for example, the hepatic asialoglycoprotein receptor (ASGP-R).

Preferably the targeting ligand comprises GalNAc.

The nucleic acid may be any nucleic acid disclosed herein.

The present invention therefore relates to a conjugate for inhibiting expression of a target gene in a cell, said conjugate comprising a nucleic acid portion and ligand portions, said nucleic acid portion comprising at least one duplex region that comprises at least a portion of a first RNA strand and at least a portion of a second RNA strand that is at least partially complementary to the first strand, wherein said first strand is at least partially complementary to at least a portion of RNA transcribed from said target gene, said ligand portions comprising a serinol-derived linker moiety and a targeting ligand for in vivo targeting of cells and being conjugated exclusively to the 3' and/or 5' ends of one or both RNA strands, wherein the 5' end of the first RNA strand is not conjugated, wherein:
(i) the second RNA strand is conjugated at the 5' end to the ligand, and wherein (a) the second RNA strand is also conjugated at the 3' end to the ligand and the 3' end of the first RNA strand is not conjugated; or (b) the first RNA strand is conjugated at the 3' end to the ligand and the 3' end of the second RNA strand is not conjugated; or (c) both the second RNA strand and the first RNA strand are also conjugated at the 3' ends; or
(ii) both the second RNA strand and the first RNA strand are conjugated at the 3' ends and the 5' end of the second RNA strand is not conjugated;
(iii) and whererin the first strand of the nucleic acid includes modified nucleotides at a plurality of positions, and wherein the nucleotides at positions 2 and 14 from the 5' end of the first strand are not modified with a 2' O-methyl modification.

The nucleic acid may have any of the following preferred features:
(i) the nucleotides at positions 2 and 14 from the 5' end of the first strand are modified;
(ii) the nucleotides at positions 2 and 14 from the 5' end of the first strand are not modified with a 2' O-methyl modification, and the nucleotide on the second strand which corresponds to position 13 of the first strand is not modified with a 2' O-methyl modification;
(iii) the nucleotides at positions 2 and 14 from the 5' end of the first strand are not modified with a 2' O-methyl modification, and the nucleotide on the second strand which corresponds to position 11 of the first strand is not modified with a 2' O-methyl modification;
(iv) nucleotides at positions 2 and 14 from the 5' end of the first strand are not modified with a 2' O-methyl modification, and the nucleotides on the second strand which corresponds to position 11 and 13 of the first strand are not modified with a 2' O-methyl modification;
(v) the nucleotides on the second strand corresponding to positions 11 and/or 13 from the 5' end of the first strand are modified;
(vi) the nucleotides at positions 2 and 14 from the 5' end of the first strand are not modified with a 2' O-methyl modification, and the nucleotides on the second strand which correspond to position 11, or 13, or 11 and 13, or 11-13 of the first strand are modified with a 2' fluoro modification;
(vii) the nucleotides at positions 2 and 14 from the 5' end of the first strand are modified with a 2' fluoro modification, and the nucleotides on the second strand which correspond to position 11, or 13, or 11 and 13, or 11-13 of the first strand are not modified with a 2' O-methyl modification;
(viii) the nucleotides at positions 2 and 14 from the 5' end of the first strand are modified with a 2' fluoro modification, and the nucleotides on the second strand which correspond to position 11, or 13, or 11 and 13, or 11-13 of the first strand are modified with a 2' fluoro modification;
(ix) greater than 50% of the nucleotides of the first and/or second strand comprise a 2' O-methyl modification, such as greater than 55%, 60%, 65%, 70%, 75%, 80%, or 85%, or more, of the first and/or second strand comprise a 2' O-methyl modification, preferably measured as a percentage of the total nucleotides of both the first and second strands;
(x) no more than 20%, (such as no more than 15% or no more than 10%) of 2' fluoro modifications on the first and/or second strand, as a percentage of the total nucleotides of both strands;
(xi) the terminal nucleotide at the 3' end of at least one of the first strand and the second strand is an inverted nucleotide and is attached to the adjacent nucleotide via the 3' carbon of the terminal nucleotide and the 3' carbon of the adjacent nucleotide and/ or the terminal nucleotide at the 5' end of at least one of the first strand and the second strand is an inverted nucleotide and is attached to the adjacent nucleotide via the 5' carbon of the terminal nucleotide and the 5' carbon of the adjacent nucleotide, or wherein the nucleic acid comprises a phosphorodithioate linkage.

In this aspect the second RNA strand (i.e. the sense strand) is conjugated at the 5' end to the targeting ligand, the first RNA strand (i.e. the antisense strand) is conjugated at the 3' end to the targeting ligand and the 3' end of the second RNA strand (i.e. the sense strand) is not conjugated, such that a conjugate with the following schematic structure is formed:

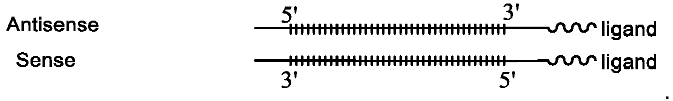

In an embodiment of the present invention, the second RNA strand (i.e. the sense strand) is conjugated at the 5' end to the targeting ligand, the second RNA strand (i.e. the sense strand) is also conjugated at the 3' end to the targeting ligand and the 3' end of the first RNA strand (i.e. the antisense strand) is not conjugated, such that a conjugate with the following schematic structure is formed:

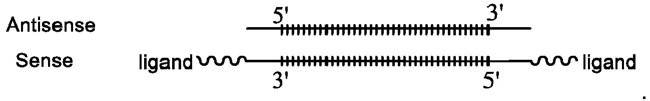

In an embodiment of the present invention, both the second RNA strand (i.e. the sense strand) and the first RNA strand (i.e. the antisense strand) are conjugated at the 3' ends to the targeting ligand and the 5' end of the second RNA strand (i.e. the sense strand) is not conjugated , such that a conjugate with the following schematic structure is formed:

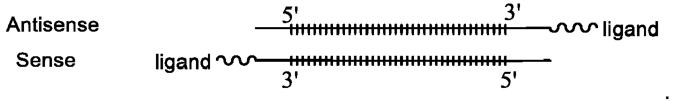

In an embodiment of the present invention, the second RNA strand (i.e. the sense strand) is conjugated at the 5' end to the targeting ligand and both the second RNA strand (i.e. the sense strand) and the first RNA strand (i.e. the antisense strand) are also conjugated at the 3' ends to the targeting ligand , such that a conjugate with the following schematic structure is formed:

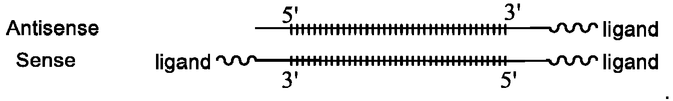

In any one of the above embodiments, 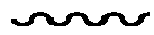 indicates the linker which conjugates the ligand to the ends of the nucleic acid portion; the ligand may be a GalNAc moiety such as GalNAc; and wherein 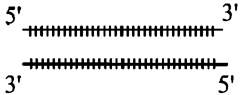 represents the nucleic acid portion.

These schematic diagrams are not intended to limit the number of nucleotides in the first or second strand, nor do the diagrams represent any kind of limitation on complementarity of the bases or any other limitation.

The ligands may be monomeric or multimeric (e.g. dimeric, trimeric, etc.).

Suitably, the ligands are monomeric, thus containing a single targeting ligand moiety, e.g. a single GalNAc moiety.

Alternatively, the ligands may be dimeric ligands wherein the ligand portions comprise two serinol-derived linker moieties, each linked to a single targeting ligand moiety.

The ligands may be trimeric ligands wherein the ligand portions comprise three serinol-derived linker moieties, each linked to a single targeting ligand moiety.

The two or three serinol-derived linker moieties may be linked in series e.g. as shown below: 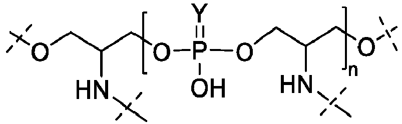 wherein n is 1 or 2 and Y is S or O.

Preferably, the ligands are monomeric.

Suitably, the conjugated RNA strands are conjugated to a targeting ligand via a serinol-derived linker moiety including a further linker wherein the further linker is or comprises a saturated, unbranched or branched C₁₋₁₅ alkyl chain, wherein optionally one or more carbons (for example 1, 2 or 3 carbons, suitably 1 or 2, in particular 1) is/are replaced by a heteroatom selected from O, N, S(O)ₚ, wherein p is 0, 1 or 2 (for example a CH₂ group is replaced with O, or with NH, or with S, or with SO₂ or a -CH₃ group at the terminus of the chain or on a branch is replaced with OH or with NH2) wherein said chain is optionally substituted by one or more oxo groups (for example 1 to 3, such as 1 group).

More suitably, the further linker comprises a saturated, unbranched C₁₋₁₅ alkyl chain wherein one or more carbons (for example 1, 2 or 3 carbons, suitably 1 or 2, in particular 1) is/are replaced by an oxygen atom.

More suitably, the further linker comprises a PEG-chain.

More suitably, the further linker comprises a saturated, unbranched C₁₋₁₅ alkyl chain.

More suitably, the further linker comprises a saturated, unbranched C₁₋₆ alkyl chain.

More suitably, the further linker comprises a saturated, unbranched C₄ or C₆ alkyl chain, e.g. a C₄ alkyl chain.

In an embodiment, 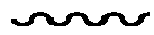 is a linking moiety of formula (III): 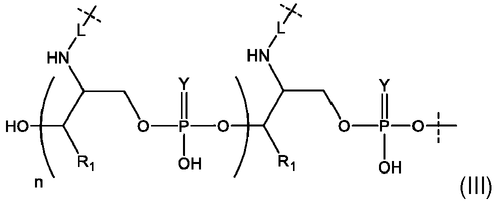 wherein n, Y, R₁ and L are defined below, L is connected to the targeting ligand e.g. GalNAc and the O of the phosphoro-group is attached to the terminal oligonucleoside of the RNA strands.

Suitably, the targeting ligand portion is a linking moiety of formula (IV): 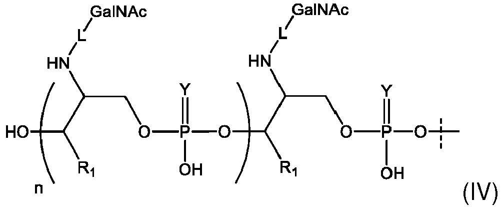 wherein n, Y, R₁ and L are defined below and the O of the phosphoro-group is attached to the terminal oligonucleoside of the RNA strands.

Suitably, L is:

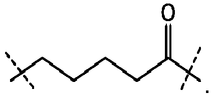

In any of the above structures, suitably the ligands are selected from GalNAc and galactose moieties, especially GalNAc moieties. Alternatively, GalNac may be replaced by another targeting ligand, e.g. a saccharide.

In an embodiment of the invention, the first RNA strand is a compound of formula (I): 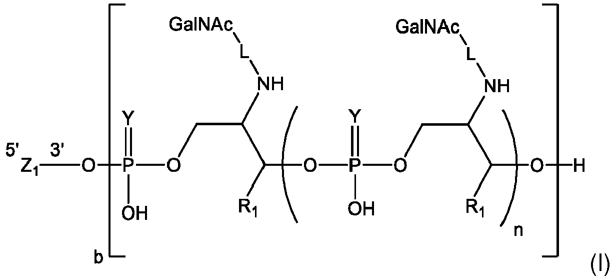 wherein b is 0 or 1; and
the second RNA strand is a compound of formula (II): 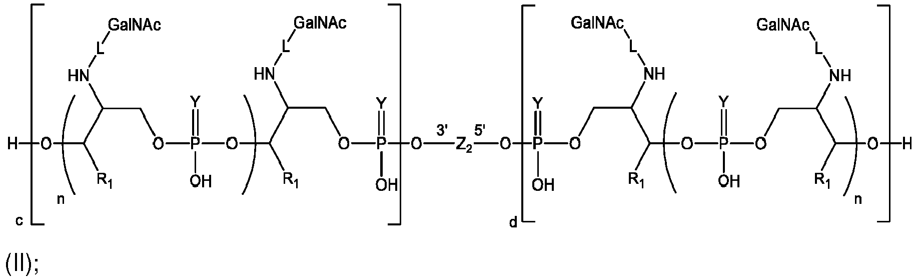 wherein c and d are independently 0 or 1;
wherein:
Z₁ and Z₂ are the RNA portions of the first and second RNA strands respectively;
Y is O or S;
R₁ is H or methyl;
n is 0, 1, 2 or 3; and
L is the same or different in formulae (I) and (II) and is selected from the group consisting of:

   -(CH₂)ᵣ-C(O)-,

   wherein r = 2-12;

   -(CH₂-CH₂-O)ₛ-CH₂-C(O)-,

   wherein s = 1-5;

   -(CH₂)ₜ-CO-NH-(CH₂)ₜ-NH-C(O)-,

   wherein t is independently is 1-5;

   -(CH₂)ᵤ-CO-NH-(CH₂)ᵤ-C(O)-,

   wherein u is independently is 1-5; and

   -(CH₂)ᵥ-NH-C(O)-,

   wherein v is 2-12; and
wherein the terminal C(O) (if present) is attached to the NH group;
   and wherein b + c + d is 2 or 3.

Suitably, b is 0, c is 1 and d is 1; b is 1, c is 0 and d is 1; b is 1, c is 1 and d is 0; or b is 1, c is 1 and d is 1.

More suitably, b is 0, c is 1 and d is 1; b is 1, c is 0 and d is 1; or b is 1, c is 1 and d is 1. Most suitably, b is 0, c is 1 and d is 1.

In one embodiment, Y is O. In another embodiment, Y is S.

In one embodiment, R₁ is H or methyl. In one embodiment, R₁ is H. In another embodiment, R₁ is methyl.

In one embodiment, n is 0, 1, 2 or 3. Suitably, n is 0.

In one embodiment, L is selected from the group consisting of:

-(CH₂)ᵣ-C(O)-,

wherein r = 2-12;

-(CH₂-CH₂-O)s-CH₂-C(O)-,

wherein s = 1-5;

-(CH₂)ₜ-CO-NH-(CH₂)ₜ-NH-C(O)-,

wherein t is independently is 1-5;

-(CH₂)ᵤ-CO-NH-(CH₂)ᵤ-C(O)-,

wherein u is independently is 1-5; and

-(CH₂)ᵥ-NH-C(O)-,

wherein v is 2-12;
wherein the terminal C(O) (if present) is attached to the NH group.

Suitably, L is -(CH₂)ᵣ-C(O)-, wherein r = 2-12. Suitably, r = 2-6. More suitably, r = 4 or 6 e.g. 4. Serinol derived linker moieties may be based on serinol in any stereochemistry i.e. derived from L-serine isomer, D-serine isomer, a racemic serine or other combination of isomers. In a preferred aspect of the invention, the serinol-GalNAc moiety (SerGN) has the following stereochemistry: 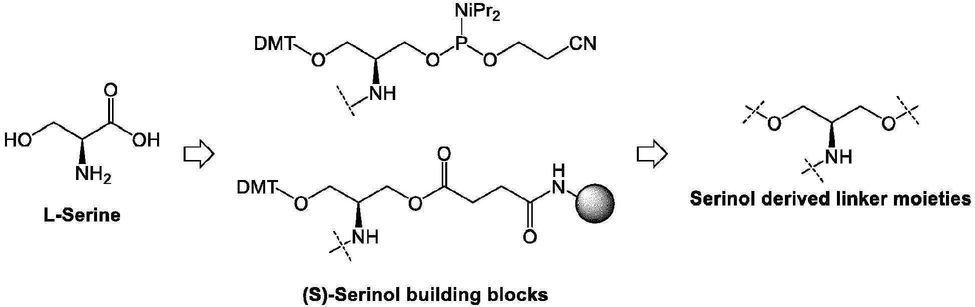 i.e. is based on an (S)-serinol-amidite or (S)-serinol succinate solid supported building block derived from L-serine isomer.

In one embodiment, the targeted cells are hepatocytes.

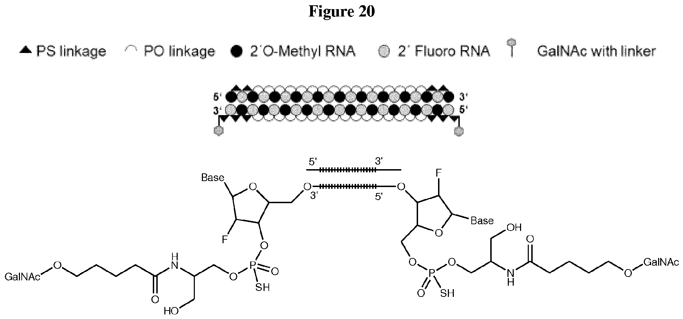

Preferred are conjugates 1, 2 and 3, and in particular conjugate 2 in Figure 20.

Preferably the nucleic acid according to the present invention is conjugated to the linker moiety of the ligand via a phosphate or modified phosphate (preferably a thiophosphate).

### General synthesis schemes

Example compounds can be synthesised according to methods described below and known to the person skilled in the art. Assembly of the oligonucleotide chain and linker building blocks may, for example, be performed by solid phase synthesis applying phosphoramidte methodology. Solid phase synthesis may start from a base or modified building block loaded Icaa CPG. Phosphoramidite synthesis coupling cycle consists of 1) DMT-removal, 2) chain elongation using the required DMT-masked phosphoramidite and an activator, which may be benzylthiotetrazole (BTT), 3) capping of non-elongated oligonucleotide chains, followed by oxidation of the P(III) to P(V) either by Iodine (if phosphodiester linkage is desired) or EDITH (if phosphorothioate linkage is desired) and again capping (Cap/Ox/Cap or Cap/Thio/Cap). GalNAc conjugation may be achieved by peptide bond formation of a GalNAc-carboxylic acid building block to the prior assembled and purified oligonucleotide having the necessary number of amino modified linker building blocks attached. The necessary building blocks are either commercially available or synthesis is described below. All final single stranded products were analysed by AEX-HPLC to prove their purity. Purity is given in %FLP (% full length product) which is the percentage of the UV-area under the assigned product signal in the UV-trace of the AEX-HPLC analysis of the final product. Identity of the respective single stranded products was proved by LC-MS analysis.

### Synthesis of Synthons

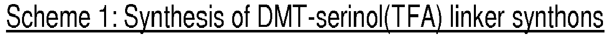

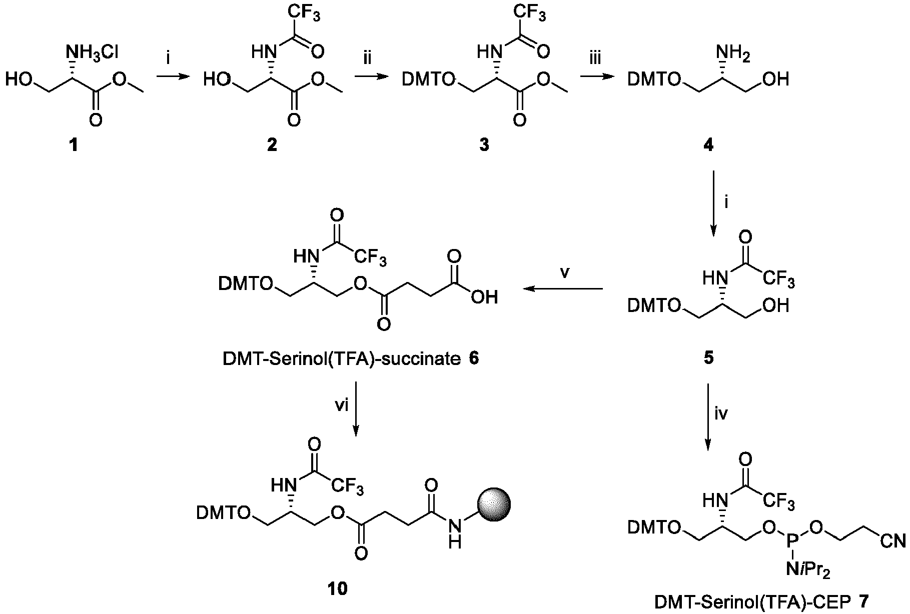

i) ethyl trifluoroacetate, NEt₃, MeOH, 0°C, 16h, 2: 86% 5: 90%, ii) DMTCI, pyridine, 0°C, 16h, 74%, iii) LiBH4, EtOH/THF (1/1, v/v), 0°C, 1h, 76%, iv) 2-cyanoethyl-N,N-diisopropylchloro phosphoramidite, EtN*i*Pr₂, CH₂Cl₂, 56%, v) succinic anhydride, DMAP, pyridine, RT, 16h, 38%, vi) HBTU, DIEA, amino-Icaa CPG (500A), RT, 18h, 29% (26 umol/g loading).

DMT-Serinol(TFA)-phosphoramidite **7** can be synthesised from serinol derivative 1 according to literature published methods (Hoevelmann et al. Chem. Sci., 2016,7, 128-135). DMT-Serinol(TFA)-succinate **6** can be made by conversion of intermediate **5** with succinic anhydride in presence of a catalyst such as DMAP.
Loading of **6** to a solid support such as a controlled pore glass (CPG) support may be achieved by peptide bond formation to a solid support such as an amino modified native CPG support (500A) using a coupling reagent such as HBTU. The DMT-Serinol(TFA)-succinate **6** and a coupling reagent such as HBTU is dissolved in a solvent such as CH₃CN. A base, such as diisopropylethylamine, is added to the solution, and the reaction mixture is stirred for 2 min. A solid support such as a native amino-Icaa-CPG support (500 A, 3 g, amine content: 136 umol/g) is added to the reaction mixture and a suspension forms. The suspension is gently shaken at room temperature on a wrist-action shaker for 16h then filtered, and washed with solvent such as DCM and EtOH. The support is dried under vacuum for 2 h. The unreacted amines on the support can be capped by stirring with acetic anhydride/lutidine/N-methylimidazole at room temperature. Washing of the support may be repeated as above.

The solid support is dried under vacuum to yield solid support **10.** 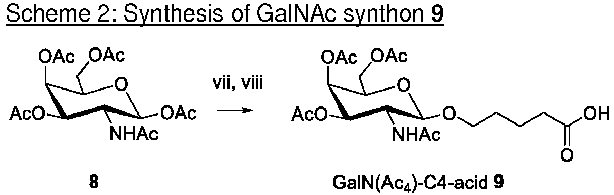 (vii) TMSOTf, DCM, hexenol, viii) RuCl₃, NalO₄, DCM, CH₃CN, H₂O, 46% over two steps. Synthesis of the GalNAc synthon **9** can be prepared according to methods as described in Nair et al. J. Am. Chem. Soc., 2014, 136 (49), pp 16958-16961, starting from commercially available per-acetylated galactose amine **8**.

### Synthesis of the single stranded serinol-derived GalNAc conjugates in conjugates 1-3 and reference conjugates 1-2

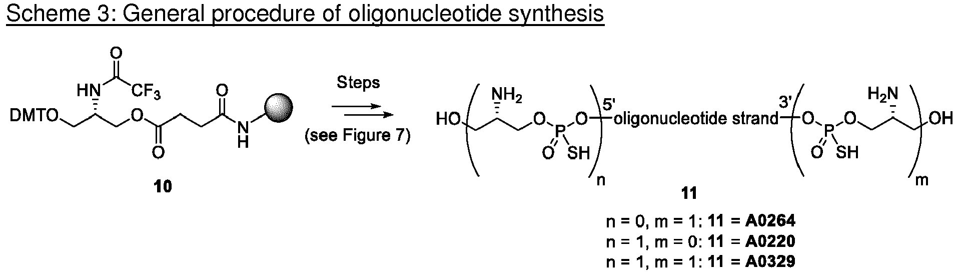

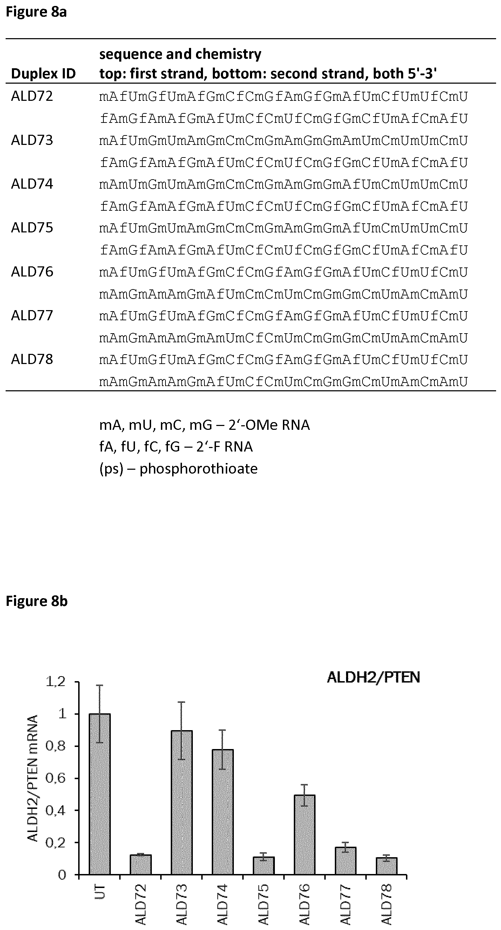

Oligonucleotide synthesis of 3' mono-GalNAc conjugated oligonucleotides (such as compound **A0264**) is outlined in Figure 8 and summarised in Scheme 3. Synthesis is commenced using DMT-Serinol(TFA) -succinate-Icaa-CPG **10** as in example compound **A0264**. In case additional serinol building blocks are needed the DMT-serinol(TFA) amidite (7) is used in the appropriate solid phase synthesis cycle. For example, to make compound **A0329**, the chain assembly is finished with an additional serinol amidite coupling after the base sequence is fully assembled. Further, oligonucleotide synthesis of 5' mono-GalNAc conjugated oligonucleotides may be commenced from a solid support loaded with the appropriate nucleoside of its respected sequence. In example compound **A0220** this may be 2'fA. The oligonucleotide chain is assembled according to its sequence and as appropriate, the building block DMT-serinol(TFA)-amidite (7) is used. Upon completion of chain elongation, the protective DMT group of the last coupled amidite building block is removed, as in step 1) of the phosphoramidite synthesis cycle.

Upon completion of the last synthesizer step, the single strands can be cleaved off the solid support by treatment with an amine such as 40% aq. methylamine treatment. Any remaining protecting groups are also removed in this step and methylamine treatment also liberates the serinol amino function. The crude products were then purified each by AEX-HPLC and SEC to yield the precursor oligonucleotide for further GalNAc conjugation.

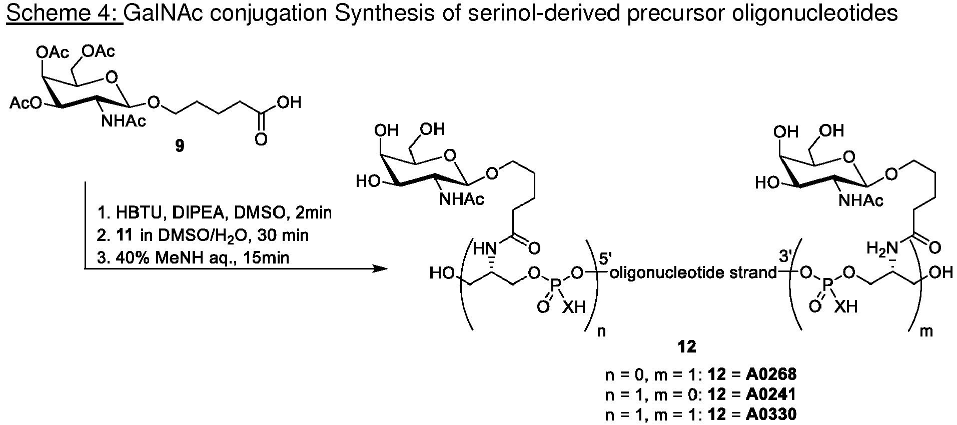

Post solid phase synthesis GalNAc-conjugation was achieved by pre-activation of the GaIN(Ac4)-C4-acid **(9)** by a peptide coupling reagent such as HBTU. The pre-activated acid **9** was then reacted with the amino-groups in **11** (e.g. **A0264**) to form the intermediate GalN(Ac4)-conjugates. The acetyl groups protecting the hydroxyl groups in the GalNAc-moieties were cleaved off by methylamine treatment to yield the desired example compounds **12** (e.g. **A0268),** which were further purified by AEX-HPLC and SEC.

### Synthesis of the single stranded tri-antennary GalNAc conjugates in reference conjugates 3-4

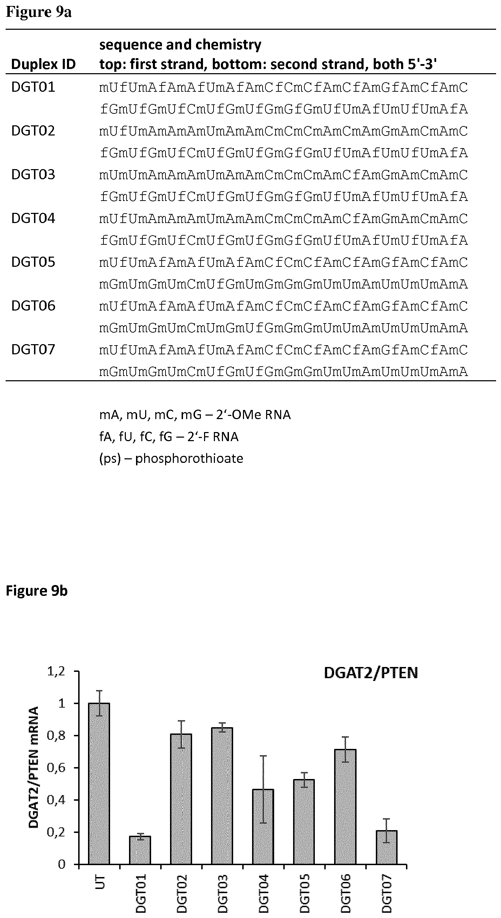

Oligonucleotides synthesis of tri-antennary GalNAc-cluster conjugated siRNA is outlined in Figure 9. Oligonucleotide chain assembly is commenced using base loaded support e.g. 5'DMT-2'FdA(bz)-succinate-Icaa-CPG as in example compound **A0006**. Phosphoramidite synthesis coupling cycle consisting of 1) DMT-removal, 2) chain elongation using the required DMT-masked phosphoramidite, 3) capping of non-elongated oligonucleotide chains, followed by oxidation of the P(III) to P(V) either by Iodine or EDITH (if phosphorothioate linkage is desired) and again capping (Cap/Ox/Cap or Cap/Thio/Cap) is repeated until full length of the product is reached. For the on column conjugation of a trivalent tri-antennary GalNAc cluster the same synthesis cycle was applied with using the necessary trivalent branching amidite **C4XLT-phos** followed by another round of the synthesis cycle using the GalNAc amidite **ST23-phos**. Upon completion of this last synthesizer step, the oligonucleotide was cleaved from the solid support and additional protecting groups may be removed by methylamine treatment. The crude products were then purified each by AEX-HPLC and SEC.

### General procedure of double strand formation

In order to obtain the double stranded conjugates, individual single strands are dissolved in a concentration of 60 OD/mL in H₂O. Both individual oligonucleotide solutions can be added together to a reaction vessel. For reaction monitoring a titration can be performed. The first strand is added in 25% excess over the second strand as determined by UV-absorption at 260nm. The reaction mixture is heated e.g. to 80°C for 5min and then slowly cooled to RT. Double strand formation may be monitored by ion pairing reverse phase HPLC. From the UV-area of the residual single strand the needed amount of the second strand can be calculated and added to the reaction mixture. The reaction is heated e.g. to 80°C again and slowly cooled to RT. This procedure can be repeated until less than 10% of residual single strand is detected.

The above process (including Schemes 1-4 and Figures 8 and 9) may be easily adapted to replace GalNac with another targeting ligand e.g. a saccharide.

In any of the above aspects, instead of post solid phase synthesis conjugation it is possible to make a preformed Serinol(GN)-phosphoramidite and use this for on-column conjugation.

The nucleic acid as described herein may be formulated with a lipid in the form of a liposome. Such a formulation may be described in the art as a lipoplex. The formulation with a lipid/liposome may be used to assist with delivery of the nucleic acid of the invention to the target cells. The lipid delivery system herein described may be used as an alternative to a conjugated ligand. The modifications herein described may be present when using a nucleic acid of the invention with a lipid delivery system or with a ligand conjugate delivery system.

Such a lipoplex may comprise a lipid formulation comprising:
i) a cationic lipid, or a pharmaceutically acceptable salt thereof;
ii) a steroid;
iii) a phosphatidylethanolamine phospholipid;
iv) a PEGylated lipid.

The cationic lipid may be an amino cationic lipid.

The cationic lipid may have the formula (I): 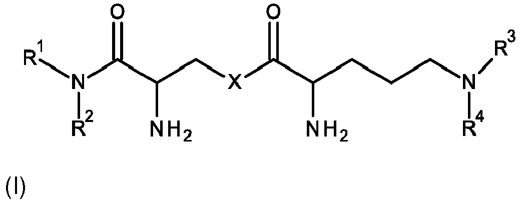 or a pharmaceutically acceptable salt thereof, wherein:
X represents O, S or NH;
R¹ and R² each independently represents a C₄-C₂₂ linear or branched alkyl chain or a C₄-C₂₂ linear or branched alkenyl chain with one or more double bonds, wherein the alkyl or alkenyl chain optionally contains an intervening ester, amide or disulfide;
when X represents S or NH, R³ and R⁴ each independently represent hydrogen, methyl, ethyl, a mono- or polyamine moiety, or R³ and R⁴ together form a heterocyclyl ring;
when X represents O, R³ and R⁴ each independently represent hydrogen, methyl, ethyl, a mono- or polyamine moiety, or R³ and R⁴ together form a heterocyclyl ring, or R³ represents hydrogen and R⁴ represents C(NH)(NH₂).

The cationic lipid may have the formula (IA): 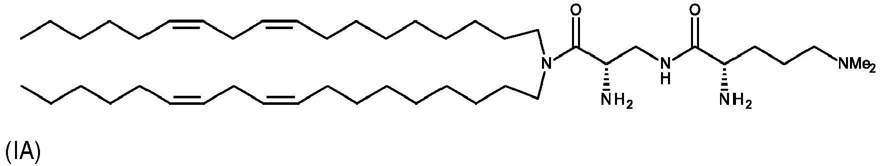 or a pharmaceutically acceptable salt thereof.

The cationic lipid may have the formula (IB): 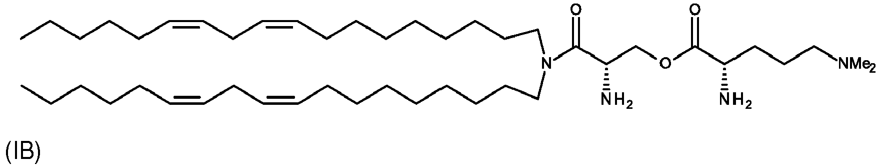 or a pharmaceutically acceptable salt thereof.

The content of the cationic lipid component may be from about 55 mol% to about 65 mol% of the overall lipid content of the formulation. In particular, the cationic lipid component is about 59 mol% of the overall lipid content of the formulation.

The formulations further comprise a steroid. the steroid may be cholesterol. The content of the steroid may be from about 26 mol% to about 35 mol% of the overall lipid content of the lipid formulation. More particularly, the content of steroid may be about 30 mol% of the overall lipid content of the lipid formulation.

The phosphatidylethanolamine phospholipid may be selected from group consisting of 1,2-diphytanoyl-sn-glycero-3-phosphoethanolamine (DPhyPE), 1,2-dioleoyl-*sn*-glycero-3-phosphoethanolamine (DOPE), 1,2-distearoyl-sn-glycero-3-phosphoethanolamine (DSPE), 1,2-Dilauroyl-sn-glycero-3-phosphoethanolamine (DLPE), 1,2-Dimyristoyl-sn-glycero-3-phosphoethanolamine (DMPE), 1,2-Dipalmitoyl-sn-glycero-3-phosphoethanolamine (DPPE), 1,2-Dilinoleoyl-sn-glycero-3-phosphoethanolamine (DLoPE), 1-Palmitoyl-2-oleoyl-sn-glycero-3-phosphoethanolamine (POPE), 1,2-Dierucoyl-sn-glycero-3-phosphoethanolamine (DEPE), 1,2-Disqualeoyl-sn-glycero-3-phosphoethanolamine (DSQPE) and 1-Stearoyl-2-linoleoyl-sn-glycero-3-phosphoethanolamine (SLPE). The content of the phospholipid may be about 10 mol% of the overall lipid content of the formulation.

The PEGylated lipid may be selected from the group consisting of 1,2-dimyristoyl-sn-glycerol, methoxypolyethylene glycol (DMG-PEG) and C16-Ceramide-PEG. The content of the PEGylated lipid may be about 1 to 5 mol% of the overall lipid content of the formulation.

The content of the cationic lipid component in the formulation may be from about 55 mol% to about 65 mol% of the overall lipid content of the lipid formulation, preferably about 59 mol% of the overall lipid content of the lipid formulation.

The formulation may have a molar ratio of the components of i):ii): iii): iv) selected from 55:34:10:1; 56:33:10:1; 57:32:10:1; 58:31:10:1; 59:30:10:1; 60:29:10:1; 61:28:10:1; 62:27:10:1; 63:26:10:1; 64:25:10:1; and 65:24:10:1.

The formulation may comprise a cationic lipid having the structure 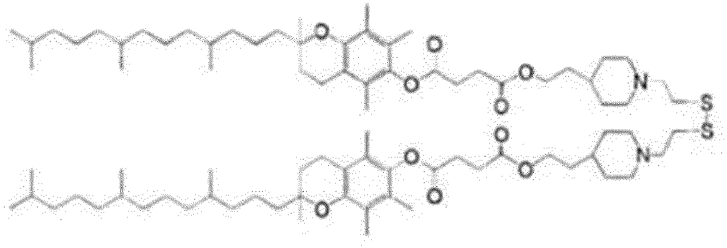 a steroid having the structure 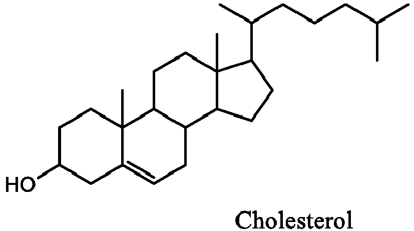 a phosphatidylethanolamine phospholipid having the structure

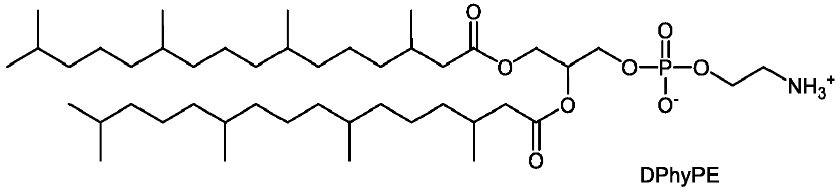

And a PEGylated lipid having the structure

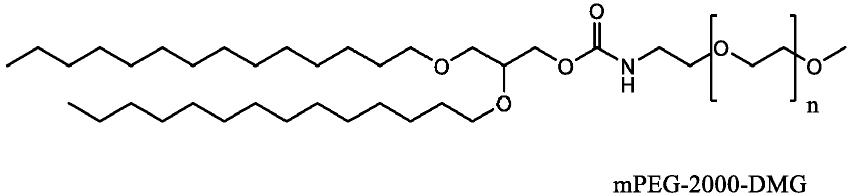

Neutral liposome compositions may be formed from, for example, dimyristoyl phosphatidylcholine (DMPC) or dipalmitoyl phosphatidylcholine (DPPC). Anionic liposome compositions may be formed from dimyristoyl phosphatidylglycerol, while anionic fusogenic liposomes may be formed primarily from dioleoyl phosphatidylethanolamine (DOPE). Another type of liposomal composition may be formed from phosphatidylcholine (PC) such as, for example, soybean PC, and egg PC. Another type is formed from mixtures of phospholipid and/or phosphatidylcholine and/or cholesterol.

A positively charged synthetic cationic lipid, N-[I-(2,3-dioleyloxy)propyl]-N,N,N-trimethylammonium chloride (DOTMA) can be used to form small liposomes that interact spontaneously with nucleic acid to form lipid-nucleic acid complexes which are capable of fusing with the negatively charged lipids of the cell membranes of tissue culture cells. DOTMA analogues can also be used to form liposomes.

Derivatives and analogues of lipids described herein may also be used to form liposomes.

A liposome containing a nucleic acid can be prepared by a variety of methods. In one example, the lipid component of a liposome is dissolved in a detergent so that micelles are formed with the lipid component. For example, the lipid component can be an amphipathic cationic lipid or lipid conjugate. The detergent can have a high critical micelle concentration and may be nonionic. Exemplary detergents include cholate, CHAPS, octylglucoside, deoxycholate, and lauroyl sarcosine. The nucleic acid preparation is then added to the micelles that include the lipid component. The cationic groups on the lipid interact with the nucleic acid and condense around the nucleic acid to form a liposome. After condensation, the detergent is removed, e.g., by dialysis, to yield a liposomal preparation of nucleic acid.

If necessary a carrier compound that assists in condensation can be added during the condensation reaction, e.g., by controlled addition. For example, the carrier compound can be a polymer other than a nucleic acid (e.g., spermine or spermidine). pH can also be adjusted to favour condensation.

Nucleic acid formulations may include a surfactant. In one embodiment, the nucleic acid is formulated as an emulsion that includes a surfactant.

A surfactant that is not ionized is a non-ionic surfactant. Examples include non-ionic esters, such as ethylene glycol esters, propylene glycol esters, glyceryl esters etc., nonionic alkanolamides, and ethers such as fatty alcohol ethoxylates, propoxylated alcohols, and ethoxylated/propoxylated block polymers.

A surfactant that carries a negative charge when dissolved or dispersed in water is an anionic surfactant. Examples include carboxylates, such as soaps, acyl lactylates, acyl amides of amino acids, esters of sulfuric acid such as alkyl sulfates and ethoxylated alkyl sulfates, sulfonates such as alkyl benzene sulfonates, acyl isethionates, acyl taurates and sulfosuccinates, and phosphates.

A surfactant that carries a positive charge when dissolved or dispersed in water is a cationic surfactant. Examples include quaternary ammonium salts and ethoxylated amines.

A surfactant that has the ability to carry either a positive or negative charge is an amphoteric surfactant. Examples include acrylic acid derivatives, substituted alkylamides, N-alkylbetaines and phosphatides.

"Micelles" are defined herein as a particular type of molecular assembly in which amphipathic molecules are arranged in a spherical structure such that all the hydrophobic portions of the molecules are directed inward, leaving the hydrophilic portions in contact with the surrounding aqueous phase. The converse arrangement exists if the environment is hydrophobic. A micelle may be formed by mixing an aqueous solution of the nucleic acid, an alkali metal alkyl sulphate, and at least one micelle forming compound.

Exemplary micelle forming compounds include lecithin, hyaluronic acid, pharmaceutically acceptable salts of hyaluronic acid, glycolic acid, lactic acid, chamomile extract, cucumber extract, oleic acid, linoleic acid, linolenic acid, monoolein, monooleates, monolaurates, borage oil, evening of primrose oil, menthol, trihydroxy oxo cholanyl glycine and pharmaceutically acceptable salts thereof, glycerin, polyglycerin, lysine, polylysine, triolein, polyoxyethylene ethers and analogues thereof, polidocanol alkyl ethers and analogues thereof, chenodeoxycholate, deoxycholate, and mixtures thereof.

Phenol and/or m-cresol may be added to the mixed micellar composition to act as a stabiliser and preservative. An isotonic agent such as glycerine may as be added.

A nucleic acid preparation may be incorporated into a particle such as a microparticle. Microparticles can be produced by spray-drying, lyophilisation, evaporation, fluid bed drying, vacuum drying, or a combination of these methods.

The present invention also provides pharmaceutical compositions comprising a nucleic acid or conjugated nucleic acid of the invention. The pharmaceutical compositions may be used as medicaments or as diagnostic agents, alone or in combination with other agents. For example, a nucleic acid or conjugated nucleic acid of the invention can be combined with a delivery vehicle (e.g., liposomes) and excipients, such as carriers, diluents. Other agents such as preservatives and stabilizers can also be added. Methods for the delivery of nucleic acids are known in the art and within the knowledge of the person skilled in the art.

A nucleic acid or conjugated nucleic acid of the present invention can also be administered in combination with other therapeutic compounds, either administrated separately or simultaneously, e.g., as a combined unit dose. The invention also includes a pharmaceutical composition comprising a nucleic acid or conjugated nucleic acid according to the present invention in a physiologically/pharmaceutically acceptable excipient, such as a stabilizer, preservative, diluent, buffer, and the like.

The pharmaceutical composition may be specially formulated for administration in solid or liquid form. The composition may be formulated for oral administration, parenteral administration (including, for example, subcutaneous, intramuscular, intravenous, or epidural injection), topical application, intravaginal or intrarectal administration, sublingual administration, ocular administration, transdermal administration, or nasal administration. Delivery using subcutaneous or intravenous methods are preferred.

Dosage levels for the medicament and pharmaceutical compositions of the invention can be determined by those skilled in the art by routine experimentation. In one embodiment, a unit dose may contain between about 0.01 mg/kg and about 100 mg/kg body weight of nucleic acid. Alternatively, the dose can be from 10 mg/kg to 25 mg/kg body weight, or 1 mg/kg to 10 mg/kg body weight, or 0.05 mg/kg to 5 mg/kg body weight, or 0.1 mg/kg to 5 mg/kg body weight, or 0.1 mg/kg to1 mg/kg body weight, or 0.1 mg/kg to 0.5 mg/kg body weight, or 0.5 mg/kg to 1 mg/kg body weight. Dosage levels may also be calculated via other parameters such as, e.g., body surface area.

The pharmaceutical composition may be a sterile injectable aqueous suspension or solution, or in a lyophilized form. In one embodiment, the pharmaceutical composition may comprise lyophilized lipoplexes or an aqueous suspension of lipoplexes. The lipoplexes preferably comprises a nucleic acid of the present invention. Such lipoplexes may be used to deliver the nucleic acid of the invention to a target cell either in vitro or in vivo.

The pharmaceutical compositions and medicaments of the present invention may be administered to a mammalian subject in a pharmaceutically effective dose. The mammal may be selected from humans, dogs, cats, horses, cattle, pig, goat, sheep, mouse, rat, hamster and guinea pig.

A further aspect of the invention relates to a nucleic acid or conjugated nucleic acid of the invention or the pharmaceutical composition comprising a nucleic acid or conjugated nucleic acid of the invention for use in the treatment of a disease or disorder. The invention includes a pharmaceutical composition comprising a nucleic acid or conjugated nucleic acid according to the present invention in a physiologically/ pharmaceutically acceptable excipient, such as a stabiliser, preservative, diluent, buffer and the like.

The pharmaceutical composition may be a sterile injectable aqueous suspension or solution, or in a lyophilised form.

Pharmaceutically acceptable compositions may comprise a therapeutically-effective amount of a nucleic acid or conjugated nucleic acid in any embodiment according to the invention, taken alone or formulated with one or more pharmaceutically acceptable carriers, excipient and/or diluents.

Examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) lubricating agents, such as magnesium state, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen- free water; (17) isotonic saline; (18) Ringer's solution; (19) ethyl alcohol; (20) pH buffered solutions; (21) polyesters, polycarbonates and/or polyanhydrides; (22) bulking agents, such as polypeptides and amino acids (23) serum component, such as serum albumin, HDL and LDL; and (22) other non-toxic compatible substances employed in pharmaceutical formulations.

Stabilisers may be agents that stabilise a nucleic acid or conjugated nucleic acid, for example a protein that can complex with the nucleic acid, chelators (e.g. EDTA), salts, RNAse inhibitors, and DNAse inhibitors.

In some cases it is desirable to slow the absorption of the drug from subcutaneous or intramuscular injection in order to prolong the effect of a drug. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

The nucleic acid described herein may be capable of inhibiting the expression of the target gene in a cell. The nucleic acid described herein may be capable of partially inhibiting the expression of the target gene in a cell. Inhibition may be complete, i.e. 0% of the expression level of target gene expression in the absence of the nucleic acid of the invention. Inhibition of target gene expression may be partial, i.e. it may be 15%, 20%, 30%, 40%, 50%, 60%, 70%, 75%, 80%, 85%, 90%, 95% of target gene expression in the absence of a nucleic acid of the invention. Inhibition may last 4 weeks, 5 weeks, 6 weeks, 7 weeks, 8 weeks, 10 weeks, 11 weeks, 12 weeks, 13 weeks, 14 weeks or up to 3 months, when used in a subject, such as a human subject. The nucleic acid or conjugated nucleic acid or composition comprising the same may be for use once, every week, every two weeks, every three weeks, every four weeks, every five weeks, every six weeks, every seven weeks, or every eight weeks. The nucleic acid or conjugated nucleic acid may be for use subcutaneously or intravenously.

In cells and/or subjects treated with or receiving a nucleic acid or conjugated nucleic acid of the present invention, the target gene expression may be inhibited compared to untreated cells and/or subjects by at least about 30%, 35%, 40%, 45%, 50%, 55%, 60%, 65%, 70%, 75%, 80%, 85%, 90%, 95%, 98%, or 100%. The level of inhibition may allow treatment of a disease associated with target gene expression or overexpression, or may allow further investigation into the functions of the target gene product.

The target gene may be Factor VII, Eg5, PCSK9, TPX2, apoB, SAA, TTR, RSV, PDGF beta gene, Erb-B gene, Src gene, CRK gene, GRB2 gene, RAS gene, MEKK gene, JNK gene, RAF gene, Erkl/2 gene, PCNA(p21) gene, MYB gene, JU gene, FOS gene, BCL-2 gene, hepcidin, Activated Protein C, Cyclin D gene, VEGF gene, EGFR gene, Cyclin A gene, Cyclin E gene, WNT-1 gene, beta-catenin gene, c-MET gene, PKC gene, NFKB gene, STAT3 gene, survivin gene, Her2/Neu gene, topoisomerase I gene, topoisomerase II alpha gene, mutations in the p73 gene, mutations in the p21(WAF I/CIPI) gene, mutations in the p27(KIPI) gene, mutations in the PPM ID gene, mutations in the RAS gene, mutations in the caveolin I gene, mutations in the MIB I gene, mutations in the MTAI gene, mutations in the M68 gene, mutations in tumor suppressor genes, and mutations in the p53 tumor suppressor gene. In particular, the target gene may be TMPRSS6 or ALDH2.

A further aspect of the invention relates to nucleic acid of the invention in the manufacture of a medicament for treating a disease or disorder.

Also included in the invention is a method of treating a disease or disorder comprising administration of a pharmaceutical composition comprising an nucleic acid as described herein, to an individual in need of treatment. The nucleic acid composition may be administered twice every week, once every week, every two weeks, every three weeks, every four weeks, every five weeks, every six weeks, every seven weeks, or every eight weeks. The nucleic acid may be administered to the subject subcutaneously or intravenously.

In one embodiment, a subject is administered an initial dose and one or more maintenance doses of a a nucleic acid or conjugated nucleic acid. The maintenance dose or doses can be the same or lower than the initial dose, e.g., one-half less of the initial dose. The maintenance doses are, for example, administered no more than once every 2, 5, 10, or 30 days. The treatment regimen may last for a period of time which will vary depending upon the nature of the particular disease, its severity and the overall condition of the patient.

In one embodiment, the composition includes a plurality of nucleic acid agent species. In another embodiment, the nucleic acid agent species has sequences that are non-overlapping and non-adjacent to another species with respect to a naturally occurring target sequence. In another embodiment, the plurality of nucleic acid agent species is specific for different naturally occurring target genes. In another embodiment, the nucleic acid agent is allele specific.

The nucleic acid or conjugated nucleic acid of the present invention can also be administered or for use in combination with other therapeutic compounds, either administered separately or simultaneously, e.g. as a combined unit dose.

The nucleic acid or conjugated nucleic acid of the present invention can be produced using routine methods in the art including chemically synthesis or expressing the nucleic acid either in vitro (e.g., run off transcription) or in vivo. For example, using solid phase chemical synthesis or using an expression vector. In one embodiment, the expression vector can produce the nucleic acid of the invention in a target cell. Methods for the synthesis of the nucleic acid described herein are known to persons skilled in the art.

In one aspect the invention relates to a nucleic acid capable of inhibiting the expression of a target gene, the nucleic acid comprising a first strand and a second strand, wherein the first stand and second strand are at least partially complementary with one another, wherein said first strand is at least partially complementary to RNA transcribed from a portion of said target gene; and wherein said first strand and/or said second strand include modified nucleotides. Further preferred features of the nucleic acid disclosed herein are as follows:

A nucleic acid for inhibiting expression of a target gene in a cell, comprising at least one duplex region that comprises at least a portion of a first strand and at least a portion of a second strand that is at least partially complementary to the first strand, wherein said first strand is at least partially complementary to at least a portion of RNA transcribed from said target gene, wherein said first strand includes modified nucleotides or unmodified nucleotides at a plurality of positions in order to facilitate processing of the nucleic acid by RISC.

In one aspect "facilitate processing by RISC" means that the nucleic acid can be processed by RISC, for example any modification present will permit the nucleic acid to be processed by RISC, suitably such that SiRNA activity can take place.

A nucleic acid as disclosed herein, wherein the nucleotides at positions 2 and 14 from the 5' end of the first strand are not modified with a 2' O-methyl modification, and the nucleotide on the second strand which corresponds to position 13 of the first strand is not modified with a 2' O-methyl modification.

A nucleotide on the second strand that "corresponds to" a position on the first strand is suitably the nucleotide that base pairs with that nucleotide on the first strand.

In one aspect the nucleotide on the second strand which corresponds to position 13 of the first strand is the nucleotide that forms a base pair with position 13 of the first strand.
In one aspect the nucleotide on the second strand which corresponds to position 11 of the first strand is the nucleotide that forms a base pair with position 11 of the first strand.
In one aspect the nucleotide on the second strand which corresponds to position 12 of the first strand is the nucleotide that forms a base pair with position 12 of the first strand.
This nomenclature may be applied to other positions of the second strand.

For example, in a 19-mer nucleic acid which is double stranded and blunt ended, position 13 of the first strand would pair with position 7 of the second strand. Position 11 of the first strand would pair with position 9 of the second strand. This nomenclature may be applied to other positions of the second strand.

The nucleotide that corresponds to position 13 of the first strand is suitably position 13 of the second strand, counting from the 3' of the second strand, starting from the first nucleotide of the double stranded region. Likewise position 11 of the second strand is suitably the 11th nucleotide from the 3' of the second strand, starting from the first nucleotide of the double stranded region. This nomenclature may be applied to other positions of the second strand.

In one aspect, in the case of a partially complementary first and second strand, the nucleotide on the second strand that "corresponds to" a position on the first strand may not necessarily form a base pair if that position is the position in which there is a mismatch, but the principle of the nomenclature still applies.

Preferred is a first and second strand that are fully complementary over the duplex region (ignoring any overhang regions) and there are no mismatches within the double stranded region of the nucleic acid.

Also preferred are :
A nucleic acid as disclosed herein, wherein the nucleotides at positions 2 and 14 from the 5' end of the first strand are not modified with a 2' O-methyl modification, and the nucleotide on the second strand which corresponds to position 11 of the first strand is not modified with a 2' O-methyl modification.

A nucleic acid as disclosed herein, wherein the nucleotides at positions 2 and 14 from the 5' end of the first strand are not modified with a 2' O-methyl modification, and the nucleotides on the second strand which corresponds to position 11 and 13 of the first strand are not modified with a 2' O-methyl modification.

In one aspect the nucleotide on the second strand which corresponds to position 12 of the first strand is not modified with a 2' O-methyl modification. This limitation on the nucleic acid may be seen with any other limitation described herein.

Therefore another aspect of the invention is a nucleic acid as disclosed herein, wherein the nucleotides at positions 2 and 14 from the 5' end of the first strand are not modified with a 2' O-methyl modification, and the nucleotides on the second strand which corresponds to position 11-13 of the first strand are not modified with a 2' O-methyl modification.

A nucleic acid as disclosed herein, wherein the nucleotides at positions 2 and 14 from the 5' end of the first strand are not modified with a 2' O-methyl modification, and the nucleotides on the second strand which correspond to position 11, or 13, or 11 and 13, or 11-13 of the first strand are modified with a 2' fluoro modification.

A nucleic acid as disclosed herein, wherein the nucleotides at positions 2 and 14 from the 5' end of the first strand are modified with a 2' fluoro modification, and the nucleotides on the second strand which correspond to position 11, or 13, or 11 and 13, or 11-13 of the first strand are not modified with a 2' O-methyl modification

A nucleic acid as disclosed herein, wherein the nucleotides at positions 2 and 14 from the 5' end of the first strand are modified with a 2' fluoro modification, and the nucleotides on the second strand which correspond to position 11, or 13, or 11 and 13, or 11-13 of the first strand are modified with a 2' fluoro modification.

A nucleic acid as disclosed herein wherein greater than 50% of the nucleotides of the first and/or second strand comprise a 2' O-methyl modification, such as greater than 55%, 60%, 65%, 70%, 75%, 80%, or 85%, or more, of the first and/or second strand comprise a 2' O-methyl modification, preferably measured as a percentage of the total nucleotides of both the first and second strands.

A nucleic acid as disclosed herein wherein greater than 50% of the nucleotides of the first and/or second strand comprise a naturally occurring RNA modification, such as wherein greater than 55%, 60%, 65%, 70%, 75%, 80%, or 85% or more of the first and/or second strands comprise such a modification, preferably measured as a percentage of the total nucleotides of both the first and second strands. Suitable naturally occurring modifications include, as well as 2 O' methyl, other 2' sugar modifications, in particular a 2' H modification resulting in a DNA nucleotide.

A nucleic acid as disclosed herein comprising no more than 20%, such as no more than 15% such as more than 10%, of nucleotides which have 2' modifications that are not 2' O methyl modifications on the first and/or second strand, preferably as a percentage of the total nucleotides of both the first and second strands.

A nucleic acid as disclosed herein comprising no more than 20%, (such as no more than 15% or no more than 10%) of 2' fluoro modifications on the first and/or second strand, preferably as a percentage of the total nucleotides of both strands.

A nucleic acid as disclosed herein, wherein all nucleotides are modified with a 2' O-methyl modification except positions 2 and 14 from the 5' end of the first strand and the nucleotides on the second strand which correspond to position 11, or 13, or 11 and 13, or 11-13 of the first strand. Preferably the nucleotides that are not modified with 2' O-methyl are modified with fluoro at the 2' position.

Preferred is a nucleic acid as disclosed herein wherein all nucleotides of the nucleic acid are modified at the 2' position of the sugar. Preferably these nucleotides are modified with a 2'-fluoro modification where the modification is not a 2' O-Methyl modification.

Nucleic acids of the invention may comprise one or more nucleotides modified at the 2' position with a 2' H, and therefore having a DNA nucleotide within the nucleic acid. Nucleic acids of the invention may comprise DNA nucleotides at positions 2 and/or 14 of the first strand counting from the 5' end of the first strand. Nucleic acids may comprise DNA nucleotides on the second strand which correspond to position 11, or 13, or 11 and 13, or 11-13 of the first strand.

In one aspect there is no more than one DNA per nucleic acid of the invention.

Nucleic acids of the invention may comprise one or more LNA nucleotides. Nucleic acids of the invention may comprise LNA nucleotides at positions 2 and/or 14 of the first strand counting from the 5' end of the first strand. Nucleic acids may comprise LNA on the second strand which correspond to position 11, or 13, or 11 and 13, or 11-13 of the first strand.

Preferably the nucleic acid as disclosed herein is an SiRNA.

In one aspect the nucleic acid is modified on the first strand with alternating 2-O methyl modifications and 2 fluoro modifications, and positions 2 and 14 (starting from the 5' end) are modified with 2' fluoro. Preferably the second strand is modified with 2' fluoro modifications at nucleotides on the second strand which correspond to position 11, or 13, or 11 and 13, or 11-13 of the first strand. Preferably the second strand is modified with 2' fluoro modifications at positions 11-13 counting from the 3' end starting at the first position of the complementary (double stranded) region, and the remaining modifications are naturally occurring modifications, preferably 2' O-methyl.

In one aspect the nucleic acid of the invention comprise one or more inverted ribonucleotides, preferably an inverted adenine, using a 5'-5' linkage or a 3'-3' linkage, preferably a 3'-3' linkage at the 3' end of the second strand.

In one aspect the nucleic acid comprises one or more phosphorodithioate linkages, such as 1, 2, 3 or 4 phosphorodithioate linkages. Preferably there are up to 4 phosphorodithioate linkages, eg one each at the 5' and 3' ends of the first and second strands.

The term **"first RNA strand"** as used herein means the antisense strand, and these terms are used interchangeably throughout the application.

The term **"second RNA strand"** as used herein means the sense strand, and these terms are used interchangeably throughout the application.

Preferably the antisense strand has at least 1 phosphorodithioate modification at each end. Preferably the antisense strand has 1-3 phosphorodithioate modifications at each end. Most preferably the antisense strand has 2 phosphorodithioate modifications at each end. Preferably the sense strand has at least 1 phosphorodithioate modification at the 3' end. Preferably the sense strand has 1-3 phosphorodithioate modifications at the 3' end. Most preferably the sense strand has 2 phosphorodithioate modifications at the 3' end.

All the features of the nucleic acids can be combined with all other aspects of the invention disclosed herein.

In particular, preferred are nucleic acids which are SiRNA molecules wherein the nucleotides at positions 2 and 14 from the 5' end of the first strand are not modified with a 2' O-methyl modification, and the nucleic acid comprises one or more or all of:
(i) an inverted nucleotide, preferably a 3'-3' linkage at the 3' end of the second strand;
(ii) one or more phosphorodithioate linkages;
(iii) the second strand nucleotide corresponding to position 11 or 13 of the first strand is not modified with a 2' O-methyl modification, preferably wherein one or both of these positions comprise a 2' fluoro modification
(iv) the nucleic acid comprises at least 80% of all nucleotides having a 2'-O-methly modification
(v) the nucleic acid comprises no more than 20% of nucleotides which have 2' fluoro modifications.

Also provided by the present invention is a nucleic acid as disclosed herein, wherein the nucleotides at positions 2 and 14 from the 5' end of the first strand and the nucleotides at positions 7 and/or 9, or 7 - 9 from the 5' end of the second strand are modified with a 2' fluoro modification, and at least 90% of the remaining nucleotides are 2'-O methyl modified or comprise another naturally occurring 2' modification.

Specific preferred examples, for a blunt double stranded 19 base nucleic acid, with no overhang, are:
A nucleic acid as disclosed herein, wherein the nucleotides at positions 2 and 14 from the 5' end of the first strand are not modified with a 2' O-methyl modification, and the nucleotide at position 7 from the 5' end of the second strand is not modified with a 2' O-methyl modification.

A nucleic acid as disclosed herein, wherein the nucleotides at positions 2 and 14 from the 5' end of the first strand are not modified with a 2' O-methyl modification, and the nucleotide at position 9 from the 5' end of the second strand is not modified with a 2' O-methyl modification

A nucleic acid as disclosed herein, wherein the nucleotides at positions 2 and 14 from the 5' end of the first strand are not modified with a 2' O-methyl modification, and the nucleotides at position 7 and 9 from the 5' end of the second strand are not modified with a 2' O-methyl modification.

A nucleic acid as disclosed herein, wherein the nucleotides at positions 2 and 14 from the 5' end of the first strand are not modified with a 2' O-methyl modification, and the nucleotides at positions 7 - 9 from the 5' end of the second strand are not modified with a 2' O-methyl modification.

A nucleic acid as disclosed herein, wherein the nucleotides at positions 2 and 14 from the 5' end of the first strand are not modified with a 2' O-methyl modification, and the nucleotides at positions 7 and/or 9, or 7-9 from the 5' end of the second strand are modified with a 2' fluoro modification.

A nucleic acid as disclosed herein, wherein the nucleotides at positions 2 and 14 from the 5' end of the first strand are modified with a 2' fluoro modification, and the nucleotides at positions 7 and/or 9, or 7 - 9 from the 5' end of the second strand are not modified with a 2' O-methyl modification

A nucleic acid as disclosed herein, wherein the nucleotides at positions 2 and 14 from the 5' end of the first strand are modified with a 2' fluoro modification, and the nucleotides at positions 7 and/or 9, or 7 - 9 from the 5' end of the second strand are modified with a 2' fluoro modification.

A nucleic acid as disclosed herein wherein greater than 50% of the nucleotides of the first and/or second strand comprise a 2' O-methyl modification, such as greater than 55%, 60%, 65%, 70%, 75%, 80%, or 85%, or more, of the first and/or second strand comprise a 2' O-methyl modification, preferably measured as a percentage of the total nucleotides of both the first and second strands.

A nucleic acid as disclosed herein wherein greater than 50% of the nucleotides of the first and/or second strand comprise a naturally occurring RNA modification, such as wherein greater than 55%, 60%, 65%, 70%, 75%, 80%, or 85% or more of the first and/or second strands comprise such a modification, preferably measured as a percentage of the total nucleotides of both the first and second strands. Suitable naturally occurring modifications include, as well as 2 O' methyl, other 2' sugar modifications, in particular a 2' H modification resulting in a DNA nucleotide.

A nucleic acid as disclosed herein comprising no more than 20%, such as no more than 15% such as more than 10%, of nucleotides which have 2' modifications that are not 2' O methyl modifications on the first and/or second strand, preferably as a percentage of the total nucleotides of both the first and second strands.

A nucleic acid as disclosed herein comprising no more than 20%, (such as no more than 15% or no more than 10%) of 2' fluoro modifications on the first and/or second strand, preferably as a percentage of the total nucleotides of both strands.

A nucleic acid as disclosed herein, wherein all nucleotides are modified with a 2' O-methyl modification except positions 2 and 14 from the 5' end of the first strand and the nucleotides at positions 7 and/or 9 from the 5' end of the second strand. Preferably the nucleotides that are not modified with 2' O-methyl are modified with fluoro at the 2' position.

A nucleic acid as disclosed herein, wherein all nucleotides are modified with a 2' O-methyl modification except positions 2 and 14 from the 5' end of the first strand and the nucleotides at positions 7 - 9 from the 5' end of the second strand. Preferably the nucleotides that are not modified with 2' O-methyl are modified with fluoro at the 2' position.

For a nucleic acid comprising a 20 base pair duplex region, the second strand preferably does not have a 2' O-methyl group at nucleotides 8 or 9 or 10 counting from the 5' end of the duplex corresponding to positions 13, 12, and 11 of the first strand respectively.

For a nucleic acid comprising a 21 base pair duplex region, the second strand preferably does not have a 2' O-methyl group at nucleotides 9 or 10 or 11 counting from the 5' end of the duplex corresponding to positions 13, 12,and 11 of the first strand respectively.

In one aspect the nucleic acid is not any one or more or all of Patisiran, Revusiran, Fitusiran, Cemdisiran, Givosiran, Inclisiran, lumasiran, Votrisiran, Cosdosiran and Teprasiran.
These have the sequences below.
Patisiran 3'CAUUGGUUCUCAUAAGGUA 5'
   5'GUAACCAAGAGUAUUCCAU 3'
Revusiran 3'-CUACCCUAAAGUACAUUGGUUCU- 5'
   5'-UGGGAUUUCAUGUAACCAAGA 3'
Fitusiran 3'-GACCAAUUGUGGUAAAUGAAGUU- 5'
   5'-GGUUAACACCAUUUACUUCAA 3'
Cemdisiran 3'-TTUUUUCGUUCUAUAAAAAUAUUAU- 5'
   5'-AAGCAAGAUAUUUUUAUAAUA 3'
Givosiran 3'-UGGUCUUUCUCACAGAGUAGAAU 5'
   5'-CAGAAAGAGUGUCUCAUCUUA 3'
Inclisiran 3'-AAGAUCUGGACAAAACGAAAACA 5'
   5'-CUAGACCUGUTUUGCUUUUGU 3'

Sequences of these molecules are also available on the WHO website http://www.who.int/medicines/services/inn/en/

For example,
Cemdisian is
   duplex of [(2S,4R)-1-{1-[(2-acetamido-2-deoxy-β-Dgalactopyranosyl) oxy]-16,16-bis({3-[(3-{5-[(2-acetamido-2-deoxy-β-D-galactopyranosyl)oxy]pentanamido} propyl)amino]-3-oxopropoxy}methyl)-5,11,18-trioxo-14-oxa-6,10,17-triazanonacosan-29-oyl}-4-hydroxypyrrolidin-2-yl]methyl hydrogen all-P-ambo-2'-O-methyl-Pthioadenylyl-( 3'→5')-2'-O-methyl-P-thioadenylyl-(3'→5')-2'-deoxy-2'-fluoroguanylyl-(3'→5')-2'-O-methylcytidylyl-(3'→5')-2'-deoxy-2'-fluoroadenylyl-(3'→5')- 2'-Omethyladenylyl-( 3'→5')-2'-deoxy-2'-fluoroguanylyl-(3'→5')-2'-O-methyladenylyl-(3'→5')-2'-deoxy-2'-fluorouridylyl-(3'→5')-2'-deoxy-2'-fluoroadenylyl-(3'→5')-2'-deoxy-2'-fluorouridylyl-(3'→5')-2'-O-methyluridylyl-(3'→5')-2'-deoxy-2'-fluorouridylyl-(3'→5')-2'-O-methyluridylyl-(3'→5')-2'-Omethyluridylyl-( 3'→5')-2'-deoxy-2'-fluoroadenylyl-(3'→5')-2'-O-methyluridylyl-(3'→5')-2'-deoxy-2'-fluoroadenylyl-(3'→5')-2'-O-methyladenylyl-(3'→5')-2'-O-methyluridylyl-(3'→5')-2'-0-methyl-3'-adenylate and all-P-ambo-thymidylyl-(5'→3')-thymidylyl-(5'→3')-2'-O-methyl-P-thiouridylyl-(5'→3')-2'-Omethyl-P-thiouridylyl-(5'→3')-2'-O-methyluridylyl-(5'→3')-2'-O-methyluridylyl-(5'-3')-2'-O-methylcytidylyl-(5'→3')-2'-deoxy-2'-fluoroguanylyl-(5'→3')-2'-O-methyluridylyl-(5'→3')-2'-deoxy-2'-fluorouridylyl-(5'→3')-2'-Omethylcytidylyl-( 5'→3')-2'-deoxy-2'-fluorouridylyl-(5'→3')-2'-O-methyladenylyl-(5'→3')-2'-O-methyluridylyl-(5'→3')-2'-O-methyladenylyl-(5'→3')-2'-deoxy-2'-fluoroadenylyl-(5'→3')-2'-O-methyladenylyl-(5'→3')-2'-deoxy-2'-fluoroadenylyl-(5'→3')-2'-O-methyladenylyl-(5'→3')-2'-Omethyluridylyl-( 5'→3')-2'-deoxy-2'-fluoroadenylyl-(5'→3')-2'-O-methyluridylyl-(5'→3')-2'-deoxy-2'-fluoro-P-thiouridylyl-(5'→3')-2'-deoxy-2'-fluoro-P-thioadenylyl-(5'→3')-2'-Omethyluridine
Patisiran is
   RNA duplex of guanylyl-(3'→5')-2'-O-methyluridylyl-(3'→5')-adenylyl-(3'→5')-adenylyl-(3'→5')-2'-O-methylcytidylyl-(3'→5')-2'-Omethylcytidylyl-( 3'→5')-adenylyl-(3'→5')-adenylyl-(3'→5')-guanylyl-(3'→5')-adenylyl-(3'→5')-guanylyl-(3'→5')-2'-O-methyluridylyl-(3'→5')-adenylyl-(3'→5')-2'-O-methyluridylyl-(3'→5')-2'-Omethyluridylyl-3'→5')-2'-O-methylcytidylyl-(3'→5')-2'-Omethylcytidylyl-( 3'→5')-adenylyl-(3'→5')-2'-O-methyluridylyl-(3'→5')-thymidylyl-(3'→5')-thymidine with thymidylyl-(5'→3')-thymidylyl-(5'→3')-cytidylyl-(5'→3')-adenylyl-(5'→3')-2'-O-methyluridylyl-(5'→3')-uridylyl-(5'→3')-guanylyl-(5'→3')-guanylyl-(5'→3')-uridylyl-(5'→3')-uridylyl-(5'→3')-cytidylyl-(5'→3')-uridylyl-(5'→3')-cytidylyl-(5'→3')-adenylyl-(5'→3')-2'-O-methyluridylyl-(5'→3')-adenylyl-(5'→3')-adenylyl-(5'→3')-guanylyl-(5'→3')-guanylyl-(5'→3')-uridylyl-(5'→3')-adenosine
Inclisiran is
   duplex of [(2S,4R)-1-{1-[(2-acetamido-2-deoxy-p-Dgalactopyranosyl) oxy]-16, 16-bis({3-[(3-{5-[(2-acetamido-2-deoxy-β-D-galactopyranosyl)oxy]pentanamido} propyl)amino]-3-oxopropoxy}methyl)-5,11,18-trioxo-14-oxa-6,10,17-triazanonacosan-29-oyl}-4-hydroxypyrrolidin-2-yl]methyl hydrogen all-P-ambo-2'-O-methyl-Pthiocytidylyl-( 3'→5')-2'-O-methyl-P-thiouridylyl-(3'→5')-2'-Omethyladenylyl-( 3'→5')-2'-O-methylguanylyl-(3'→5')-2'-Omethyladenylyl-( 3'→5')-2'-O-methylcytidylyl-(3'→5')-2'-deoxy-2'-fluorocytidylyl-(3'→5')-2'-O-methyluridylyl-(3'→5')-2'-deoxy-2'-fluoroguanylyl-(3'→5')-2'-O-methyluridylyl-(3'→5')-thymidylyl-(3'→5')-2'-O-methyluridylyl-(3'→5')-2'-Omethyluridylyl-( 3'→5')-2'-O-methylguanylyl-(3'→5')-2'-Omethylcytidylyl-( 3'→5')-2'-O-methyluridylyl-(3'→5')-2'-Omethyluridylyl-( 3'→5')-2'-O-methyluridylyl-(3'→5')-2'-Omethyluridylyl-( 3'→5')-2'-O-methylguanylyl-(3'→5')-2'-Omethyl-3'-uridylate and all-P-ambo-2'-O-methyl-P-thioadenylyl-(5'→3')-2'-O-methyl-P-thioadenylyl-(5'→3')-2'-O-methylguanylyl-(5'→3')-2'-O-methyladenylyl-(5'→3')-2'-O-methyluridylyl-(5'→3')-2'-deoxy-2'-fluorocytidylyl-(5'→3')-2'-O-methyluridylyl-(5'→3')-2'-deoxy-2'-fluoroguanylyl-(5'→3')-2'-O-methylguanylyl-(5'→3')-2'-deoxy-2'-fluoroadenylyl-(5'→3')-2'-O-methylcytidylyl-(5'→3')-2'-deoxy-2'-fluoroadenylyl-(5'→3')-2'-Omethyladenylyl-( 5'→3')-2'-deoxy-2'-fluoroadenylyl-(5'→3')-2'-O-methyladenylyl-(5'→3')-2'-deoxy-2'-fluorocytidylyl-(5'→3')-2'-O-methylguanylyl-(5'→3')-2'-deoxy-2'-fluoroadenylyl-(5'→3')-2'-deoxy-2'-fluoroadenylyl-(5'→3')-2'-deoxy-2'-fluoroadenylyl-(5'→3')-2'-O-methyl-P-thioadenylyl-(5'→3')-2'-deoxy-2'-fluoro-P-thiocytidylyl-(5'→3')-2'-O-methyladenosine
Givosiran is
   duplex of [(2S,4R)-1-{1-[(2-acetamido-2-deoxy-β-Dgalactopyranosyl) oxy]-16, 16-bis({3-[(3-{5-[(2-acetamido-2-deoxy-β-D-galactopyranosyl)oxy]pentanamido}propyl) amino]-3-oxopropoxy}methyl)-5,11,18-trioxo-14-oxa-6,10,17-triazanonacosan-29-oyl}-4-hydroxypyrrolidin-2-yl]methyl hydrogen all-P-ambo-2'-O-methyl-P-thiocytidylyl-(3'→5')-2'-O-methyl-P-thioadenylyl-(3'→5')-2'-Omethylguanylyl-( 3'→5')-2'-O-methyladenylyl-(3'→5')-2'-Omethyladenylyl-( 3'→5')-2'-O-methyladenylyl-(3'→5')-2'-deoxy-2'-fluoroguanylyl-(3'→5')-2'-O-methyladenylyl-(3'→5')-2'-deoxy-2'-fluoroguanylyl-(3'→5')-2'-Omethyluridylyl-( 3'→5')-2'-deoxy-2'-fluoroguanylyl-(3'→5')-2'-O-methyluridylyl-(3'→5')-2'-deoxy-2'-fluorocytidylyl-(3'→5')-2'-O-methyluridylyl-(3'→5')-2'-deoxy-2'-fluorocytidylyl-(3'→5')-2'-O-methyladenylyl-(3'→5')-2'-O-methyluridylyl-(3'→5')-2'-O-methylcytidylyl-(3'→5')-2'-O-methyluridylyl-(3'→5')-2'-O-methyluridylyl-(3'→5')-2'-O-methyl-3'-adenylate and all-P-ambo-2'-O-methyl-P-thiouridylyl-(5'→3')-2'-O-methyl-P-thioguanylyl-(5'→3')-2'-Omethylguanylyl-( 5'→3')-2'-deoxy-2'-fluorouridylyl-(5'→3')-2'-O-methylcytidylyl-(5'→3')-2'-deoxy-2'-fluorouridylyl-(5'→3')-2'-O-methyluridylyl-(5'→3')-2'-deoxy-2'-fluorouridylyl-(5'→3')-2'-O-methylcytidylyl-(5'→3')-2'-deoxy-2'-fluorouridylyl-(5'→3')-2'-O-methylcytidylyl-(5'→3')-2'-deoxy-2'-fluoroadenylyl-(5'→3')-2'-Omethylcytidylyl-( 5'→3')-2'-deoxy-2'-fluoroadenylyl-(5'→3')-2'-O-methylguanylyl-(5'→3')-2'-deoxy-2'-fluoroadenylyl-(5'→3')-2'-O-methylguanylyl-(5'→3')-2'-deoxy-2'-fluorouridylyl-(5'→3')-2'-O-methyladenylyl-(5'→3')-2'-deoxy-2'-fluoroguanylyl-(5'→3')-2'-deoxy-2'-fluoro-P-thioadenylyl-(5'→3')-2'-deoxy-2'-fluoro-P-thioadenylyl-(5'→3')-2'-O-methyluridine
Revusiran is [(2S,4R)-1-{30-(2-acetamido-2-deoxy-β-D-galactopyranosyl)-14,14-bis[16-(2-acetamido-2-deoxy-β-Dgalactopyranosyl)-5,11-dioxo-2,16-dioxa-6,10-diazahexadecyl]-12,19,25-trioxo-16,30-dioxa-13,20,24-triazatriacontanoyl}-4-hydroxypyrrolidin-2-yl]methyl hydrogen 2'-deoxy-2'-fluorouridylyl-(3'→5')-2'-O-methylguanylyl-(3'→5')-2'-deoxy-2'-fluoroguanylyl-(3'→5')-2'-O-methylguanylyl-(3'→5')-2'-deoxy-2'-fluoroadenylyl-(3'→5')-2'-O-methyluridylyl-(3'→5')-2'-deoxy-2'-fluorouridylyl-(3'→5')-2'-O-methyluridylyl-(3'→5')-2'-deoxy-2'-fluorocytidylyl-(3'→5')-2'-deoxy-2'-fluoroadenylyl-(3'→5')-2'-deoxy-2'-fluorouridylyl-(3'→5')-2'-O-methylguanylyl-(3'→5')-2'-deoxy-2'-fluorouridylyl-(3'→5')-2'-O-methyladenylyl-(3'→5')-2'-O-methyladenylyl-(3'→5')-2'-O-methylcytidylyl-(3'→5')-2'-deoxy-2'-fluorocytidylyl-(3'→5')-2'-O-methyladenylyl-(3'→5')-2'-deoxy-2'-fluoroadenylyl-(3'→5')-2'-O-methylguanylyl-(3'→5')-2'-deoxy-2'-fluoroadenylate duplex with 2'-O-methyl-Pthiocytidylyl-( 5'→3')-2'-deoxy-2'-fluoro-P-thiouridylyl-(5'→3')-2'-O-methyladenylyl-(5'→3')-2'-deoxy-2'-fluorocytidylyl-(5'→3')-2'-O-methylcytidylyl-(5'→3')-2'-deoxy-2'-fluorocytidylyl-(5'→3')-2'-O-methyluridylyl-(5'→3')-2'-deoxy-2'-fluoroadenylyl-(5'→3')-2'-O-methyladenylyl-(5'→3')-2'-deoxy-2'-fluoroadenylyl-(5'→3')-2'-O-methylguanylyl-(5'→3')-2'-O-methyluridylyl-(5'→3')-2'-Omethyladenylyl-( 5'→3')-2'-deoxy-2'-fluorocytidylyl-(5'→3')-2'-Omethyladenylyl-( 5'→3')-2'-deoxy-2'-fluorouridylyl-(5'→3')-2'-deoxy-2'-fluorouridylyl-(5'→3')-2'-deoxy-2'-fluoroguanylyl-(5'→3')-2'-O-methylguanylyl-(5'→3')-2'-deoxy-2'-fluorouridylyl-(5'→3')-2'-O-methyluridylyl-(5'→3')-2'-deoxy-2'-fluorocytidylyl-(5'→3')-2'-O-methyluridine
Fitusiran is
   duplex of [(2S,4R)-1-{30-(2-acetamido-2-deoxy-β-Dgalactopyranosyl)-14,14-bis[16-(2-acetamido-2-deoxy-β-Dgalactopyranosyl)-5,11-dioxo-2,16-dioxa-6,10-diazahexadecyl]-12,19,25-trioxo-16,30-dioxa-13,20,24-triazatriacontanoyl}-4-hydroxypyrrolidin-2-yl]methyl hydrogen (P-RS)-2'-deoxy-2'-fluoro-P-thioguanylyl-(3'→5')-(P-RS)-2'-O-methyl-P-thioguanylyl-(3'→5')-2'-deoxy-2'-fluorouridylyl-(3'→5')-2'-O-methyluridylyl-(3'→5')-2'-deoxy-2'-fluoroadenylyl-(3'→5')-2'-O-methyladenylyl-(3'→5')-2'-deoxy-2'-fluorocytidylyl-(3'→5')-2'-O-methyladenylyl-(3'→5')-2'-deoxy-2'-fluorocytidylyl-(3'→5')-2'-deoxy-2'-fluorocytidylyl-(3'→5')-2'-deoxy-2'-fluoroadenylyl-(3'→5')-2'-O-methyluridylyl-(3'→5')-2'-deoxy-2'-fluorouridylyl-(3'→5')-2'-O-methyluridylyl-(3'→5')-2'-deoxy-2'-fluoroadenylyl-(3'→5')-2'-O-methylcytidylyl-(3'→5')-2'-deoxy-2'-fluorouridylyl-(3'→5')-2'-O-methyluridylyl-(3'→5')-2'-deoxy-2'-fluorocytidylyl-(3'→5')-2'-O-methyladenylyl-(3'→5')-2'-deoxy-2'-fluoroadenylate and and (P-RS)-2'-O-methyl-P-thiouridylyl-(3'→5')-(P-RS)-2'-deoxy-2'-fluoro-P-thiouridylyl-(3'→5')-2'-O-methylguanylyl-(3'→5')-2'-deoxy-2'-fluoroadenylyl-(3'→5')-2'-Omethyladenylyl-( 3'→5')-2'-deoxy-2'-fluoroguanylyl-(3'→5')-2'-O-methyluridylyl-(3'→5')-2'-deoxy-2'-fluoroadenylyl-(3'→5')-2'-O-methyladenylyl-(3'→5')-2'-deoxy-2'-fluoroadenylyl-(3'→5')-2'-O-methyluridylyl-(3'→5')-2'-Omethylguanylyl-( 3'→5')-2'-O-methylguanylyl-(3'→5')-2'-deoxy-2'-fluorouridylyl-(3'→5')-2'-O-methylguanylyl-(3'→5')-2'-deoxy-2'-fluorouridylyl-(3'→5')-2'-O-methyluridylyl-(3'→5')-2'-deoxy-2'-fluoroadenylyl-(3'→5')-2'-Omethyladenylyl-( 3'→5')-2'-deoxy-2'-fluorocytidylyl-(3'→5')-(P-RS)-2'-O-methyl-P-thiocytidylyl-(3'→5')-(P-RS)-2'-Omethyl-P-thioadenylyl-(3'→5')-2'-O-methylguanosine
Lumasiran is
   {(2S,4R)-1-{1-[(2-acetamido-2-deoxy-β-Dgalactopyranosyl) oxy]-16,16-bis-({3-[(3-{5-[(2-acetamido-2-deoxy-β-Dgalactopyranosyl) oxy]pentanamido}propyl)amino]-3-oxopropoxy}methyl)-5,11,18-trioxo-14-oxa-6,10,17-triazanonacosan-29-oyl}-4-hydroxypyrrolidin-2-yl}methyl hydrogen all-P-ambo-2'-O-methyl-P-thioguanylyl-(3'-5')-2'-O-methyl-P-thioadenylyl-(3'→5')-2'-O-methylcytidylyl-(3'→5')-2'-O-methyluridylyl-(3'→5')-2'-O-methyluridylyl-(3'→5')-2'-O-methyluridylyl-(3'→5')-2'-deoxy-2'-fluorocytidylyl-(3'→5')-2'-O-methyladenylyl-(3'→5')-2'-deoxy-2'-fluorouridylyl-(3'→5')-2'-deoxy-2'-fluorocytidylyl-(3'→5')-2'-deoxy-2'-fluorocytidylyl-(3'→5')-2'-Omethyluridylyl-( 3'→5')-2'-O-methylguanylyl-(3'→5')-2'-Omethylguanylyl-( 3'→5')-2'-O-methyladenylyl-(3'→5')-2'-Omethyladenylyl-( 3'→5')-2'-O-methyladenylyl-(3'→5')-2'-Omethyluridylyl-( 3'→5')-2'-O-methyladenylyl-(3'→5')-2'-Omethyluridylyl-( 3'→5')-2'-O-methyl-3'-adenylate duplex with all-P-ambo-2'-O-methyl-P-thioadenylyl-(5'→3')-2'-O-methyl-P-thiocytidylyl-(5'→3')-2'-O-methylcytidylyl-(5'→3')-2'-O-methyluridylyl-(5'→3')-2'-O-methylguanylyl-(5'→3')-2'-O-methyladenylyl-(5'→3')-2'-O-methyladenylyl-(5'→3')-2'-deoxy-2'-fluoroadenylyl-(5'→3')-2'-Omethylguanylyl-( 5'→3')-2'-deoxy-2'-fluorouridylyl-(5'→3')-2'-O-methyladenylyl-(5'→3')-2'-O-methylguanylyl-(5'→3')-2'-O-methylguanylyl-(5'→3')-2'-O-methyladenylyl-(5'→3')-2'-deoxy-2'-fluorocytidylyl-(5'→3')-2'-deoxy-2'-fluorocytidylyl-(5'→3')-2'-O-methyluridylyl-(5'→3')-2'-deoxy-2'-fluorouridylyl-(5'→3')-2'-O-methyluridylyl-(5'→3')-2'-Omethyladenylyl-( 5'→3')-2'-O-methyl-P-thiouridylyl-(5'→3')-2'-deoxy-2'-fluoro-P-thioadenylyl-(5'→3')-2'-Omethyluridine 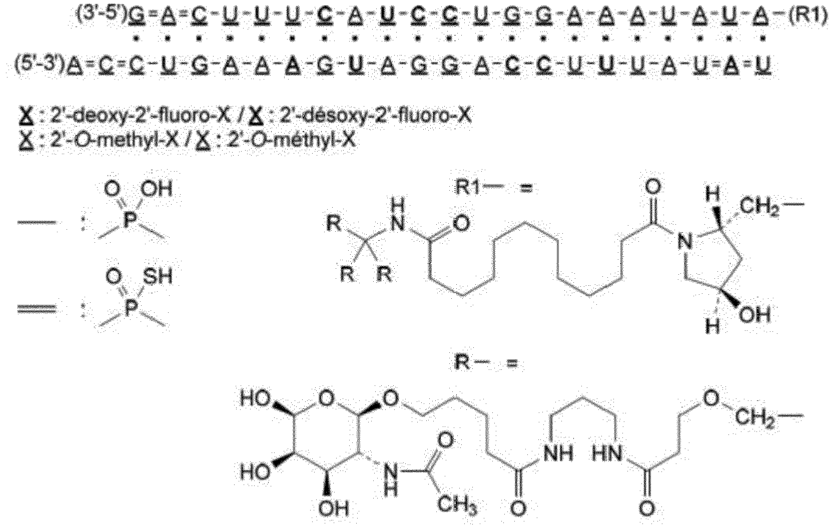
Votrisiran is:
   {(2S,4R)-1-{1-[(2-acetamido-2-deoxy-β-Dgalactopyranosyl) oxy]-16,16-bis-({3-[(3-{5-[(2-acetamido-2-deoxy-β-Dgalactopyranosyl) oxy]pentanamido}propyl)amino]-3-oxopropoxy}methyl)-5,11,18-trioxo-14-oxa-6,10,17-triazanonacosan-29-oyl}-4-hydroxypyrrolidin-2-yl}methyl hydrogen all-P-ambo-2'-O-methyl-P-thiouridylyl-(3'→5')-2'-O-methyl-P-thioguanylyl-(3'→5')-2'-O-methylguanylyl-(3'→5')-2'-O-methylguanylyl-(3'→5')-2'-O-methyladenylyl-(3'→5')-2'-O-methyluridylyl-(3'→5')-2'-deoxy-2'-fluorouridylyl-(3'→5')-2'-O-methyluridylyl-(3'→5')-2'-deoxy-2'-fluorocytidylyl-(3'→5')-2'-deoxy-2'-fluoroadenylyl-(3'→5')-2'-deoxy-2'-fluorouridylyl-(3'→5')-2'-O-methylguanylyl-(3'→5')-2'-O-methyluridylyl-(3'→5')-2'-O-methyladenylyl-(3'→5')-2'-O-methyladenylyl-(3'→5')-2'-O-methylcytidylyl-(3'→5')-2'-O-methylcytidylyl-(3'→5')-2'-O-methyladenylyl-(3'→5')-2'-O-methyladenylyl-(3'→5')-2'-O-methylguanylyl-(3'→5')-2'-O-methyl-3'-adenylate duplex with all-P-ambo-2'-O-methyl-P-thiocytidylyl-(5'→3')-2'-O-methyl-P-thiouridylyl-(5'→3')-2'-O-methyladenylyl-(5'→3')-2'-O-methylcytidylyl-(5'→3')-2'-O-methylcytidylyl-(5'→3')-2'-O-methylcytidylyl-(5'→3')-2'-O-methyluridylyl-(5'→3')-2'-deoxy-2'-fluoroadenylyl-(5'→3')-2'-O-methyladenylyl-(5'→3')-2'-deoxy-2'-fluoroadenylyl-(5'→3')-2'-O-methylguanylyl-(5'→3')-2'-O-methyluridylyl-(5'→3')-2'-O-methyladenylyl-(5'→3')-2'-O-methylcytidylyl-(5'→3')-2'-deoxy-2'-fluoroadenylyl-(5'→3')-2'-O-methyluridylyl-(5'→3')-2'-Omethyluridylyl-( 5'→3')-2'-deoxy-2'-fluoroguanylyl-(5'→3')-2'-O-methylguanylyl-(5'→3')-2'-O-methyluridylyl-(5'→3')-2'-Omethyl-P-thiouridylyl-(5'→3')-2'-deoxy-2'-fluoro-Pthiocytidylyl-( 5'→3')-2'-O-methyluridine 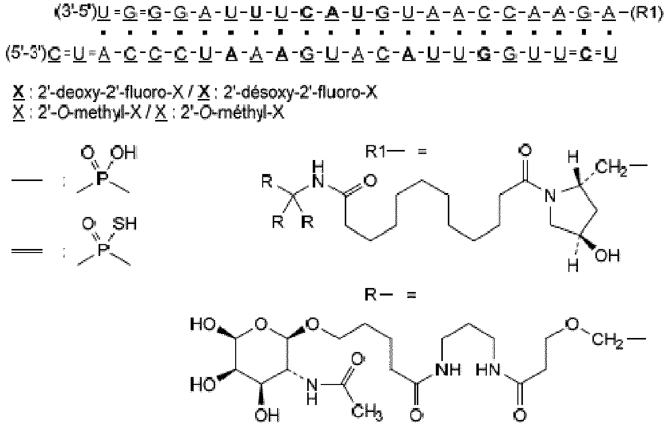
Cosdosiran is:
   adenylyl-(3'→5')-2'-O-methylguanylyl-(3'→5')-guanylyl-(3'→5')-2'-O-methyladenylyl-(3'→5')-guanylyl-(3'→5')-2'-Omethyluridylyl-3'→5')-uridylyl-(3'→5')-2'-O-methylcytidylyl-(3'→5')-cytidylyl-(3'→5')-adenylyl-(3'→5')-2'-Omethylcytidylyl-( 3'→5')-adenylyl-(3'→5')-2'-Omethyluridylyl-( 3'→5')-uridylyl-(3'→5')-2'-O-methylcytidylyl-(3'→5')-uridylyl-(3'→5')-2'-O-methylguanylyl-(3'→5')-guanylyl-(3'→5')-2'-O-methylcytidine duplex with [(2R,3S)-3-hydroxyoxolan-2-yl]methyl hydrogen uridylyl-(5'→3')-2'-deoxycytidylyl-(5'→3')-cytidylyl-(5'→3')-uridylyl-(5'→3')-cytidylyl-(5'→3')-adenylyl-(5'→3')-adenylyl-(5'→3')-guanylyl-(5'→3')-guanylyl-(5'→3')-uridylyl-(5'→3')-guanylyl-(5'→3')-uridylyl-(5'→3')-adenylyl-(5'→3')-adenylyl-(5'→3')-guanylyl-(5'→3')-adenylyl-(5'→3')-cytidylyl-(5'→3')-cytidylyl-(5'→3')-5'-guanylate
Teprasiran is:
   guanylyl-(3'→5')-2'-O-methyladenylyl-(3'→5')-guanylyl-(3'→5')-2'-O-methyladenylyl-(3'→5')-adenylyl-(3'→5')-2'-Omethyluridylyl-( 3'→5')-adenylyl-(3'→5')-2'-O-methyluridylyl-(3'→5')-uridylyl-(3'→5')-2'-O-methyluridylyl-(3'→5')-cytidylyl-(3'→5')-2'-O-methyladenylyl-(3'→5')-cytidylyl-(3'→5')-2'-O-methylcytidylyl-(3'→5')-cytidylyl-(3'→5')-2'-Omethyluridylyl-( 3'→5')-uridylyl-(3'→5')-2'-O-methylcytidylyl-(3'→5')-adenosine duplex with 2'-O-methyluridylyl-(3'→5')-guanylyl-(3'→5')-2'-O-methyladenylyl-(3'→5')-adenylyl-(3'→5')-2'-O-methylguanylyl-(3'→5')-guanylyl-(3'→5')-2'-Omethylguanylyl-( 3'→5')-uridylyl-(3'→5')-2'-Omethylguanylyl-( 3'→5')-adenylyl-(3'→5')-2'-Omethyladenylyl-( 3'→5')-adenylyl-(3'→5')-2'-Omethyluridylyl-( 3'→5')-adenylyl-(3'→5')-2'-O-methyluridylyl-(3'→5')-uridylyl-(3'→5')-2'-O-methylcytidylyl-(3'→5')-uridylyl-(3'→5')-2'-O-methylcytidine

### Screening method

The present invention relates to a method for selecting an siRNA molecule having RNAi [RNA interference] activity, the method comprising the steps of
(1) modification of a nucleotide on the second (sense) strand which corresponds to position 11 or 13 of the first (antisense) strand to introduce a 2' O methyl modification on the nucleotide sugar,
(2) determining whether the 2' O methyl modification reduces the activity of the siRNA compared with an siRNA differing only in that there is no modification at that 2' position, or having a 2' fluoro modification at the same nucleotide; and
(3) if the modification affects SiRNA activity, select a molecule which does not have a modification which is a 2' O methyl modification at the position which reduces activity of the siRNA, and/or modify the 2' position of the nucleotide with 2 fluoro, or use an unmodified nucleotide at that position.

A method for selecting an siRNA molecule having RNAi [RNA interference] activity, the method comprising the steps of
(1) modification of a nucleotide on the second (sense) strand which corresponds to position 11 or 13 of the first (antisense) strand to introduce a nucleotide having a modification other than a 2' fluoro modification on the nucleotide sugar,
(2) determining whether the modification reduces the activity of the siRNA compared with an siRNA differing only in that there is no modification at that 2' position, or having a 2' fluoro modification at the same nucleotide; and
(3) if the modification affects siRNA activity, select a molecule which does not have that modification which reduces activity of the siRNA, and/or modify the 2' position of the nucleotide with 2' fluoro modification, or use an unmodified nucleotide at that position of the siRNA.

In another aspect, the method involves a method as above, but the modification of a nucleotide on the second (sense) strand which corresponds to position 11 or 13 of the first (antisense) introduces a modification at the 2' position which is bulky, such as a modification that is bulkier than fluoro or an unmodified RNA nucleotide, and the assessment on siRNA activity is made in respect of that 2' bulky modification. The O - methyl modification is considered to be bulky, and in one aspect the modification is at least as bulky or large as 2 O methyl.

The modification may be a modification containing a group which is of 2'-O-(2-Methoxyethyl), 2'-O-allyl, 2'-O-DNP, 2'-CE, 2'-EA, 2'-AEM, 2'-APM and 2'-GE.

The present invention also relates to a method for selecting an siRNA molecule having RNAi [RNA interference] activity, the method comprising the steps of
1 modifying an siRNA molecule by changing the 2' position of the sugar moiety of each base to be a 2' O-methyl residue;
2 assessing activity of the SiRNA to identify positions in which 2' O-methyl modification reduces the activity of the siRNA compared with the same siRNA having a 2' fluoro modification at the same position, or compared with an siRNA having no modification at the same position; and
3 selecting an siRNA molecule modified with 2' O methyl at all positions which do not show reduced activity in step 2.

In another aspect, the method involves the method as above, but the modification of a 2' nucleotide position in step (1) introduces a modification at the 2' position which is bulky, such as a modification that is bulkier than fluoro or an unmodified RNA nucleotide, and the assessment on siRNA activity is made in respect of that 2' bulky modification. The modification may be a modification containing a group which is of 2-O-(2-Methoxyethyl), 2'-O-allyl, 2'-O-DNP, 2'-CE, 2'-EA, 2'-AEM, 2'-APM and 2'-GE.

RNAi activity may be assessed by any method disclosed herein, or others known in the art.

The invention also relates to a method for providing an siRNA molecule, comprising formulating into a pharmaceutical composition those siRNA molecules comprising 2' O methyl modifications at all positions which do not show reduced siRNA activity vs a 2' fluoro modification at the same position. Suitably these siRNA molecules have been identified using one of the above methods. For example, the siRNA having may be linked to GalNac or other targeting ligand as described herein.

The siRNA is preferably a nucleic acid of the present invention, as described above.

In the preceding disclosure, a 2' -NH2 modification may be used as an alternative to a 2' fluoro modification in any aspect of the invention, especially in siRNA modification. A 2' fluoro modification is however more preferred.

In any aspect or embodiment of the invention described herein, the nucleic acid (or use, method, composition or any other teaching involving a nucleic acid) comprises one DNA nucleotide at position 2, or 14, counting from the 5' end of the first strand and additionally, and/ or alternatively, comprises 1, 2, or 3 DNA nucleotides at positions on the second strand which correspond to any one, two or three positions 11, 12 and 13 of the first strand.

In any aspect or embodiment of the invention described herein, the nucleic acid (or use, method, composition or any other teaching involving a nucleic acid) comprises a DNA nucleotide, or a 2'fluoro modification, at a position or positions on the second strand which corresponds to positions 11- 13 of the first strand. More than one modification may be present.

In any aspect or embodiment of the invention described herein, the nucleic acid - or any use, method, composition or any other teaching involving a nucleic acid herein - does not comprise a bulky modification group - such as a 2'-O methyl group - at any one of position 2, or 14, or both, counting from the 5' end of the first strand, and/ or at any position of the second strand which corresponds to positions 11, 12 or 13 of the first strand. A bulky modification may be any modification that is bigger than an 'OH group, for example, at the 2' position of the RNA sugar moiety.

In further embodiments of the invention, the invention relates to any nucleic acid, conjugated nucleic acid, nucleic acid for use, method, composition or use according to any disclosure herein, wherein the terminal nucleotide at the 3' end of at least one of the first strand and the second strand is an inverted nucleotide and is attached to the adjacent nucleotide via the 3' carbon of the terminal nucleotide and the 3' carbon of the adjacent nucleotide and/ or the terminal nucleotide at the 5' end of at least one of the first strand and the second strand is an inverted nucleotide and is attached to the adjacent nucleotide via the 5' carbon of the terminal nucleotide and the 5' carbon of the adjacent nucleotide,
optionally wherein
the 3' and/or 5' inverted nucleotide of the first and/or second strand is attached to the adjacent nucleotide via a phosphate group by way of a phosphodiester linkage; or
the 3' and/or 5' inverted nucleotide of the first and/or second strand is attached to the adjacent nucleotide via a phosphorothioate group or
the 3' and/or 5' inverted nucleotide of the first and/or second strand is attached to the adjacent nucleotide via a phosphorodithioate group.

In further embodiments of the invention, the invention relates to any nucleic acid, conjugated nucleic acid, nucleic acid for use, method, composition or use according to any disclosure herein, wherein the nucleic acid comprises a phosphorodithioate linkage, optionally wherein the linkage is between the 2 most 5' nucleosides and/or the 2 most 3' nucleosides of the second strand, and/or optionally wherein the nucleic acid additionally does not comprise any internal phosphorothioate linkages.

The invention also relates to any first strand or any second strand of nucleic acid as disclosed herein, which comprises no more than 2 base changes when compared to the specific sequence ID provided. For example, one base may be changed within any sequence.

In one embodiment, the change may be made to the 5' most nucleotide of the antisense (first) strand. In one embodiment, the change may be made to the 3' most nucleotide of the antisense (first) strand. In one embodiment, the change may be made to the 5' most nucleotide of the sense (second) strand. In one embodiment, the change may be made to the 3' most nucleotide of the sense (second) strand.

In one embodiment, the change is made to the 5' most nucleotide of the antisense (first) strand. The base of the 5' nucleotide may be changed to any other nucleotide. An A or a U at the 5' end are preferred, and an A or a U are taught herein as the potential 5' terminal base for all of the antisense sequences disclosed herein

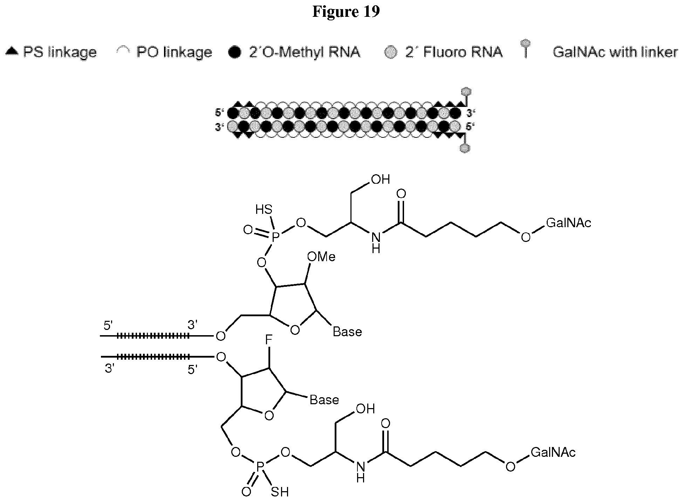

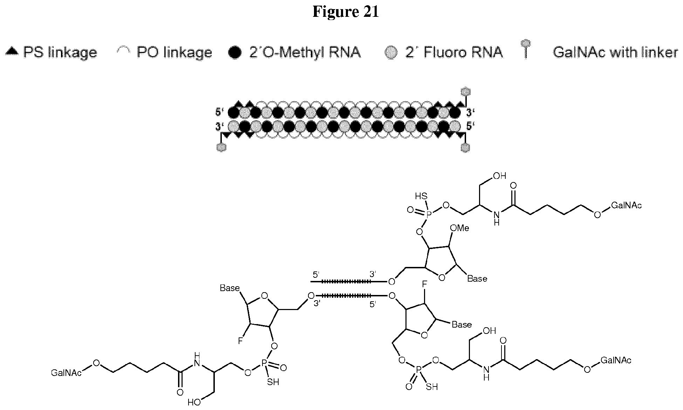

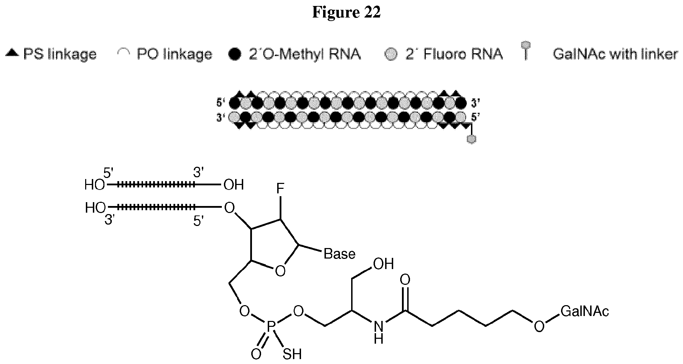

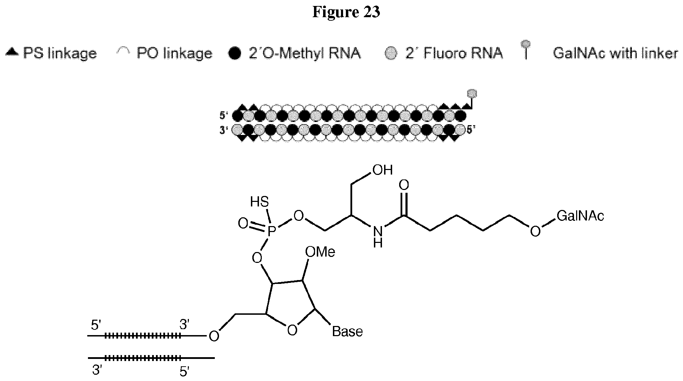

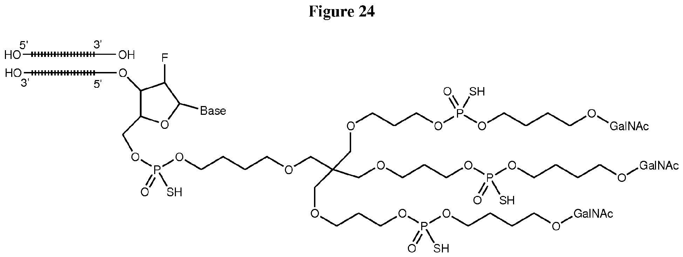

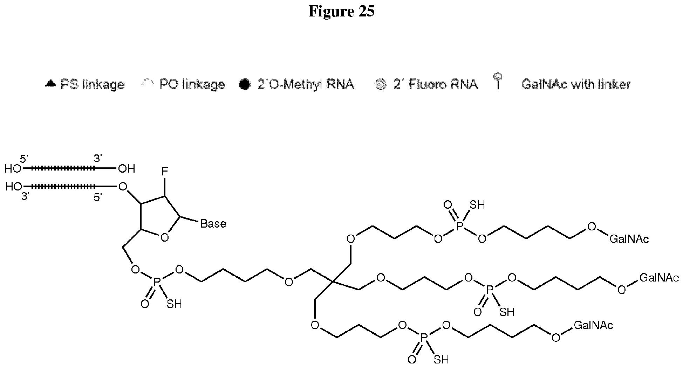

The invention will now be described with reference to the following non-limiting figures and examples in which:
Figure 1a and 1b show in vitro knockdown activity of siRNAs that are modified with 2'-OMe or 2'-OH at position 14 of the first strand;
Figure 2a and 2b show in vitro knockdown activity of siRNAs with 2'-OMe or 2'-OH at position 14 of the first strand;
Figure 3a and 3b show in vitro knockdown activity of siRNAs with 2'-OMe or 2'-OH at positions 2, 3 and 4 of the first strand;
Figure 4a and 4b show in vitro knockdown activity of siRNAs with 2'-OMe and 2'-OH at positions 2, 3 and 4 of the first strand;
Figure 5a and 5b show in vitro knockdown activity of siRNAs with 2'-OMe and 2'-F at position 2 of the first strand;
Figure 6a-c show knockdown activity of differently modified ALDH2 variants derived from one sequence;
Figure 7a and b show knockdown activity of differently modified ALDH2 sequences;
Figure 8a and b show knockdown activity of differently modified ALDH2 sequences;
Figure 9a and b show knockdown activity of differently modified DGAT2 sequences;
Figure 10a and b show the effect of DNA modifications at certain positions of a TMPRSS6 siRNA sequence;
Figure 11a and b show the effect of LNA modifications at certain positions of a TMPRSS6 siRNA sequence;
Figure 12a - d show knockdown activity of GalNAc conjugates with different modification patterns both in liposomal transfections and receptor-mediated uptake;
Figure 13a and b show tolerance for DNA modification at more than one position in a TMPRSS6 siRNA sequence;
Figure 14a and b disclose tolerance for DNA in an siRNA targeting ALDH2 ;
Figure 15 and b disclose tolerance for DNA in a second siRNA targeting ALDH2 ;
Figure 16a and b disclose tolerance for DNA in an siRNA targeting DGAT2 ;
Figure 17a and b disclose the effect of 2-O-MOE at certain positions; and
Figure 18a and b disclose tolerance for 2'-OMe in an siRNA targeting GHR.
**Figure 19** depicts Conjugate 1.
**Figure 20** depicts Conjugate 2.
**Figure 21** depicts Conjugate 3.
**Figure 22** depicts Reference Conjugate 1.
**Figure 23** depicts Reference Conjugate 2.
**Figure 24** depicts Reference Conjugate 3.
**Figure 25** depicts Reference Conjugate 4.
   In each of Figures 19-25, the top strand is the antisense strand and the bottom strand is the sense strand i.e. 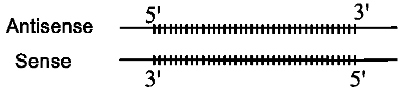 In addition, to show more clearly the connection between the nucleic acid and ligand portions, the nucleotide at the end of the respective conjugated strands is drawn in full.

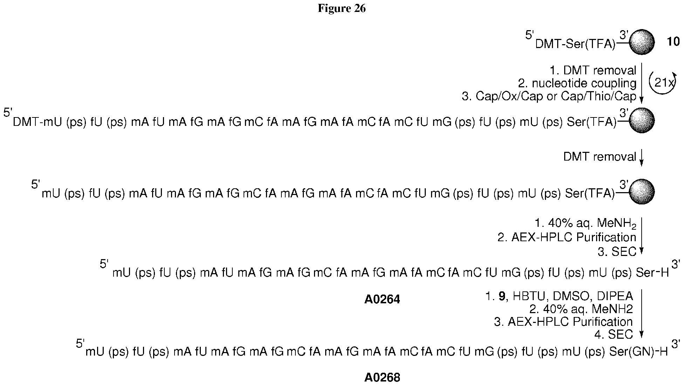

**Figure 26** shows the synthesis of **A0268** which is a 3' mono-GalNAc conjugated single stranded oligonucleotide and is the starting material in the synthesis of Conjugate 1 and Conjugate 3. (ps) denotes phosphorothioate linkage.

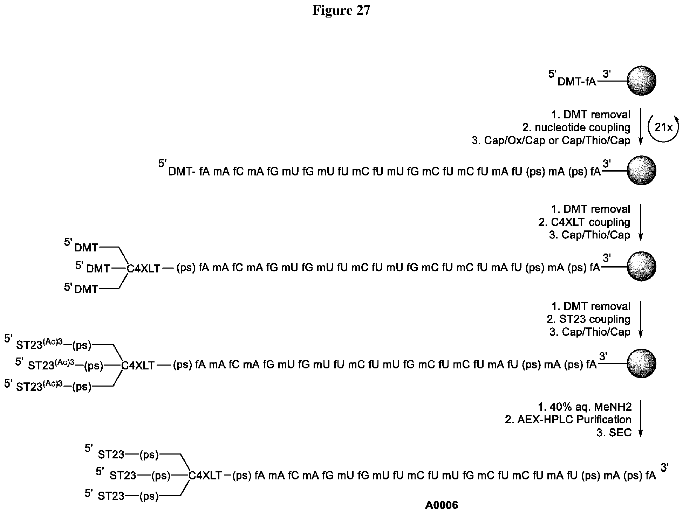

**Figure 27** shows the synthesis of **A0006** which is a 5' tri-antennary GalNAc conjugated single stranded oligonucleotide used for the synthesis of Reference Conjugate 4. (ps) denotes phosphorothioate linkage.

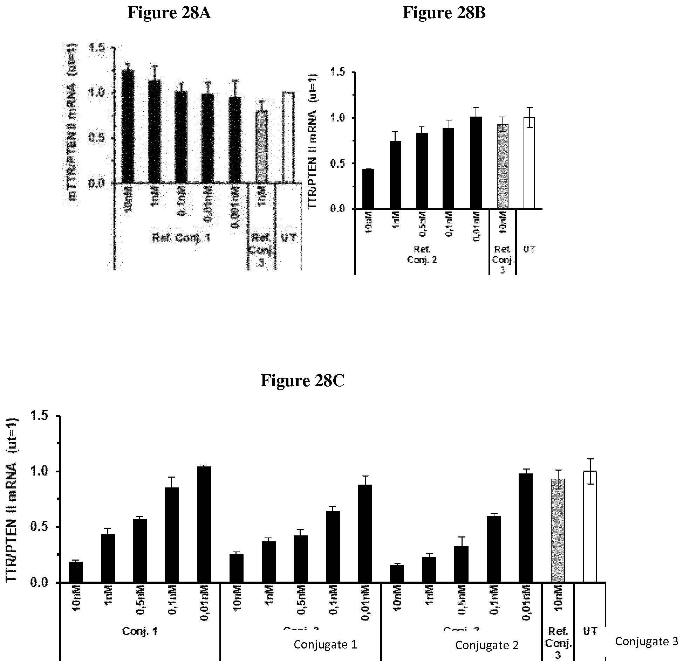

**Figure 28** illustrates the *in vitro* determination of TTR knockdown. In particular, **Figure 28A** shows the *in vitro* determination of TTR knockdown by Reference Conjugates (RC) 1 and 3 as well as the untreated control "UT"; **Figure 28B** shows the *in vitro* determination of TTR knockdown by Reference Conjugates (RC) 2 and 3, as well as by the untreated control "UT"; and **Figure 28C** shows the *in vitro* determination of TTR knockdown by Conjugates 1, 2 and 3, as well as by RC3 and untreated control "UT". Reference Conjugates 1 and 2 represent comparator conjugates. Reference Conjugate 3 represents a non-targeting GalNAc siRNA and "untreated" ("UT") represents untreated cells. Both RC3 and UT are negative controls. mRNA level were normalised against Ptenll.

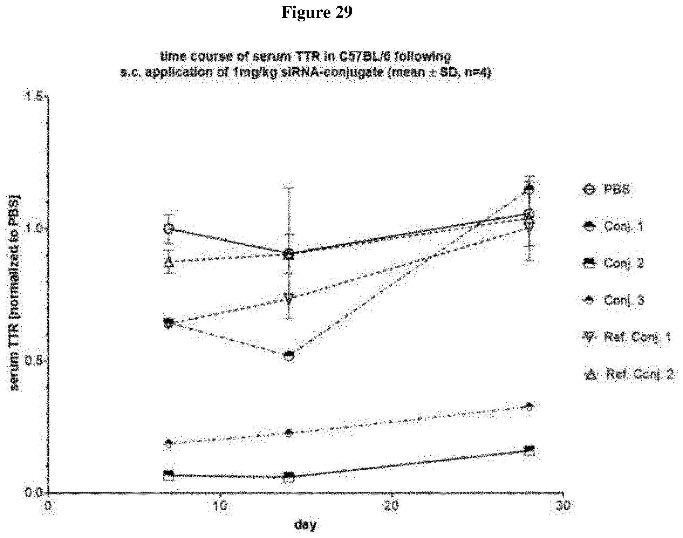

**Figure 29** shows a time course of serum TTR in c57BL/6 mice cohorts of n=4 at 7, 14, and 27 days post s.c. treatment with 1mg/kg - Conjugates 1-3, Reference Conjugates (RC) 1 and 2 and mock treated (PBS) individuals.

### Examples

### Example 1

All Oligonucleotides were either obtained from a commercial oligonucleotide manufacturer (Eurogentec, Belgium) or synthesized on an AKTA oligopilot 10 synthesiser (GE Healthcare) in a 10µmol scale using phosphoramidite chemistry. Commercially available base loaded CPG solid support (500A, 50µmol/g), 2'O-Methyl RNA phosphoramidites and 2'Fluoro DNA phosphoramidites (ChemGenes and LinkTech) were used according to the manufacturers recommended procedures. Amidite coupling was performed using 0.1 M solutions of the phosphoramidite in acetonitrile in presence of 0.3 M benzylthiotetrazole (BTT) activator. As ancillary reagents, 0.05 M I₂ in pyridine/H₂O (9/1, v/v) as oxidizer, 40% Ac2O in acetonitrile as CapA, 20% N-methylimidazole in acetonitrile as CapB, 3% dichloroacetic acid in toluene as DMT removal and 20% diethylamine in acetonitrile as final wash were used (EMP Biotech). EDITH (LinkTech) was used as thiolation reagent. Acetonitrile (<20 ppm H₂O) was purchased from EMP Biotech. All other reagents and solvents were commercially available and used in standard reagent quality.

ST23 is a GalNac C4 phosphoramidite (structure components as below, described in WO2017/174657):

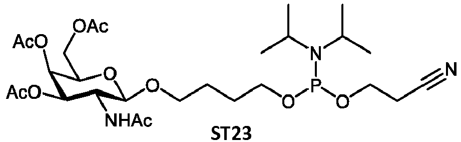

ST41 is as follows (and as described in WO2017/174657):

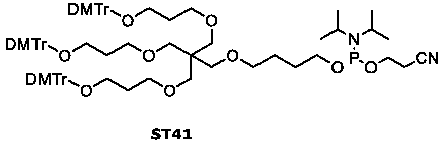

Phosphorothioates were introduced using 50 mM EDITH in acetonitrile. All oligonucleotides were synthesised in DMT-off mode. Diethylamine wash was performed upon completion of the assembly of the oligonucleotide chain on the solid support.

The single strands were cleaved off the CPG and all remaining protective groups were removed by using in 40% aq. methylamine solution (90 min, RT). The crude product was concentrated and purified by Ion exchange chromatography (Resource Q, 6mL, GE Healthcare) on a AKTA Pure HPLC System (GE Healthcare) using a Sodium chloride gradient (10 mM Tris buffer pH = 7.5, 10% acetonitril). Product containing fractions were analysed and pooled and concentrated. Salt removal was achieved by size exclusion chromatography (Zetadex, EMP Biotech). Finally, the individual single strands were lyophilised.

For duplex formation, single strands were reconstituted in ∼2mg/mL concentration in water. Equimolar amounts of the respective single strands were added, mixed and heated to 80°C for 5min. After cooling the resulting siRNA was analyzed for full double strand formation by native IP-RP HPLC. Product solutions were stored at -20°C until further use.

### The present examples utilise 19mer SiRNAs, unless otherwise apparent from the description and figures.

### Example 2

The influence of 2'-OMe at position 14 of the first strand on siRNA activity was tested using a sequence targeting mouse CLIC4. CLC01 is modified with alternating 2'-OMe/2'-OH. CLC15 is modified with 2'-OMe at position 14 of the first strand, whereas CLC16 is not modified with 2'-OMe at this position. All other positions in CLC15 and CLC16 are modified similarly. "UT" indicates an untreated sample the siRNA-treated samples were normalized to. "Luc" was used as non-targeting control.

The experiment was conducted in MS1. Cells were seeded at a density of 40,000 cells per 6-well 24 h before transfection, transfected with 5 nM siRNA and 1 µg/ml Atufect and lysed after 48 h. Total RNA was extracted and CLIC4 and PTEN mRNA levels were determined by Taqman qRT-PCR. Each bar represents mean ± SD of three technical replicates.

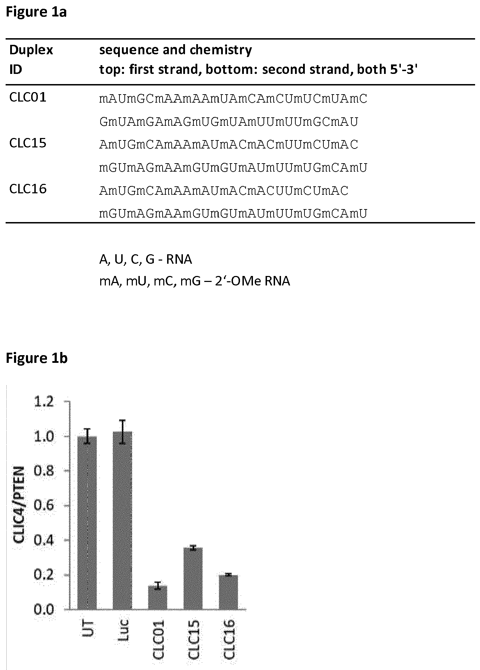

Data are shown in Figures 1a and 1b.

### Example 3

The influence of 2'-OMe at position 14 of the first strand on siRNA activity was tested using a sequence targeting mouse CLIC4. CLC01 is modified with alternating 2'-OMe/2'-OH. CLC22 is modified with 2'-OMe at position 4, 9 and 14 of the first strand, whereas CLC28 is modified with 2'-OMe at positions 4, 9 and 15 of the first strand. The second strands of CLC22 and CLC28 are modified similarly. "UT" indicates an untreated sample the siRNA-treated samples were normalized to. "Luc" was used as non-targeting control.

The experiment was conducted in MS1. Cells were seeded at a density of 40,000 cells per 6-well 24 h before transfection, transfected with 5 nM siRNA and 1 µg/ml Atufect and lysed after 48 h. Total RNA was extracted and CLIC4 and Actin mRNA levels were determined by Taqman qRT-PCR. Each bar represents mean ± SD of three technical replicates.

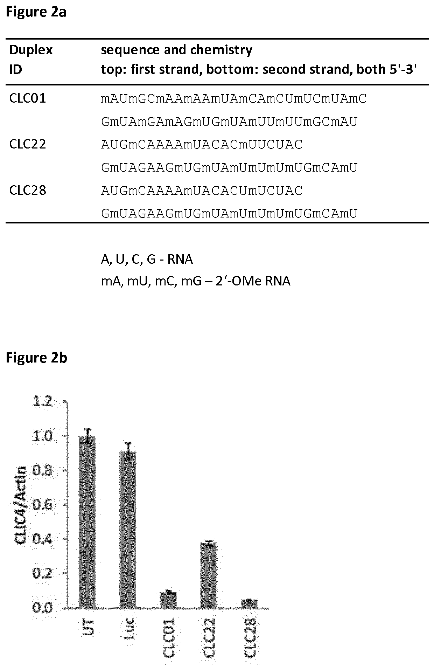

Data are shown in Figure 2a and 2b.

### Example 4

The influence of 2'-OMe at position 2 of the first strand on siRNA activity was tested using a sequence targeting mouse CLIC4. CLC56 is modified with 2'-OMe at position 2 and 4 of the first strand, and 2'-OH at position 3. In contrast, CLC57 has 2'-OH at positions 2 and 4, and 2'-OMe at position 3. All other positions of the first and second strand are modified similarly. "UT" indicates an untreated sample the siRNA-treated samples were normalized to. "Luc" was used as non-targeting control.

The experiment was conducted in MS1. Cells were seeded at a density of 40,000 cells per 6-well 24 h before transfection, transfected with 5 and 1 nM siRNA and 1 µg/ml Atufect and lysed after 48 h. Total RNA was extracted and CLIC4 and PTEN mRNA levels were determined by Taqman qRT-PCR. Each bar represents mean ± SD of three technical replicates.

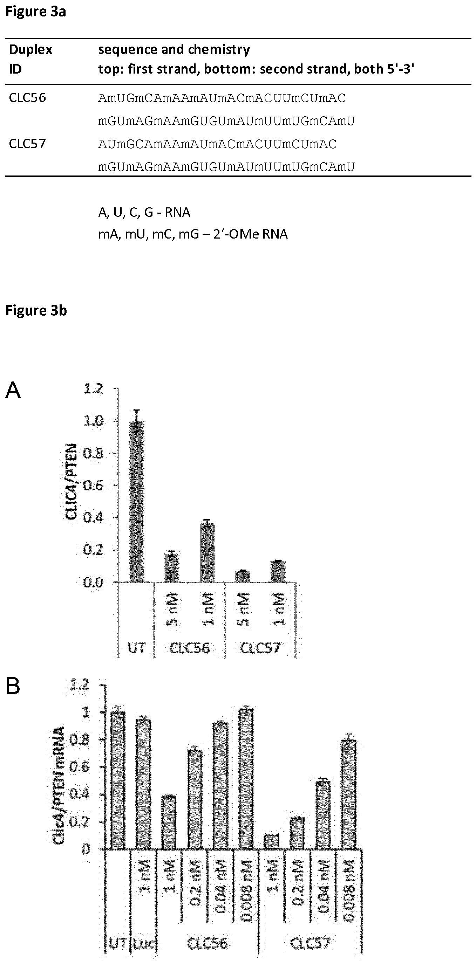

Data are shown in Figures 3a and 3b (A).

### Example 5

The influence of 2'-OMe at position 2 of the first strand on siRNA activity was tested using a sequence targeting mouse CLIC4. CLC56 is modified with 2'-OMe at position 2 and 4 of the first strand, and 2'-OH at position 3. In contrast, CLC57 has 2'-OH at positions 2 and 4, and 2'-OMe at position 3. All other positions of the first and second strand are modified similarly. "UT" indicates an untreated sample the siRNA-treated samples were normalized to. "Luc" was used as non-targeting control.

The experiment was conducted in MS1. Cells were seeded at a density of 40,000 cells per 6-well 24 h before transfection, transfected with 1 to 0.008 nM siRNA and 1 µg/ml Atufect and lysed after 48 h. Total RNA was extracted and CLIC4 and PTEN mRNA levels were determined by Taqman qRT-PCR. Each bar represents mean ± SD of three technical replicates.

Data are shown in Figures 3a and 3b (B).

### Example 6

The influence of 2'-OMe at position 2 of the first strand on siRNA activity was tested using a sequence targeting mouse CLIC4. CLC01 is modified with alternating 2'-OMe/2'-OH. CLC28 has 2'-OMe at position 4 of the first strand, whereas CLC59 has 2'-OMe at position 2 and CLC60 has 2'-OMe at position 3 of the first strand. All other positions of the first and second strand are modified similarly. "UT" indicates an untreated sample the siRNA-treated samples were normalized to. "Luc" was used as non-targeting control.

The experiment was conducted in MS1. Cells were seeded at a density of 40,000 cells per 6-well 24 h before transfection, transfected with 5 and 1 nM siRNA (A) or 1 to 0.008 nM siRNA (B) and 1 µg/ml Atufect and lysed after 48 h. Total RNA was extracted and CLIC4 and PTEN mRNA levels were determined by Taqman qRT-PCR. Each bar represents mean ± SD of three technical replicates.

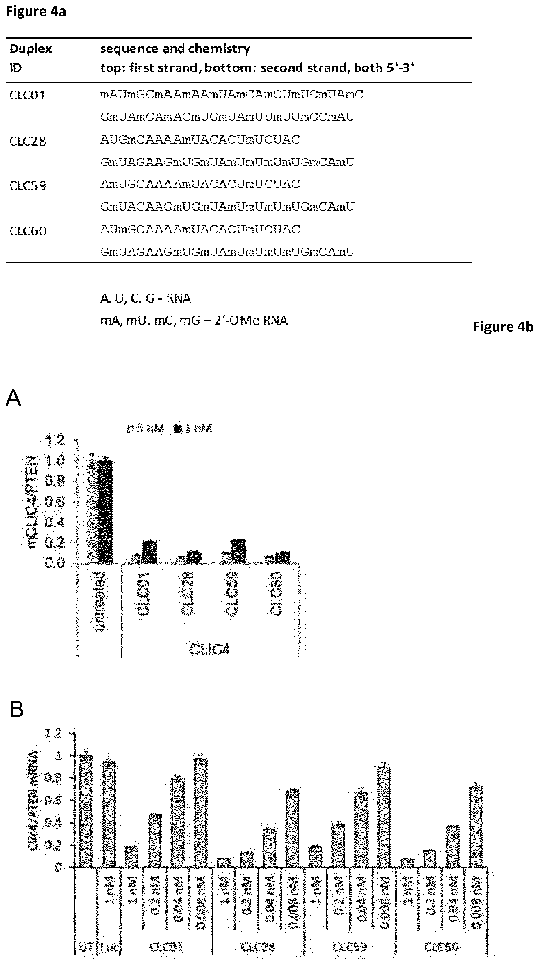

Data are shown in Figures 4a and 4b.

### Example 7

The influence of 2'-OMe at position 2 of the first strand on siRNA activity was tested using a sequence targeting human HFE2. In the first strand, HFE04 is modified with 2'-F at position 2 and 2'-OMe at position 3, whereas HFE06 is modified with 2'-OMe at position 2 and 2'-F at position 3. All other positions of the first and second strand are modified similarly. "UT" indicates an untreated sample the siRNA-treated samples were normalized to. "Luc" was used as non-targeting control.

The experiment was conducted in Hep3B. Cells were seeded at a density of 120,000 cells per 6-well 24 h before transfection, transfected with 1 nM siRNA and 1 µg/ml Atufect and lysed after 72 h. Total RNA was extracted and HFE2 and PTEN mRNA levels were determined by Taqman qRT-PCR. Each bar represents mean ± SD of three technical replicates.

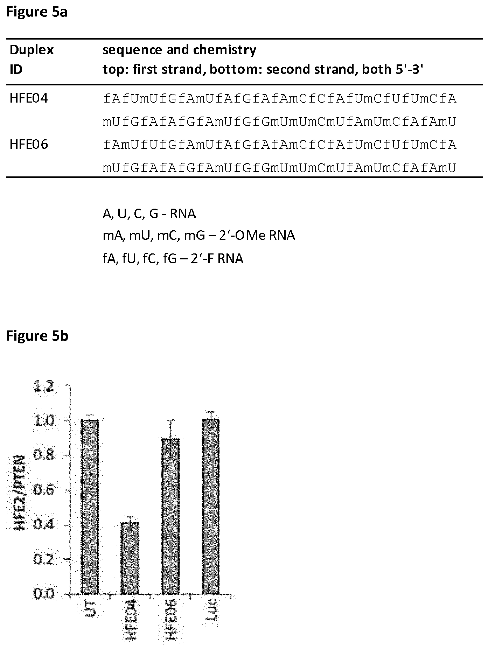

Data are shown in Figures 5a and 5b.

### Example 8

Examples 8a and 8b represent biological replicates of the same experiment.
**Example 8a** The tolerance for 2'-OMe was investigated by addressing one position at a time in the context of an alternating pattern (change 2'-OMe into 2'-F and vice versa). ALD01 is completely alternating, ALD13 - ALD21 contains 2'-F into 2'-Me changes at all even positions of the first strand, ALD22 - ALD31 contains 2'-OMe into 2'-F changes at all odd positions of the first strand, ALD32 - ALD41 contains 2'-F into 2'-OMe changes at all odd positions of the second strand, ALD42 - ALD50 contains 2'-OMe into 2'-F changes at all even positions of the second strand. ALD13 contains 2'-OMe at first strand position 2, ALD19 contains 2'-OMe at first strand position 14, ALD35 contains 2'-OMe at second position 7, ALD36 contains 2'-OMe at second strand position 9.

The experiment was conducted in Hep3B. Cells were seeded at a density of 150,000 cells per 6-well 24 h before transfection, transfected with 0.1 nM siRNA and 1 µg/ml Atufect and lysed after 48 h. Total RNA was extracted and ALDH2 and actin mRNA levels were determined by Taqman qRT-PCR. Each bar represents mean ± SD of three technical replicates.

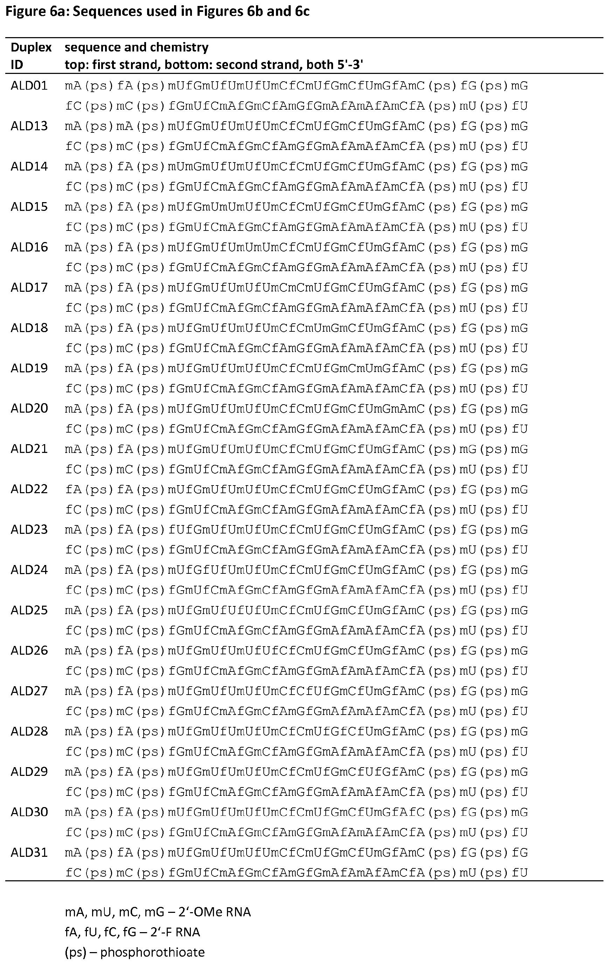

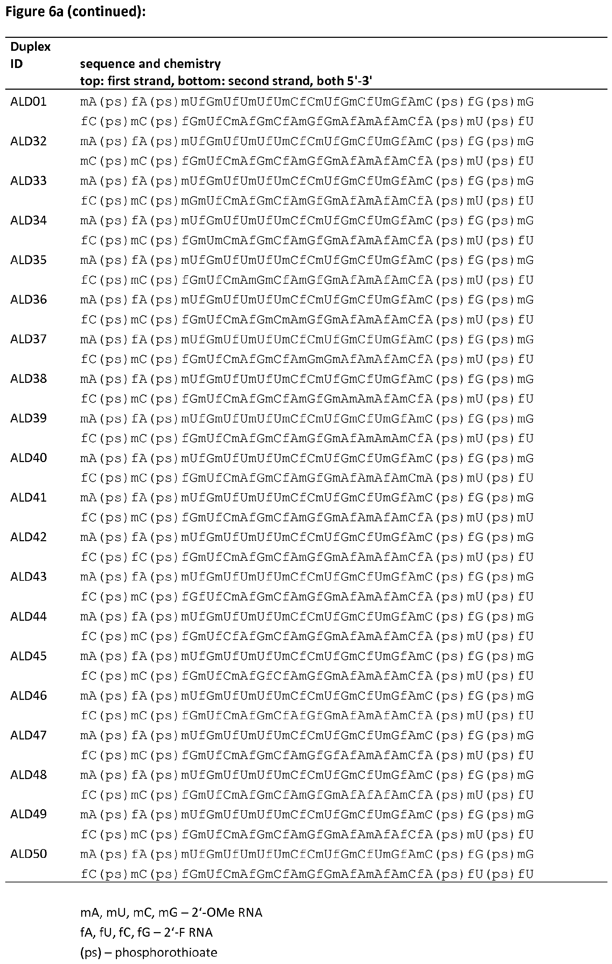

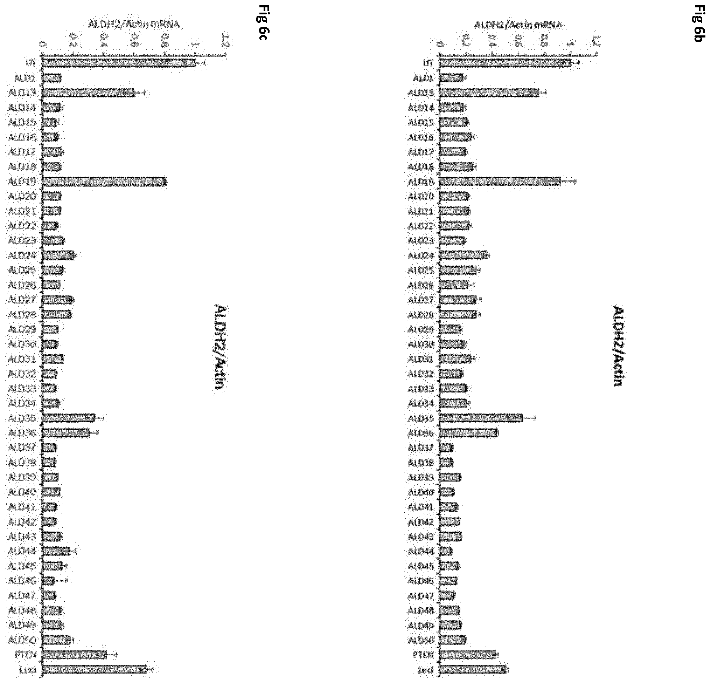

Sequences are listed in Figure 6a and results are shown in figure 6b.

### Example 8b:

The tolerance for 2'-OMe was investigated by addressing one position at a time in the context of an alternating pattern (change 2'-OMe into 2'-F and vice versa). ALD01 is completely alternating, ALD13 - ALD21 contains 2'-F into 2'-Me changes at all even positions of the first strand, ALD22 - ALD31 contains 2'-OMe into 2'-F changes at all odd positions of the first strand, ALD32 - ALD41 contains 2'-F into 2'-OMe changes at all odd positions of the second strand, ALD42 - ALD50 contains 2'-OMe into 2'-F changes at all even positions of the second strand. ALD13 contains 2'-OMe at first strand position 2, ALD19 contains 2'-OMe at first strand position 14, ALD35 contains 2'-OMe at second strand position 7, ALD36 contains 2'-OMe at second strand position 9.

The experiment was conducted in Hep3B. Cells were seeded at a density of 150,000 cells per 6-well 24 h before transfection, transfected with 0.1 nM siRNA and 1 µg/ml Atufect and lysed after 48 h. Total RNA was extracted and ALDH2 and actin mRNA levels were determined by Taqman qRT-PCR. Each bar represents mean ± SD of three technical replicates.

Sequences are listed in Figure 6a and results are shown in Figure 6c.

### Example 9

### Influence of modifications on the activity of two different ALDH2 siRNA sequences

Experiment 9-1 Tolerance of positions 2 and 14 for 2'-OMe in the first strand and tolerance of positions 7 and 9 for 2'-OMe in the second strand of an siRNA against ALDH2 was analysed. ALD58 contains alternating 2'-OMe/2'-F in both strands. ALD59 - ALD61 contain 2'-F at position 2 and/or 14 of the first strand with an all alternating second strand, whereas ALD62 - ALD64 contain 2'-F at position 7 and/or 9 of the second strand with an all alternating first strand. Positions 2 (ALD60) and 14 (ALD59) loose activity upon modification with 2'-OMe, whereas no 2'-OMe at position 2 and 14 restores activity (ALD61). Of the second strand, position 7 (ALD63) and position 9 (ALD62) loose activity upon modification with 2'OMe, whereas no 2'-OMe at positions 7 and 9 restores activity (ALD64).

The experiment was conducted in Hep3B. Cells were seeded at a density of 150,000 cells per 6-well, transfected with 0.1 nM siRNA and 1µg/ml Atufect after 24 h and lysed after 48 h. Total RNA was extracted and ALDH2 and PTEN mRNA levels were determined by Taqman qRT-PCR. Each bar represents mean ± SD from three technical replicates.

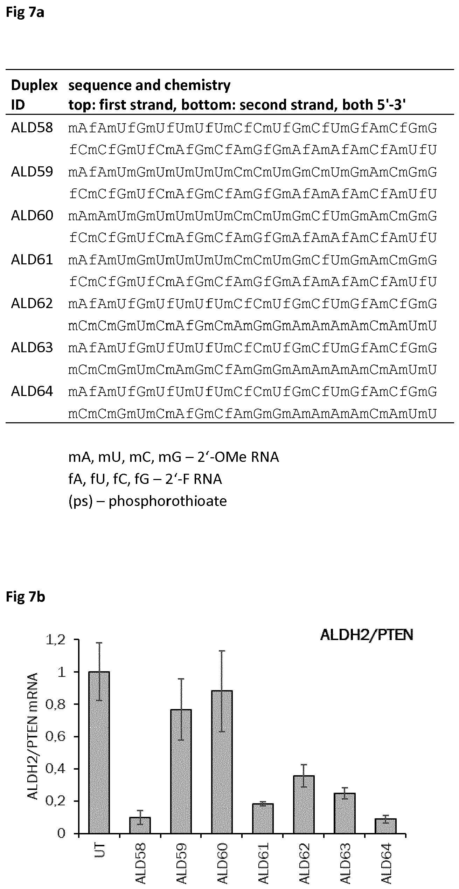

Results are shown in Figure 7a and b.

### Experiment 9-2

Tolerance of positions 2 and 14 for 2'-OMe in the first strand and tolerance of positions 7 and 9 for 2'-OMe in the second strand of a different siRNA against ALDH2 was analyzed. ALD72 contains alternating 2'-OMe/2'-F in both strands. ALD73 - ALD75 contain 2'-F at position 2 and/or 14 of the first strand with an all alternating second strand, whereas ALD76 - ALD78 contain 2'-F at position 7 and/or 9 of the second strand with an all alternating first strand. Positions 2 (ALD74) and 14 (ALD73) loose activity upon modification with 2'-OMe, whereas no 2'-OMe at position 2 and 14 restores activity (ALD75). Of the second strand, position 7 (ALD77) and position 9 (ALD76) loose activity upon modification with 2'OMe, whereas no 2'-OMe at positions 7 and 9 restores activity (ALD78).

The experiment was conducted in Hep3B. Cells were seeded at a density of 150,000 cells per 6-well, transfected with 0.1 nM siRNA and 1µg/ml Atufect after 24 h and lysed after 48 h. Total RNA was extracted and ALDH2 and PTEN mRNA levels were determined by Taqman qRT-PCR. Each bar represents mean ± SD from three technical replicates.

Results are shown in Figure 8a and b.

### Example 10

### Influence of modifications on the activity of an siRNA targeting DGAT2

Tolerance of positions 2 and 14 for 2'-OMe in the first strand and tolerance of positions 7 and 9 for 2'-OMe in the second strand of an siRNA against DGAT2 was analyzed. DGT01 contains alternating 2'-OMe/2'-F in both strands. DGT02 - DGT04 contain 2'-F at position 2 and/or 14 of the first strand with an all alternating second strand, whereas DGT05 - DGT07 contain 2'-F at position 7 and/or 9 of the second strand with an all alternating first strand. Positions 2 (DGT03) and 14 (DGT02) loose activity upon modification with 2'-OMe, whereas no 2'-OMe at position 2 and 14 restores activity at least partially (DGT04). Of the second strand, Position 7 (DGT06) and position 9 (DGT05) loose activity upon modification with 2'OMe, whereas no 2'-OMe at positions 7 and 9 restores activity (DGT07).

The experiment was conducted in Huh-7. Cells were seeded at a density of 80,000 cells per 6-well, transfected with 1 nM siRNA and 1 µg/ml Atufect after 24 h and lysed after 48 h. Total RNA was extracted and ALDH2 and PTEN mRNA levels were determined by Taqman qRT-PCR. Each bar represents mean ± SD of three technical replicates.

Results are shown in Figure 9a and b.

### Example 11

### Influence of DNA modifications on siRNA activity

Tolerance of positions 2 and 14 for DNA modification in the first strand and tolerance of positions 7 and 9 for DNA modification in the second strand of an siRNA against TMPRSS6 was analszed. TMP01 contains alternating 2'-OMe/2'-F in both strands. TMP93 contains 2'-OMe at position 14 of the first strand, whereas TMP113 contains 2'-H at the same position. TMP94 contains 2'-OMe at position 2 of the first strand, whereas TMP112 contains 2'-H at the same position. TMP97 contains 2'-OMe at position 9 of the second strand, whereas TMP117 contains 2'-H at the same position. TMP98 contains 2'-OMe at position 7 of the second strand, whereas TMP116 contains 2'-H at the same position.

The experiment was conducted in Hep3B. Cells were seeded at a density of 150,000 cells per 6-well, transfected with 0.1 nM siRNA and 1µg/ml Atufect after 24 h and lysed after 48 h. Total RNA was extracted and ALDH2 and Actin mRNA levels were determined by Taqman qRT-PCR. Each bar represents mean ± SD from three technical replicates.

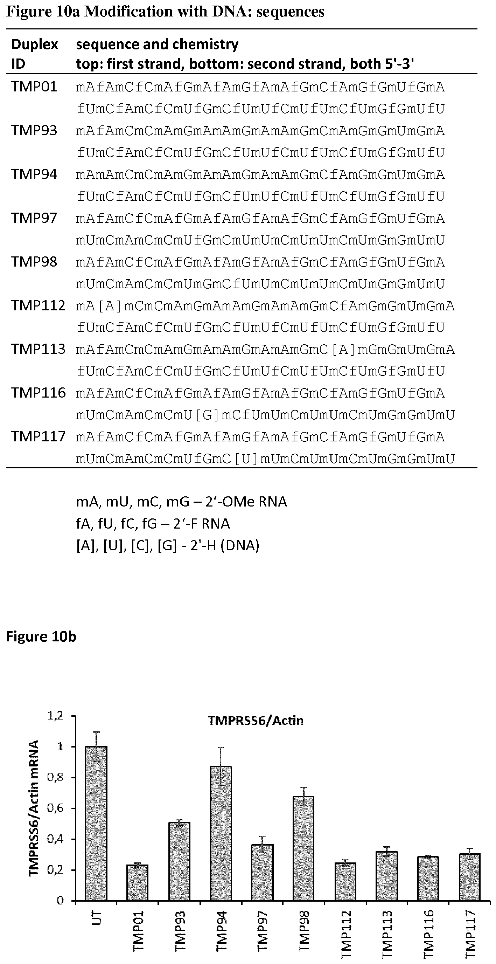

Results are shown in Figure 10 a and b.

### Example 12

### Influence of LNA modifications on siRNA activity

Tolerance of positions 2 and 14 for LNA modification in the first strand and tolerance of positions 7 and 9 for LNA modification in the second strand of an siRNA against TMPRSS6 was analysed. TMP01 contains alternating 2'-OMe/2'-F in both strands. TMP93 contains 2'-OMe at position 14 of the first strand, whereas TMP111 contains LNA at the same position. TMP94 contains 2'-OMe at position 2 of the first strand, whereas TMP110 contains LNA at the same position. TMP97 contains 2'-OMe at position 9 of the second strand, whereas TMP115 contains LNA at the same position. TMP98 contains 2'-OMe at position 7 of the second strand, whereas TMP114 contains LNA at the same position.

The experiment was conducted in Hep3B. Cells were seeded at a density of 150,000 cells per 6-well, transfected with 0.1 nM siRNA and 1 µg/ml Atufect after 24 h and lysed after 48 h. Total RNA was extracted and ALDH2 and Actin mRNA levels were determined by Taqman qRT-PCR. Each bar represents mean ± SD from three technical replicates.

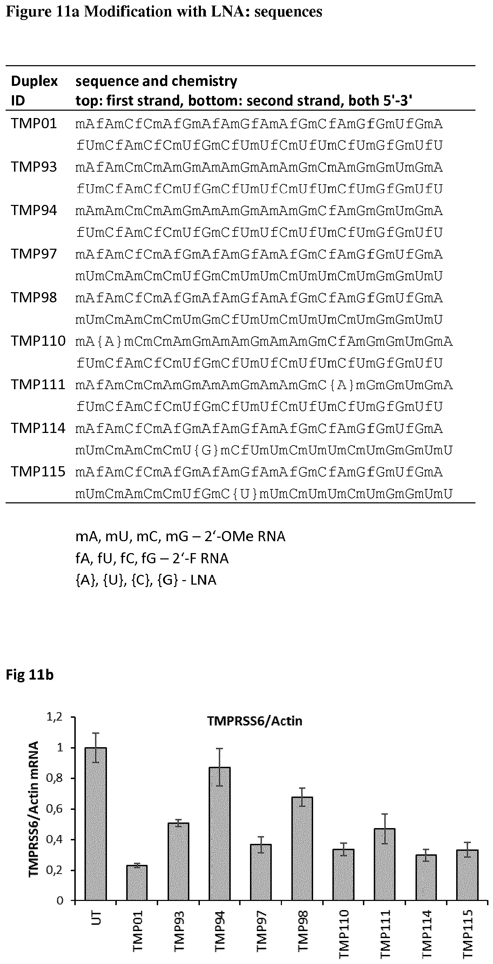

Results are shown in Figure 11a and b.

### Example 13

### Knockdown activity of different GalNAc-siRNA conjugates targeting TMPRSS6

### 13A

The influence of 2-O-methylation at certain second strand strand positions was investigated in the context of GalNAc-siRNA conjugates. All conjugates contain the same first strand. STS12009V23 contains an all-2'-O-methylated second strand, STS12009V25 has one 2'-F modification at second strand position 9, STS12009V26 has one 2'-F modification at second strand position 7, and STS12009V27 has three 2'-F modifications at second strand positions 7-9.

The experiment was conducted in Hep3B. Cells were seeded at a density of 150,000 cells per 6-well, transfected with 5 to 0.005 nM siRNA and 1 µg/ml Atufect after 24 h and lysed after 72 h. Total RNA was extracted and TMPRSS6 and PTEN mRNA levels were determined by Taqman qRT-PCR. Each bar represents mean ± SD from three technical replicates.

### 13B

The influence of 2-O-methylation at certain second strand positions was investigated in the context of GalNAc-siRNA conjugates. All conjugates contain the same first strand. STS12009V41L4 contains a second strand with alternating 2'-F/2'-OMe, STS12009V23 contains an all-2'-O-methylated second strand, STS12009V25 has one 2'-F modification at second strand position 9, STS12009V26 has one 2'-F modification at second strand position 7, and STS12009V27 has three 2'-F modifications at second strand positions 7 - 9.

The experiment was conducted in Hep3B. Cells were seeded at a density of 150,000 cells per 6-well, transfected with 10 to 0.001 nM siRNA and 1 µg/ml Atufect after 24 h and lysed after 72 h. Total RNA was extracted and TMPRSS6 and PTEN mRNA levels were determined by Taqman qRT-PCR. Each bar represents mean ± SD from three technical replicates.

### 13C

The influence of 2'-O-methylation at certain second strand positions was investigated in the context of GalNAc-siRNA conjugates. All conjugates contain the same first strand. STS12009V23 contains an all-2'-O-methylated second strand, STS12009V25 has one 2'-F modification at second strand position 9, STS12009V26 has one 2'-F modification at second strand position 7, and STS12009V27 has three 2'-F modifications at second strand positions 7-9.

The experiment was conducted in mouse primary hepatocytes. Cells were seeded at a density of 250,000 cells per 6-well and treated with 100 to 0.25 nM GalNAc-siRNA. Transfections with 10 nM GalNAc-siRNA and 1 µg/ml Atufect served as control.

Cells were lysed after 24 h, total RNA was extracted and TMPRSS6 and Actin mRNA levels were determined by Taqman qRT-PCR. Each bar represents mean ± SD from three technical replicates.

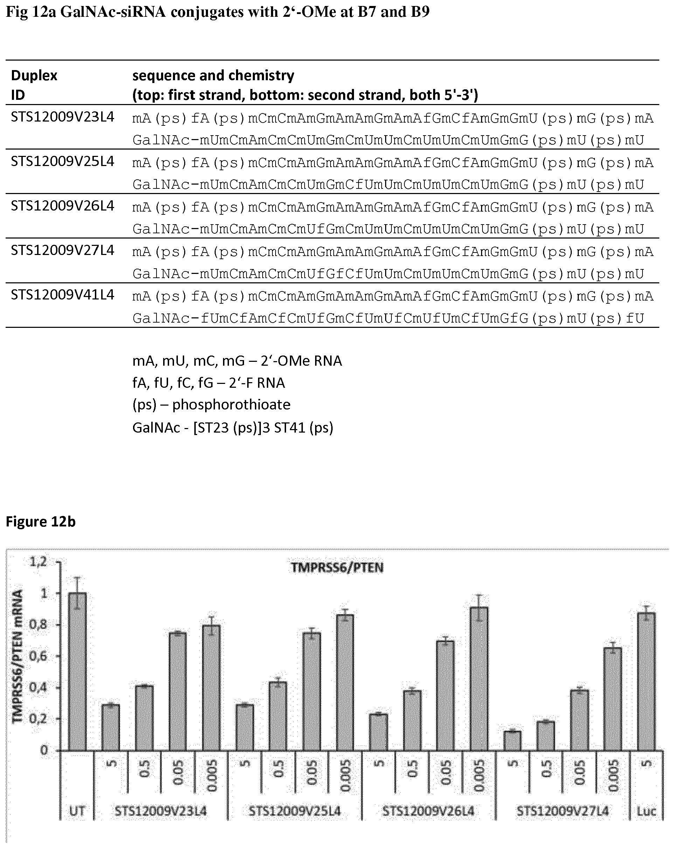

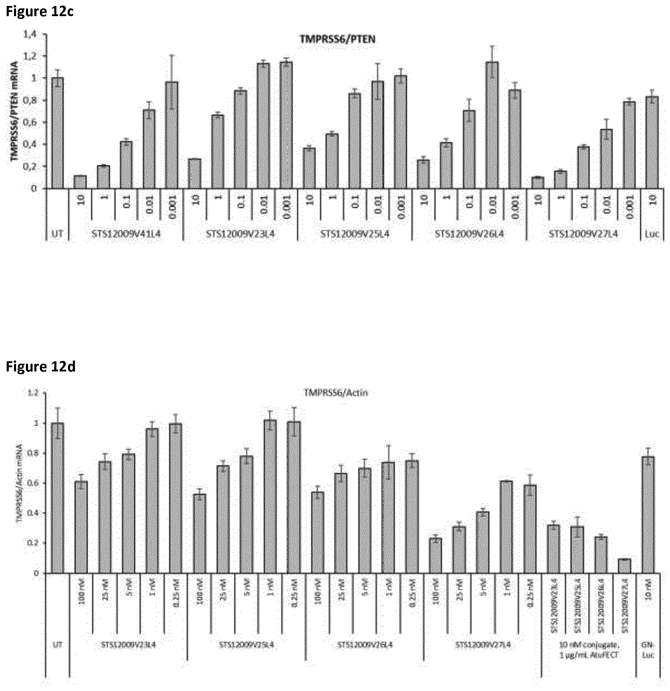

Results are shown in Figure 12a - d.

### Example 14

### Influence of DNA modification at multiple positions

Tolerance of positions 2 and 14 for DNA in the first strand and tolerance of positions 7-9 for DNA in the second strand of an siRNA against TMPRSS6 was analyzed. TMP70 contains alternating 2'-OMe/2'-F in both strands, whereas TMP119 contains 2'-OMe at all positions except of first strand positions 2 and 14 and second strand positions 7-9. TMP120-TMP126 contain a different number of DNA substitutions at 2'-F positions.

The experiment was conducted in Hep3B. Cells were seeded at a density of 150,000 cells per 6-well, transfected with 1 nM and 0.1 nM siRNA and 1µg/ml Atufect after 24 h and lysed after 48 h. Total RNA was extracted and TMPRSS6 and PTEN mRNA levels were determined by Taqman qRT-PCR. Each bar represents mean ± SD from three technical replicates.

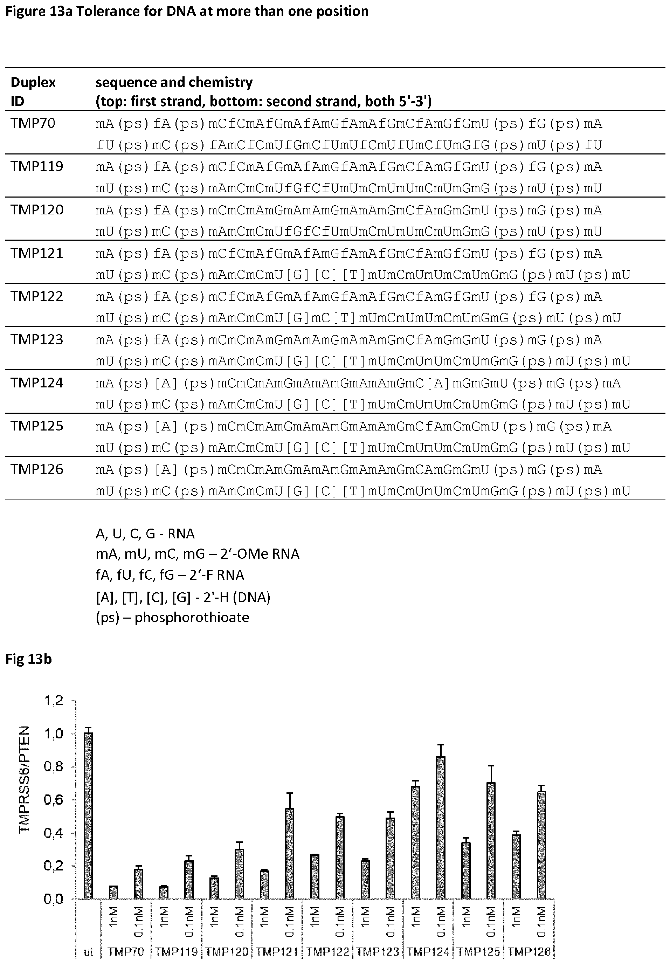

Results are shown in Figure 13a and b.

### Example 15

### Incorporation of DNA at key positions.

Tolerance of first strand positions 2 and 14 for DNA and tolerance of second strand positions 7-9 for DNA was analyzed with an siRNA targeting human ALDH2. ALD58 contains alternating 2'-OMe/2'-F in both strands, whereas ALD61 and ALD90-ALD92 contain a reduced 2'-F pattern in the first strand with DNA at position 2 (ALD90), DNA at position 14 (ALD91) and DNA at position 2 and 14 (ALD92), ALD93-ALD96 contain a reduced 2'-F pattern in the second strand with DNA at position 7 (ALD94), DNA at position 9 (ALD95) and DNA at position 7 and 9 (ALD96). ALD97 contains 2'-F at positions 7, 8 and 9 of the second strand, whereas ALD98 contains DNA at these positions.

The experiment was conducted in Hep3B. Cells were seeded at a density of 150,000 cells per 6-well, transfected with 0.1 nM and 0.01 nM siRNA and 1 µg/ml Atufect after 24 h and lysed after 48 h. Total RNA was extracted and ALDH2 and PTEN mRNA levels were determined by Taqman qRT-PCR. Each bar represents mean ± SD of three technical replicates.

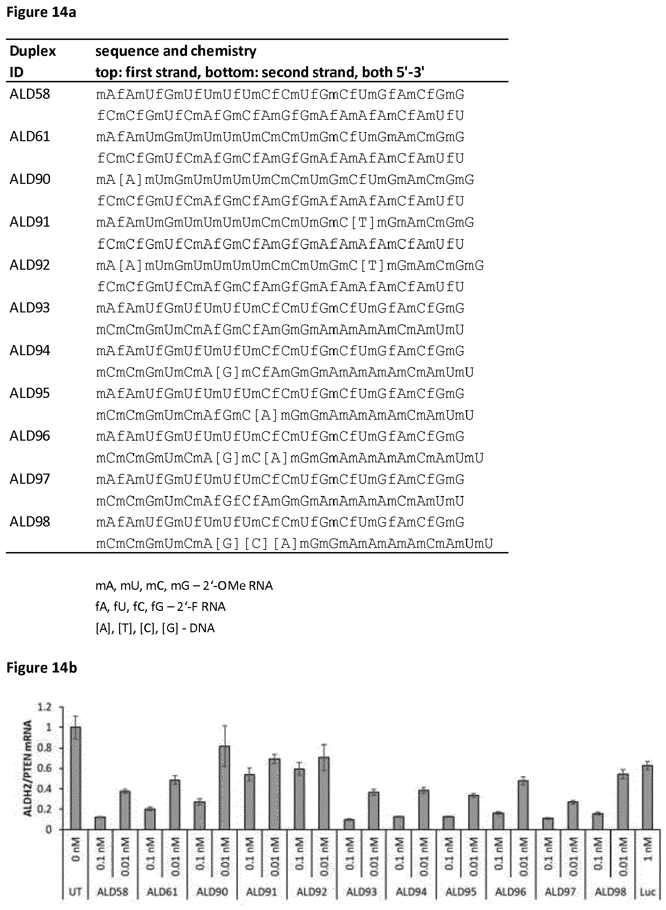

Data is shown in Figure 14a and b.

### Example 16

### Incorporation of DNA at key positions.

Tolerance of first strand positions 2 and 14 for DNA and tolerance of second strand positions 7-9 for DNA was analyzed with a second siRNA targeting human ALDH2. ALD72 contains alternating 2'-OMe/2'-F in both strands, whereas ALD75 and ALD99-ALD101 contain a reduced 2'-F pattern in the first strand with DNA at position 2 (ALD99), DNA at position 14 (ALD100) and DNA at position 2 and 14 (ALD101). ALD102-ALD105 contain a reduced 2'-F pattern in the second strand with DNA at position 7 (ALD103), DNA at position 9 (ALD104) and DNA at position 7 and 9 (ALD105). ALD106 contains 2'-F at positions 7, 8 and 9 of the second strand, whereas ALD107 contains DNA at these positions.

The experiment was conducted in Hep3B. Cells were seeded at a density of 150,000 cells per 6-well, transfected with 0.1 nM and 0.01 nM siRNA and 1 µg/ml Atufect after 24 h and lysed after 48 h. Total RNA was extracted and ALDH2 and PTEN mRNA levels were determined by Taqman qRT-PCR. Each bar represents mean ± SD of three technical replicates.

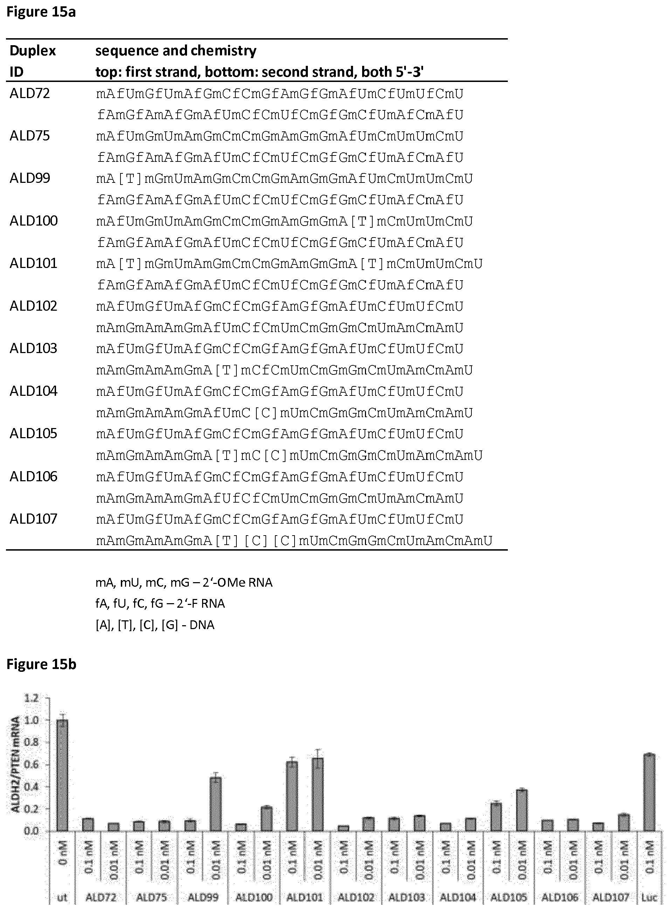

Data is shown in Figure 15a and b.

### Example 17

### Incorporation of DNA at key positions.

Tolerance of first strand positions 2 and 14 for DNA and tolerance of second strand positions 7-9 for DNA was analyzed with an siRNA targeting human DGAT2. DGT01 contains alternating 2'-OMe/2'-F in both strands, whereas DGT04 and DGT11-DGT13 contain a reduced 2'-F pattern in the first strand with DNA at position 2 (DGT11), DNA at position 14 (DGT12) and DNA at position 2 and 14 (DGT13). DGT14-DGT17 contain a reduced 2'-F pattern in the second strand with DNA at position 7 (DGT15), DNA at position 9 (DGT16) and DNA at position 7 and 9 (DGT17). DGT18 contains 2'-F at positions 7, 8 and 9 of the second strand, whereas DGT19 contains DNA at these positions.

The experiment was conducted in Huh7. Cells were seeded at a density of 80,000 cells per 6-well, transfected with 10 nM and 1 nM siRNA and 1 µg/ml Atufect after 24 h and lysed after 72 h. Total RNA was extracted and DGAT2 and PTEN mRNA levels were determined by Taqman qRT-PCR. Each bar represents mean ± SD of three technical replicates.

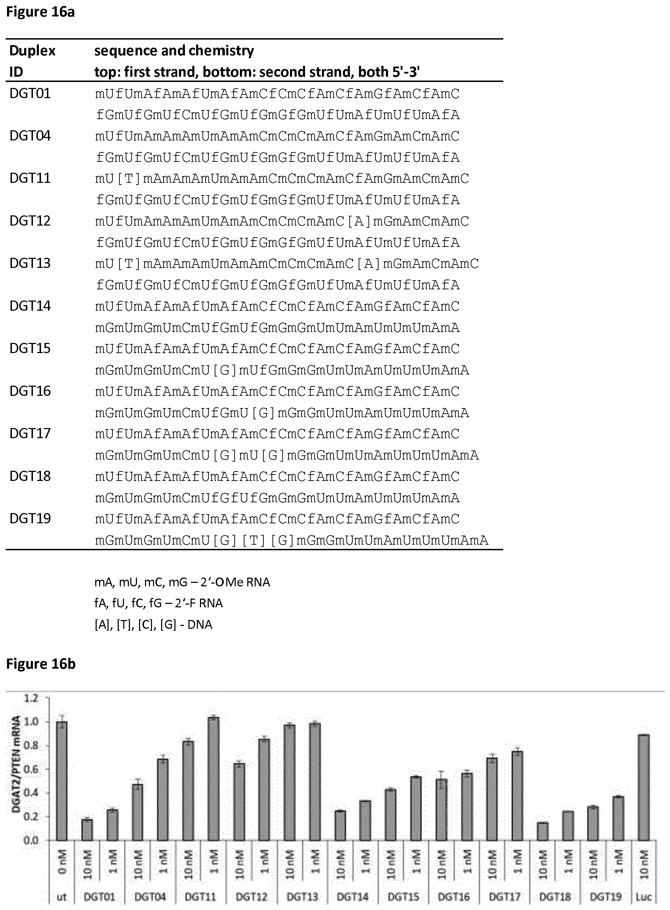

Data is shown in Figure 16a and b.

### Example 18

### Incorporation of 2'-O-methoxyethyl (MOE) at key positions.

Tolerance of first strand positions 2 and 14 for 2'-O-MOE and tolerance of second strand positions 7 and 9 for 2'-O-MOE was analyzed with an siRNA targeting ALDH2. ALD108 contains a reduced number of 2'-F in both strands. In this context, 2'-O-MOE is placed at position 2 (ALD115), at position 14 (ALD116) or at both positions 2 and 14 of the first strand (ALD117). Similarly, 2'-O-MOE is placed at position 7 (ALD118), position 9 (ALD119) or at both positions 7 and 9 of the second strand (ALD120). An siRNA against Luciferase was used as non-targeting control ("Luc").

The experiment was conducted in Hep3B. Cells were seeded at a density of 150,000 cells per 6-well, transfected with 0.1 nM siRNA and 1 µg/ml Atufect after 24 h and lysed after 48 h. Total RNA was extracted and ALDH2 and Actin mRNA levels were determined by Taqman qRT-PCR. Each bar represents mean ± SD of three technical replicates.

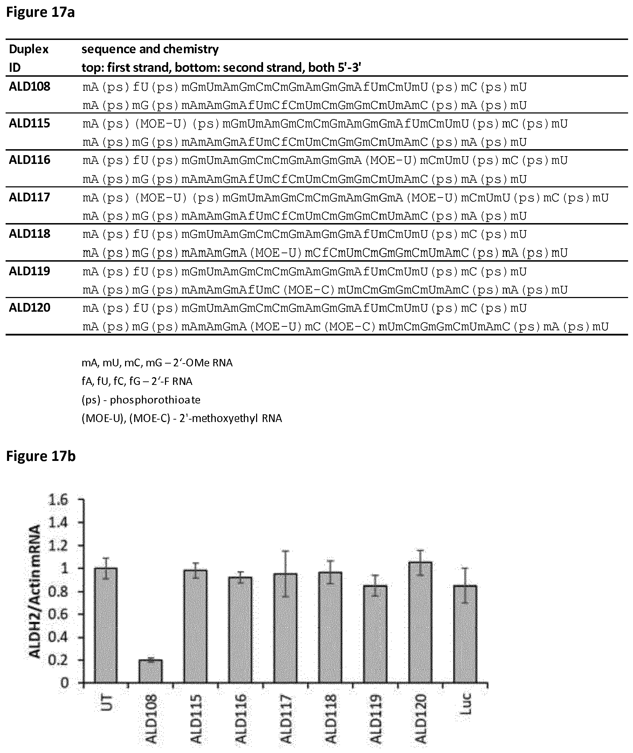

Data is shown in Figure 17a and b.

### Example 19

### Identification of key positions in the first strand.

The tolerance for 2'-OMe was investigated by addressing one position at a time in the context of an alternating pattern (change 2'-OMe into 2'-F and vice versa) in an siRNA targeting GHR. GHR03 contains completely alternating 2'-OMe/2'-F, GHR07 - GHR15 contain 2'-F into 2'-OMe changes at all even positions of the first strand, GHR16 - GHR25 contain 2'-OMe into 2'-F changes at all odd positions of the first strand. GHR07 contains 2'-OMe at first strand position 2, GHR13 contains 2'-OMe at first strand position 14. An siRNA targeting Luciferase ("Luc") was used as control.

The experiment was conducted in MCF-7 cells. The cells were seeded at a density of 120,000 cells per 6-well 24 h before transfection, transfected with 1 nM siRNA and 1 µg/ml Atufect and lysed after 48 h. Total RNA was extracted and GHR and PTEN mRNA levels were determined by Taqman qRT-PCR. Each bar represents mean ± SD of three technical replicates.

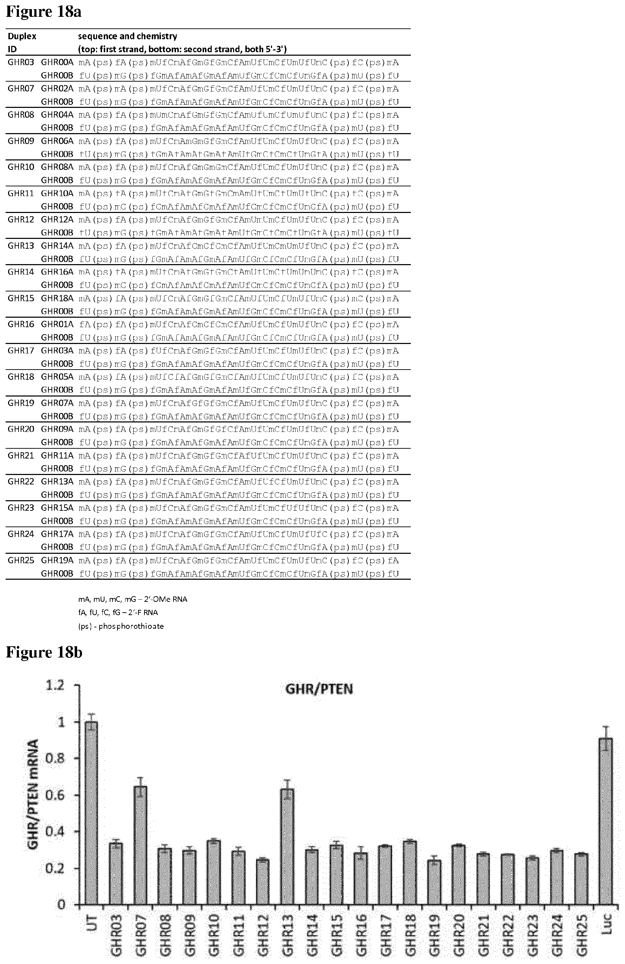

Data is shown in Figures 18a and b.

### Example 20: Synthesis of conjugates

Example compounds were synthesised according to methods described below and methods known to the person skilled in the art. Assembly of the oligonucleotide chain and linker building blocks was performed by solid phase synthesis applying phosphoramidte methodology. GalNAc conjugation was achieved by peptide bond formation of a GalNAc-carboxylic acid building block to the prior assembled and purified oligonucleotide having the necessary number of amino modified linker building blocks attached.

Oligonucleotide synthesis, deprotection and purification followed standard procedures that are known in the art.

All Oligonucleotides were synthesized on an AKTA oligopilot synthesizer using standard phosphoramidite chemistry. Commercially available solid support and 2'O-Methyl RNA phosphoramidites, 2'Fluoro, 2'Deoxy RNA phosphoramidites (all standard protection, ChemGenes, LinkTech) and commercially available 3'-Amino Modifier TFA Amino C-6 Icaa CPG 500Å (Chemgenes) were used. Per-acetylated galactose amine 8 is commercially available.

Ancillary reagents were purchased from EMP Biotech. Synthesis was performed using a 0.1 M solution of the phosphoramidite in dry acetonitrile and benzylthiotetrazole (BTT) was used as activator (0.3M in acetonitrile). Coupling time was 15 min. A Cap/OX/Cap or Cap/Thio/Cap cycle was applied (Cap: Ac₂O/NMI/Lutidine/Acetonitrile, Oxidizer: 0.1M I₂ in pyridine/H₂O). Phosphorothioates were introduced using standard commercially available thiolation reagent (EDITH, Link technologies). DMT cleavage was achieved by treatment with 3% dichloroacetic acid in toluene. Upon completion of the programmed synthesis cycles a diethylamine (DEA) wash was performed. All oligonucleotides were synthesized in DMT-off mode.

Attachment of the serinol-derived linker moiety was achieved by use of either base-loaded DMT-Serinol(TFA)-succinate-lcaa-CPG **10** or a DMT-Serinol(TFA) phosphoramidite **7** (synthesis was performed as described in literature Hoevelmann et al. Chem. Sci., 2016,7, 128-135). Tri-antennary GalNAc clusters (ST23 or C4XLT) were introduced by successive coupling of the respective trebler amidite derivatives (C4XLT-phos) followed by the GalNAc amidite (ST23-phos).

The single strands were cleaved off the CPG by 40% aq. methylamine treatment. The resulting crude oligonucleotide was purified by ion exchange chromatography (Resource Q, 6mL, GE Healthcare) on a AKTA Pure HPLC System using a sodium chloride gradient. Product containing fractions were pooled, desalted on a size exclusion column (Zetadex, EMP Biotech) and lyophilised.

Individual single strands were dissolved in a concentration of 60 OD/mL in H₂O. Both individual oligonucleotide solutions were added together in a reaction vessel. For easier reaction monitoring a titration was performed. The first strand was added in 25% excess over the second strand as determined by UV-absorption at 260nm. The reaction mixture was heated to 80°C for 5min and then slowly cooled to RT. Double strand formation was monitored by ion pairing reverse phase HPLC. From the UV-area of the residual single strand the needed amount of the second strand was calculated and added to the reaction mixture. The reaction was heated to 80°C again and slowly cooled to RT. This procedure was repeated until less than 10% of residual single strand was detected.

### Synthesis of compounds 2-10

Compounds **2** to **5** and DMT-Serinol(TFA)-phosphoramidite 7 were synthesised according to literature published methods (Hoevelmann et al. Chem. Sci., 2016,7, 128-135).

### (S)-4-(3-(bis(4-methoxyphenyl)(phenyl)methoxy)-2-(2,2,2-trifluoroacetamido)propoxy)-4-oxobutanoic acid (6).

To a solution of **5** in pyridine was added succinic anhydride, followed by DMAP. The resulting mixture was stirred at room temperature overnight. All starting material was consumed, as judged by TLC. The reaction was concentrated. The crude material was chromatographed in silica gel using a gradient 0% to 5% methanol in DCM (+ 1% triethylamine) to afford 1.33 g of **6** (yield = 38%). m/z (ESI-): 588.2 (100%), (calcd. for C30H29F3NO8⁻ [M-H]⁻ 588.6). 1 H-NMR: (400 MHz, CDCl3) δ [ppm] = 7.94 (d, 1H, NH), 7.39 - 7.36 (m, 2H, CHaryl), 7.29 - 7.25 (m, 7H, CHaryl), 6.82-6.79 (m, 4H, CHaryl), 4.51 - 4.47 (m, 1H), 4.31 - 4.24 (m, 2H), 3.77 (s, 6H, 2xDMTr-OMe), 3.66 - 3.60 (m, 16H, HNEt₃⁺), 3.26 - 3.25 (m, 2H), 2.97 - 2.81 (m, 20H, NEt₃), 2.50-2.41 (4H, m), 1.48 - 1.45 (m, 26H, HNEt₃⁺), 1.24 - 1.18 (m, 29H, NEt₃).

### DMT-Serinol(TFA)-succinate-lcaa-CPG (10)

The DMT-Serinol(TFA)-succinate (159 mg, 270 umol) and HBTU (113 mg, 299 umol) were dissolved in CH₃CN (10 mL). Diisopropylethylamine (DIPEA, 94 µL, 540 umol) was added to the solution, and the mixture was swirled for 2 min followed by addition native amino-Icaa-CPG (500 A, 3 g, amine content: 136 umol/g). The suspension was gently shaken at room temperature on a wrist-action shaker for 16h then filtered, and washed with DCM and EtOH. The solid support was dried under vacuum for 2 h. The unreacted amines on the support were capped by stirring with acetic anhydride/lutidine/N-methylimidazole at room temperature. The washing of the support was repeated as above. The solid was dried under vacuum to yield solid support **10** (3 g, 26 umol/g loading).

### GalNAc Synthon (9)

Synthesis of the GalNAc synthon **9** was performed as described in Nair et al. J. Am. Chem. Soc., 2014, 136 (49), pp 16958-16961, in 46% yield over two steps.

The characterising data matched the published data.

### Synthesis of Oligonucleotides

All single stranded oligonucleotides were synthesised according to the reaction conditions described above and in Figure 8 and 9.

All final single stranded products were analysed by AEX-HPLC to prove their purity. Purity is given in %FLP (% full length product) which is the percentage of the UV-area under the assigned product signal in the UV-trace of the AEX-HPLC analysis of the final product. Identity of the respective single stranded products (non-modified, amino-modified precursors or GalNAc conjugated oligonucleotides) was proved by LC-MS analysis.

**Table 2: Single stranded un-coniuaated oligonucleotides**

| **Product (11)** | **Name** | **MW calc.** | **MW (ESI-) found** | **%FLP (AEX- HPLC)** |
|---|---|---|---|---|
| A0002 | STS16001A | 6943.3 Da | 6943.0 Da | 86.6% |
| A0006 | STS16001BL4 | 8387.5 Da | 8387.5 Da | 94.1% |
| A0130 | STS18001A | 6259.9 Da | 6259.8 Da | 76.5% |
| A0131 | STS18001BL4 | 7813.2 Da | 7813.1 Da | 74.3% |
| A0220 | STS16001B-5'1xNH2 | 6982.2 Da | 6982.1 Da | 95.7% |
| A0237 | STS16001A | 6943.3 Da | 6943.3 Da | 95.6% |
| A0244 | STS16001BV1 | 6845.2 Da | 6844.9 Da | 98.2% |
| A0264 | STS16001AV4-3'1xNH2 | 7112.4 Da | 7112.2 Da | 95.4% |
| A0329 | STS16001BV6-3'5'1xNH2 | 7183.3 Da | 7183.2 Da | 88.8% |

5'1 x NH2 means refers to the position (5' end) and number (1 x NH2) of free serinol derived amino groups which are available for conjugation. For example, 1x3'NH2 on **A0264** means there is free amino group which can be reacted with GalNAc synthon **9** at the 3' end of the strand **A0264.** 3'5'1xNH2 means there is one serinol-derived free amino group which can be reacted with GalNAc linker **9** at the 3' end and the 5' end of the strand.

### Synthesis of conjugates 1-3 and reference conjugates 1-2

### Conjugated singles strands for conjugates 1-2 and reference conjugates 1-2

Conjugation of the GalNac synthon (**9**) was achieved by coupling to the serinol-amino function of the respective oligonucleotide strand **11** using a peptide coupling reagent. Therefore, the respective amino-modified precursor molecule **11** was dissolved in H₂O (500 OD/mL) and DMSO (DMSO/H₂O, 2/1, v/v) was added, followed by DIPEA (2.5% of total volume). In a separate reaction vessel pre-activation of the GalN(Ac4)-C4-acid (**9**) was performed by reacting 2 eq. (per amino function in the amino-modified precursor oligonucleotide **11**) of the carboxylic acid component with 2 eq. of HBTU in presence of 8 eq. DIPEA in DMSO. After 2 min the pre-activated compound 9 was added to the solution of the respective amino-modified precursor molecule. After 30 min the reaction progress was monitored by LCMS or AEX-HPLC. Upon completion of the conjugation reaction the crude product was precipitated by addition of 10x *i*PrOH and 0.1x 2M NaCl and harvested by centrifugation and decantation. To set free the acetylated hydroxyl groups in the GalNAc moieties the resulting pellet was dissolved in 40% MeNH2 (1mL per 500 OD) and after 15 min at RT diluted in H₂O (1:10) and finally purified again by anion exchange and size exclusion chromatography and lyophilised to yield the final product **12.**

**Table 3: Single stranded GalNAc-conjugated oligonucleotides**

| **Product (12)** | **Starting Material** | **Name** | **MW calc.** | **MW (ESI-) found** | **%FLP (AEX- HPLC)** |
|---|---|---|---|---|---|
| A0241 | A0220 | STS16001BL20 | 7285.5 Da | 7285.3 Da | 91.8% |
| A0268 | A0264 | STS16001AV4L33 | 7415.7 Da | 7415.4 Da | 96.9% |
| A0330 | A0329 | STS16001 BV6L42 | 7789.8 Da | 7789.8 Da | 95.5% |

### Double strand formation

Double strand formation was performed according to the methods described above.

The double strand purity is given in % double strand which is the percentage of the UV-area under the assigned product signal in the UV-trace of the IP-RP-HPLC analysis.

**Table 4: Nucleic acid conjugates**

| **Product** | **Starting Materials** | | **Name** | **% double strand** |
|---|---|---|---|---|
| | **First Strand** | **Second Strand** | | |
| Ref. Conj. 1 | A0237 | A0241 | STS16001L20 | 97.7% |
| Ref. Conj. 2 | A0268 | A0244 | STS16001L33 | 97.8% |
| Ref. Conj. 3 | A0130 | A0131 | STS18001L4 | 96.8% |
| Ref. Conj. 4 | A0002 | A0006 | STS16001L4 | 90.1% |
| Conjugate 1 | A0268 | A0241 | STS16001L24 | 96.0% |
| Conjugate 2 | A0237 | A0330 | STS16001V1L42 | 98.5% |
| Conjugate 3 | A0268 | A0330 | STS16001V1L43 | 98.2% |

### Sequences

Modifications key for the following sequences:
f denotes 2'Fluoro 2'deoxyribonucleotide or 2'-fluoro ribonucleotide (the terms are interchangeable)
m denotes 2'O Methyl ribonucleotide
(ps) denotes phosphorothioate linkage
Ser(GN) is a GalNAc-C4 building block attached to serinol derived linker moiety: 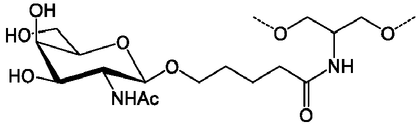 wherein the O--- is the linkage between the oxygen atom and e.g. H, phosphordiester linkage or phosphorothioate linkage.

C4XLT is:

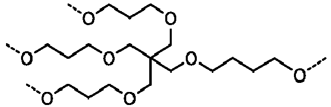

ST23 is:

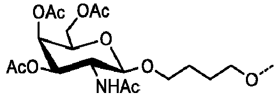

Synthesis of the phosphoramidite derivatives of C4XLT (C4XLT-phos) as well as ST23 (ST23-phos) can be performed as described in WO2017/174657.

C4XLT-phos:

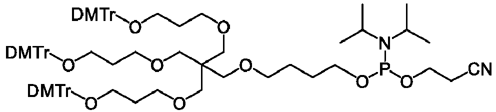

ST23-phos:

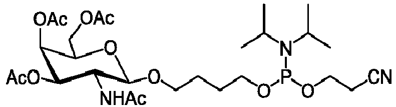

The following conjugates are provided

### Conjugate 1

Antisense strand - STS16001AL33
5' mU (ps) fU (ps) mA fU mA fG mA fG mC fA mA fG mA fA mC fA mC fU mG (ps) fU (ps) mU (ps) Ser(GN) 3'
Sense strand - STS16001BL20
5' Ser(GN) (ps) fA mA fC mA fG mU fG mU fU mC fU mU fG mC fU mC fU mA fU (ps) mA (ps) fA 3'

### Conjugate 2

Antisense strand - STS16001A
mU (ps) fU (ps) mA fU mA fG mA fG mC fA mA fG mA fA mC fA mC fU mG (ps) fU (ps) mU Sense strand - STS16001BV1L42
Ser(GN) (ps) fA (ps) mA (ps) fC mA fG mU fG mU fU mC fU mU fG mC fU mC fU mA fU (ps) mA (ps) fA (ps) Ser(GN)

### Conjugate 3

Antisense strand - STS16001AL33
5' mU (ps) fU (ps) mA fU mA fG mA fG mC fA mA fG mA fA mC fA mC fU mG (ps) fU (ps) mU (ps) Ser(GN) 3'
Sense strand - STS16001BV1L42
5' Ser(GN) (ps) fA (ps) mA (ps) fC mA fG mU fG mU fU mC fU mU fG mC fU mC fU mA fU (ps) mA (ps) fA (ps) Ser(GN) 3'

### Reference conjugate 1

Antisense strand - STS16001A
mU (ps) fU (ps) mA fU mA fG mA fG mC fA mA fG mA fA mC fA mC fU mG (ps) fU (ps) mU Sense strand - STS16001 BL20
Ser(GN) (ps) fA mA fC mA fG mU fG mU fU mC fU mU fG mC fU mC fU mA fU (ps) mA (ps) fA

### Reference conjugate 2

Antisense strand - STS16001AL33
mU (ps) fU (ps) mA fU mA fG mA fG mC fA mA fG mA fA mC fA mC fU mG (ps) fU (ps) mU (ps) Ser(GN)
Sense strand - STS16001V1B
fA (ps) mA (ps) fC mA fG mU fG mU fU mC fU mU fG mC fU mC fU mA fU (ps) mA (ps) fA

### Reference Conjugate 3

Antisense strand - STS18001A (A0130)
mU(ps)fC(ps)mGfAmAfGmUfAmUfUmCfCmGfCmGfUmA(ps)fC(ps)mG
Sense strand - STS18001BL4 (A0131)
[(ST23) (ps)]3 C4XLT(ps)fCmGfUmAfCmGfCmGfGmAfAmUfAmCfUmUfC (ps)mG (ps)fA

### Reference Conjugate 4

Antisense strand - STS16001AL33
mU (ps) fU (ps) mA fU mA fG mA fG mC fA mA fG mA fA mC fA mC fU mG (ps) fU (ps) mU Sense strand - STS16001BL4
5'[(ST23)(ps)3 C4XLT(ps) fA (ps) mA (ps) fC mA fG mU fG mU fU mC fU mU fG mC fU mC fU mA fU (ps) mA (ps) fA

### In vitro determination of TTR knockdown of various TTR siRNA GalNAc conjugates

Murine primary hepatocytes were seeded into collagen pre-coated 96 well plates (Thermo Fisher Scientific, #A1142803) at a cell density of 30,000 cells per well and treated with siRNA-conjugates at concentrations ranging from 10nM to 0.0001 nM. 24h post treatment cells were lysed and RNA extracted with InviTrap® RNA Cell HTS 96 Kit / C24 x 96 preps (Stratec #7061300400) according to the manufactures protocol. Transcripts levels of TTR and housekeeping mRNA (Ptenll) were quantified by TaqMan analysis.

Target gene expression in primary murine hepatocytes 24h following treatment with the conjugates of the invention, Conjugates 1-3, showed that target gene expression decreases as the dose of the conjugate increased compared to the negative controls (see "UT" column and Reference Conjugate 3), as shown in Figure 28. This indicates that the first strand is binding to the target gene, thus lowering gene expression. Figure 28 also shows the target gene expression levels of Reference Conjugates 1 and 2 which act as comparator conjugates. As can be seen from a comparison between the data presented in Figures 28A and 28C, and 28B and 28C, the conjugates of the invention (Conjugates 1-3) decrease the target gene expression compared to Reference Conjugates 1 and 2. The most effective conjugate at 0.01 nM appears to be Conjugate 2. The most effective conjugate at 0.1 nM, 0.5 nM, 1 nM and 10 nM appears to be Conjugate 3.
C57BL/6 mice were treated s.c. with 1 mg/kg siRNA-conjugates at day 0. Serum samples were taken at day 7, 14, and 27 by orbital sinus bleeding and stored at -20°C until analysis. Serum TTR quantification was performed with a Mouse Prealbumin ELISA (ALPCO, 41-PALMS/lot 22, 2008003B) according to the manufacturers protocol (sample dilution 1:8000 or 1:800). The results of the time course of serum TTR in c57BL/6 mice cohorts of n=4 at 7, 14, and 27 days post s.c. treatment with 1mg/kg Conjugates 1-3 and mock treated (PBS) individuals is shown in Figure 29. As indicated by the data in Figure 11, the conjugates of the invention are particularly effective at reducing target gene expression compared to the negative control (PBS) and Reference Conjugates 1, 2, and in particular to Reference Conjugate 4. Conjugates 2 and 3 are also more effective than Reference Conjugates 1, 2 and 4. The most effective conjugate is Conjugate 2.
All patents and patent applications referred to herein are incorporated by reference in their entirety.

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

| **SEQ ID** | **Name** | **Sequence (5'-3')** |
|---|---|---|
| 1 | CLC28-a | AUGmCAAAAmUACACUmUCUAC |
| 2 | CLC28-b | GmUAGAAGmUGmUAmUmUmUmUGmCAmU |
| 3 | CLC59-a | AmUGCAAAAmUACACUmUCUAC |
| 4 | CLC59-b | GmUAGAAGmUGmUAmUmUmUmUGmCAmU |
| 5 | CLC60-a | AUmGCAAAAmUACACUmUCUAC |
| 6 | CLC60-b | GmUAGAAGmUGmUAmUmUmUmUGmCAmU |
| 7 | CLC56-a | AmUGmCAmAAmAUmACmACUUmCUmAC |
| 8 | CLC56-b | mGUmAGmAAmGUGUmAUmUUmUGmCAmU |
| 9 | CLC57-a | AUmGCAmAAmAUmACmACUUmCUmAC |
| 10 | CLC57-b | mGUmAGmAAmGUGUmAUmUUmUGmCAmU |
| 11 | CLC01-a | mAUmGCmAAmAAmUAmCAmCUmUCmUAmC |
| 12 | CLC01-b | GmUAmGAmAGmUGmUAmUUmUUmGCmAU |
| 13 | CLC22-a | AUGmCAAAAmUACACmUUCUAC |
| 14 | CLC22-b | GmUAGAAGmUGmUAmUmUmUmUGmCAmU |
| 15 | CLC28-a | AUGmCAAAAmUACACUmUCUAC |
| 16 | CLC28-b | GmUAGAAGmUGmUAmUmUmUmUGmCAmU |
| 17 | CLC16-a | AmUGmCAmAAmAUmACmACUUmCUmAC |
| 18 | CLC16-b | mGUmAGmAAmGUmGUmAUmUUmUGmCAmU |
| 19 | CLC15-a | AmUGmCAmAAmAUmACmACmUUmCUmAC |
| 20 | CLC15-b | mGUmAGmAAmGUmGUmAUmUUmUGmCAmU |
| 21 | HFE04-a | fAfUmUfGfAmUfAfGfAfAmCfCfAfUmCfUfUmCfA |
| 22 | HFE04-b | mUfGfAfAfGfAmUfGfGmUmUmCmUfAmUmCfAfAmU |
| 23 | HFE06-a | fAmUfUfGfAmUfAfGfAfAmCfCfAfUmCfUfUmCfA |
| 24 | HFE06-b | mUfGfAfAfGfAmUfGfGmUmUmCmUfAmUmCfAfAmU |
| 25 | ALD01-a | mA(ps)fA(ps)mUfGmUfUmUfUmCfCmUfGmCfUmGfAmC(ps)fG(p s)mG |
| 26 | ALD01-b | fC(ps)mC(ps)fGmUfCmAfGmCfAmGfGmAfAmAfAmCfA(ps)mU(p s)fU |
| 27 | ALD13-a | mA(ps)mA(ps)mUfGmUfUmUfUmCfCmUfGmCfUmGfAmC(ps)fG( ps)mG |
| 28 | ALD13-b | fC(ps)mC(ps)fGmUfCmAfGmCfAmGfGmAfAmAfAmCfA(ps)mU(p s)fU |
| 29 | ALD14-a | mA(ps)fA(ps)mUmGmUfUmUfUmCfCmUfGmCfUmGfAmC(ps)fG( ps)mG |
| 30 | ALD14-b | fC(ps)mC(ps)fGmUfCmAfGmCfAmGfGmAfAmAfAmCfA(ps)mU(p s)fU |
| 31 | ALD15-a | mA(ps)fA(ps)mUfGmUmUmUfUmCfCmUfGmCfUmGfAmC(ps)fG( ps)mG |
| 32 | ALD15-b | fC(ps)mC(ps)fGmUfCmAfGmCfAmGfGmAfAmAfAmCfA(ps)mU(p s)fU |
| 33 | ALD16-a | mA(ps)fA(ps)mUfGmUfUmUmUmCfCmUfGmCfUmGfAmC(ps)fG( ps)mG |
| 34 | ALD16-b | fC(ps)mC(ps)fGmUfCmAfGmCfAmGfGmAfAmAfAmCfA(ps)mU(p s)fU |
| 35 | ALD17-a | mA(ps)fA(ps)mUfGmUfUmUfUmCmCmUfGmCfUmGfAmC(ps)fG( ps)mG |
| 36 | ALD17-b | fC(ps)mC(ps)fGmUfCmAfGmCfAmGfGmAfAmAfAmCfA(ps)mU(p s)fU |
| 37 | ALD18-a | mA(ps)fA(ps)mUfGmUfUmUfUmCfCmUmGmCfUmGfAmC(ps)fG( ps)mG |
| 38 | ALD18-b | fC(ps)mC(ps)fGmUfCmAfGmCfAmGfGmAfAmAfAmCfA(ps)mU(p s)fU |
| 39 | ALD19-a | mA(ps)fA(ps)mUfGmUfUmUfUmCfCmUfGmCmUmGfAmC(ps)fG( ps)mG |
| 40 | ALD19-b | fC(ps)mC(ps)fGmUfCmAfGmCfAmGfGmAfAmAfAmCfA(ps)mU(p s)fU |
| 41 | ALD20-a | mA(ps)fA(ps)mUfGmUfUmUfUmCfCmUfGmCfUmGmAmC(ps)fG( ps)mG |
| 42 | ALD20-b | fC(ps)mC(ps)fGmUfCmAfGmCfAmGfGmAfAmAfAmCfA(ps)mU(p s)fU |
| 43 | ALD21-a | mA(ps)fA(ps)mUfGmUfUmUfUmCfCmUfGmCfUmGfAmC(ps)mG( ps)mG |
| 44 | ALD21-b | fC(ps)mC(ps)fGmUfCmAfGmCfAmGfGmAfAmAfAmCfA(ps)mU(p s)fU |
| 45 | ALD22-a | fA(ps)fA(ps)mUfGmUfUmUfUmCfCmUfGmCfUmGfAmC(ps)fG(ps )mG |
| 46 | ALD22-b | fC(ps)mC(ps)fGmUfCmAfGmCfAmGfGmAfAmAfAmCfA(ps)mU(p s)fU |
| 47 | ALD23-a | mA(ps)fA(ps)fUfGmUfUmUfUmCfCmUfGmCfUmGfAmC(ps)fG(ps )mG |
| 48 | ALD23-b | fC(ps)mC(ps)fGmUfCmAfGmCfAmGfGmAfAmAfAmCfA(ps)mU(p s)fU |
| 49 | ALD24-a | mA(ps)fA(ps)mUfGfUfUmUfUmCfCmUfGmCfUmGfAmC(ps)fG(ps )mG |
| 50 | ALD24-b | fC(ps)mC(ps)fGmUfCmAfGmCfAmGfGmAfAmAfAmCfA(ps)mU(p s)fU |
| 51 | ALD25-a | mA(ps)fA(ps)mUfGmUfUfUfUmCfCmUfGmCfUmGfAmC(ps)fG(ps )mG |
| 52 | ALD25-b | fC(ps)mC(ps)fGmUfCmAfGmCfAmGfGmAfAmAfAmCfA(ps)mU(p s)fU |
| 53 | ALD26-a | mA(ps)fA(ps)mUfGmUfUmUfUfCfCmUfGmCfUmGfAmC(ps)fG(ps )mG |
| 54 | ALD26-b | fC(ps)mC(ps)fGmUfCmAfGmCfAmGfGmAfAmAfAmCfA(ps)mU(p s)fU |
| 55 | ALD27-a | mA(ps)fA(ps)mUfGmUfUmUfUmCfCfUfGmCfUmGfAmC(ps)fG(ps )mG |
| 56 | ALD27-b | fC(ps)mC(ps)fGmUfCmAfGmCfAmGfGmAfAmAfAmCfA(ps)mU(p s)fU |
| 57 | ALD28-a | mA(ps)fA(ps)mUfGmUfUmUfUmCfCmUfGfCfUmGfAmC(ps)fG(ps )mG |
| 58 | ALD28-b | fC(ps)mC(ps)fGmUfCmAfGmCfAmGfGmAfAmAfAmCfA(ps)mU(p s)fU |
| 59 | ALD29-a | mA(ps)fA(ps)mUfGmUfUmUfUmCfCmUfGmCfUfGfAmC(ps)fG(ps )mG |
| 60 | ALD29-b | fC(ps)mC(ps)fGmUfCmAfGmCfAmGfGmAfAmAfAmCfA(ps)mU(p s)fU |
| 61 | ALD30-a | mA(ps)fA(ps)mUfGmUfUmUfUmCfCmUfGmCfUmGfAfC(ps)fG(ps )mG |
| 62 | ALD30-b | fC(ps)mC(ps)fGmUfCmAfGmCfAmGfGmAfAmAfAmCfA(ps)mU(p s)fU |
| 63 | ALD31-a | mA(ps)fA(ps)mUfGmUfUmUfUmCfCmUfGmCfUmGfAmC(ps)fG(p s)fG |
| 64 | ALD31-b | fC(ps)mC(ps)fGmUfCmAfGmCfAmGfGmAfAmAfAmCfA(ps)mU(p s)fU |
| 65 | ALD32-a | mA(ps)fA(ps)mUfGmUfUmUfUmCfCmUfGmCfUmGfAmC(ps)fG(p s)mG |
| 66 | ALD32-b | mC(ps)mC(ps)fGmUfCmAfGmCfAmGfGmAfAmAfAmCfA(ps)mU( ps)fU |
| 67 | ALD33-a | mA(ps)fA(ps)mUfGmUfUmUfUmCfCmUfGmCfUmGfAmC(ps)fG(p s)mG |
| 68 | ALD33-b | fC(ps)mC(ps)mGmUfCmAfGmCfAmGfGmAfAmAfAmCfA(ps)mU( ps)fU |
| 69 | ALD34-a | mA(ps)fA(ps)mUfGmUfUmUfUmCfCmUfGmCfUmGfAmC(ps)fG(p s)mG |
| 70 | ALD34-b | fC(ps)mC(ps)fGmUmCmAfGmCfAmGfGmAfAmAfAmCfA(ps)mU( ps)fU |
| 71 | ALD35-a | mA(ps)fA(ps)mUfGmUfUmUfUmCfCmUfGmCfUmGfAmC(ps)fG(p s)mG |
| 72 | ALD35-b | fC(ps)mC(ps)fGmUfCmAmGmCfAmGfGmAfAmAfAmCfA(ps)mU( ps)fU |
| 73 | ALD36-a | mA(ps)fA(ps)mUfGmUfUmUfUmCfCmUfGmCfUmGfAmC(ps)fG(p s)mG |
| 74 | ALD36-b | fC(ps)mC(ps)fGmUfCmAfGmCmAmGfGmAfAmAfAmCfA(ps)mU( ps)fU |
| 75 | ALD37-a | mA(ps)fA(ps)mUfGmUfUmUfUmCfCmUfGmCfUmGfAmC(ps)fG(p s)mG |
| 76 | ALD37-b | fC(ps)mC(ps)fGmUfCmAfGmCfAmGmGmAfAmAfAmCfA(ps)mU( ps)fU |
| 77 | ALD38-a | mA(ps)fA(ps)mUfGmUfUmUfUmCfCmUfGmCfUmGfAmC(ps)fG(p s)mG |
| 78 | ALD38-b | fC(ps)mC(ps)fGmUfCmAfGmCfAmGfGmAmAmAfAmCfA(ps)mU( ps)fU |
| 79 | ALD39-a | mA(ps)fA(ps)mUfGmUfUmUfUmCfCmUfGmCfUmGfAmC(ps)fG(p s)mG |
| 80 | ALD39-b | fC(ps)mC(ps)fGmUfCmAfGmCfAmGfGmAfAmAmAmCfA(ps)mU( ps)fU |
| 81 | ALD40-a | mA(ps)fA(ps)mUfGmUfUmUfUmCfCmUfGmCfUmGfAmC(ps)fG(p s)mG |
| 82 | ALD40-b | fC(ps)mC(ps)fGmUfCmAfGmCfAmGfGmAfAmAfAmCmA(ps)mU( ps)fU |
| 83 | ALD41-a | mA(ps)fA(ps)mUfGmUfUmUfUmCfCmUfGmCfUmGfAmC(ps)fG(p s)mG |
| 84 | ALD41-b | fC(ps)mC(ps)fGmUfCmAfGmCfAmGfGmAfAmAfAmCfA(ps)mU(p s)mU |
| 85 | ALD42-a | mA(ps)fA(ps)mUfGmUfUmUfUmCfCmUfGmCfUmGfAmC(ps)fG(p s)mG |
| 86 | ALD42-b | fC(ps)fC(ps)fGmUfCmAfGmCfAmGfGmAfAmAfAmCfA(ps)mU(ps) fU |
| 87 | ALD43-a | mA(ps)fA(ps)mUfGmUfUmUfUmCfCmUfGmCfUmGfAmC(ps)fG(p s)mG |
| 88 | ALD43-b | fC(ps)mC(ps)fGfUfCmAfGmCfAmGfGmAfAmAfAmCfA(ps)mU(ps) fU |
| 89 | ALD44-a | mA(ps)fA(ps)mUfGmUfUmUfUmCfCmUfGmCfUmGfAmC(ps)fG(p s)mG |
| 90 | ALD44-b | fC(ps)mC(ps)fGmUfCfAfGmCfAmGfGmAfAmAfAmCfA(ps)mU(ps) fU |
| 91 | ALD45-a | mA(ps)fA(ps)mUfGmUfUmUfUmCfCmUfGmCfUmGfAmC(ps)fG(p s)mG |
| 92 | ALD45-b | mA(ps)fA(ps)mUfGmUfUmUfUmCfCmUfGmCfUmGfAmC(ps)fG(p s)mG |
| 93 | ALD46-a | mA(ps)fA(ps)mUfGmUfUmUfUmCfCmUfGmCfUmGfAmC(ps)fG(p s)mG |
| 94 | ALD46-b | fC(ps)mC(ps)fGmUfCmAfGmCfAfGfGmAfAmAfAmCfA(ps)mU(ps) fU |
| 95 | ALD47-a | mA(ps)fA(ps)mUfGmUfUmUfUmCfCmUfGmCfUmGfAmC(ps)fG(p s)mG |
| 96 | ALD47-b | fC(ps)mC(ps)fGmUfCmAfGmCfAmGfGfAfAmAfAmCfA(ps)mU(ps) fU |
| 97 | ALD48-a | mA(ps)fA(ps)mUfGmUfUmUfUmCfCmUfGmCfUmGfAmC(ps)fG(p s)mG |
| 98 | ALD48-b | fC(ps)mC(ps)fGmUfCmAfGmCfAmGfGmAfAfAfAmCfA(ps)mU(ps) fU |
| 99 | ALD49-a | mA(ps)fA(ps)mUfGmUfUmUfUmCfCmUfGmCfUmGfAmC(ps)fG(p s)mG |
| 100 | ALD49-b | fC(ps)mC(ps)fGmUfCmAfGmCfAmGfGmAfAmAfAfCfA(ps)mU(ps) fU |
| 101 | ALD50-a | mA(ps)fA(ps)mUfGmUfUmUfUmCfCmUfGmCfUmGfAmC(ps)fG(p s)mG |
| 102 | ALD50-b | fC(ps)mC(ps)fGmUfCmAfGmCfAmGfGmAfAmAfAmCfA(ps)fU(ps) fU |
| 103 | ALD58-a | mAfAmUfGmUfUmUfUmCfCmUfGmCfUmGfAmCfGmG |
| 104 | ALD58-b | fCmCfGmUfCmAfGmCfAmGfGmAfAmAfAmCfAmUfU |
| 105 | ALD59-a | mAfAmUmGmUmUmUmUmCmCmUmGmCmUmGmAmCmGmG |
| 106 | ALD59-b | fCmCfGmUfCmAfGmCfAmGfGmAfAmAfAmCfAmUfU |
| 107 | ALD60-a | mAmAmUmGmUmUmUmUmCmCmUmGmCfUmGmAmCmGmG |
| 108 | ALD60-b | fCmCfGmUfCmAfGmCfAmGfGmAfAmAfAmCfAmUfU |
| 109 | ALD61-a | mAfAmUmGmUmUmUmUmCmCmUmGmCfUmGmAmCmGmG |
| 110 | ALD61-b | fCmCfGmUfCmAfGmCfAmGfGmAfAmAfAmCfAmUfU |
| 111 | ALD62-a | mAfAmUfGmUfUmUfUmCfCmUfGmCfUmGfAmCfGmG |
| 112 | ALD62-b | mCmCmGmUmCmAfGmCmAmGmGmAmAmAmAmCmAmUmU |
| 113 | ALD63-a | mAfAmUfGmUfUmUfUmCfCmUfGmCfUmGfAmCfGmG |
| 114 | ALD63-b | mCmCmGmUmCmAmGmCfAmGmGmAmAmAmAmCmAmUmU |
| 115 | ALD64-a | mAfAmUfGmUfUmUfUmCfCmUfGmCfUmGfAmCfGmG |
| 116 | ALD64-b | mCmCmGmUmCmAfGmCfAmGmGmAmAmAmAmCmAmUmU |
| 117 | ALD72-a | mAfUmGfUmAfGmCfCmGfAmGfGmAfUmCfUmUfCmU |
| 118 | ALD72-b | fAmGfAmAfGmAfUmCfCmUfCmGfGmCfUmAfCmAfU |
| 119 | ALD73-a | mAfUmGmUmAmGmCmCmGmAmGmGmAmUmCmUmUmCmU |
| 120 | ALD73-b | fAmGfAmAfGmAfUmCfCmUfCmGfGmCfUmAfCmAfU |
| 121 | ALD74-a | mAmUmGmUmAmGmCmCmGmAmGmGmAfUmCmUmUmCmU |
| 122 | ALD74-b | fAmGfAmAfGmAfUmCfCmUfCmGfGmCfUmAfCmAfU |
| 123 | ALD75-a | mAfUmGmUmAmGmCmCmGmAmGmGmAfUmCmUmUmCmU |
| 124 | ALD75-b | fAmGfAmAfGmAfUmCfCmUfCmGfGmCfUmAfCmAfU |
| 125 | ALD76-a | mAfUmGfUmAfGmCfCmGfAmGfGmAfUmCfUmUfCmU |
| 126 | ALD76-b | mAmGmAmAmGmAfUmCmCmUmCmGmGmCmUmAmCmAmU |
| 127 | ALD77-a | mAfUmGfUmAfGmCfCmGfAmGfGmAfUmCfUmUfCmU |
| 128 | ALD77-b | mAmGmAmAmGmAmUmCfCmUmCmGmGmCmUmAmCmAmU |
| 129 | ALD78-a | mAfUmGfUmAfGmCfCmGfAmGfGmAfUmCfUmUfCmU |
| 130 | ALD78-b | mAmGmAmAmGmAfUmCfCmUmCmGmGmCmUmAmCmAmU |
| 131 | DGT01-a | mUfUmAfAmAfUmAfAmCfCmCfAmCfAmGfAmCfAmC |
| 132 | DGT01-b | fGmUfGmUfCmUfGmUfGmGfGmUfUmAfUmUfUmAfA |
| 133 | DGT02-a | mUfUmAmAmAmUmAmAmCmCmCmAmCmAmGmAmCmAmC |
| 134 | DGT02-b | fGmUfGmUfCmUfGmUfGmGfGmUfUmAfUmUfUmAfA |
| 135 | DGT03-a | mUmUmAmAmAmUmAmAmCmCmCmAmCfAmGmAmCmAmC |
| 136 | DGT03-b | fGmUfGmUfCmUfGmUfGmGfGmUfUmAfUmUfUmAfA |
| 137 | DGT04-a | mUfUmAmAmAmUmAmAmCmCmCmAmCfAmGmAmCmAmC |
| 138 | DGT04-b | fGmUfGmUfCmUfGmUfGmGfGmUfUmAfUmUfUmAfA |
| 139 | DGT05-a | mUfUmAfAmAfUmAfAmCfCmCfAmCfAmGfAmCfAmC |
| 140 | DGT05-b | mGmUmGmUmCmUfGmUmGmGmGmUmUmAmUmUmUmAmA |
| 141 | DGT06-a | mUfUmAfAmAfUmAfAmCfCmCfAmCfAmGfAmCfAmC |
| 142 | DGT06-b | mGmUmGmUmCmUmGmUfGmGmGmUmUmAmUmUmUmAmA |
| 143 | DGT07-a | mUfUmAfAmAfUmAfAmCfCmCfAmCfAmGfAmCfAmC |
| 144 | DGT07-b | mGmUmGmUmCmUfGmUfGmGmGmUmUmAmUmUmUmAmA |
| 145 | TMP01-a | mAfAmCfCmAfGmAfAmGfAmAfGmCfAmGfGmUfGmA |
| 146 | TMP01-b | fUmCfAmCfCmUfGmCfUmUfCmUfUmCfUmGfGmUfU |
| 147 | TMP93-a | mAfAmCmCmAmGmAmAmGmAmAmGmCmAmGmGmUmGmA |
| 148 | TMP93-b | fUmCfAmCfCmUfGmCfUmUfCmUfUmCfUmGfGmUfU |
| 149 | TMP94-a | mAmAmCmCmAmGmAmAmGmAmAmGmCfAmGmGmUmGmA |
| 150 | TMP94-b | fUmCfAmCfCmUfGmCfUmUfCmUfUmCfUmGfGmUfU |
| 151 | TMP97-a | mAfAmCfCmAfGmAfAmGfAmAfGmCfAmGfGmUfGmA |
| 152 | TMP97-b | mUmCmAmCmCmUfGmCmUmUmCmUmUmCmUmGmGmUmU |
| 153 | TMP98-a | mAfAmCfCmAfGmAfAmGfAmAfGmCfAmGfGmUfGmA |
| 154 | TMP98-b | mUmCmAmCmCmUmGmCfUmUmCmUmUmCmUmGmGmUmU |
| 155 | TMP112-a | mA[A]mCmCmAmGmAmAmGmAmAmGmCfAmGmGmUmGmA |
| 156 | TMP112-b | fUmCfAmCfCmUfGmCfUmUfCmUfUmCfUmGfGmUfU |
| 157 | TMP113-a | mAfAmCmCmAmGmAmAmGmAmAmGmC[A]mGmGmUmGmA |
| 158 | TMP113-b | fUmCfAmCfCmUfGmCfUmUfCmUfUmCfUmGfGmUfU |
| 159 | TMP116-a | mAfAmCfCmAfGmAfAmGfAmAfGmCfAmGfGmUfGmA |
| 160 | TMP116-b | mUmCmAmCmCmU[G]mCfUmUmCmUmUmCmUmGmGmUmU |
| 161 | TMP117-a | mAfAmCfCmAfGmAfAmGfAmAfGmCfAmGfGmUfGmA |
| 162 | TMP117-b | mUmCmAmCmCmUfGmC[U]mUmCmUmUmCmUmGmGmUmU |
| 163 | TMP110-a | mA{A}mCmCmAmGmAmAmGmAmAmGmCfAmGmGmUmGmA |
| 164 | TMP110-b | fUmCfAmCfCmUfGmCfUmUfCmUfUmCfUmGfGmUfU |
| 165 | TMP111-a | mAfAmCmCmAmGmAmAmGmAmAmGmC{A}mGmGmUmGmA |
| 166 | TMP111-b | fUmCfAmCfCmUfGmCfUmUfCmUfUmCfUmGfGmUfU |
| 167 | TMP114-a | mAfAmCfCmAfGmAfAmGfAmAfGmCfAmGfGmUfGmA |
| 168 | TMP114-b | mUmCmAmCmCmU{G}mCfUmUmCmUmUmCmUmGmGmUmU |
| 169 | TMP115-a | mAfAmCfCmAfGmAfAmGfAmAfGmCfAmGfGmUfGmA |
| 170 | TMP115-a | mUmCmAmCmCmUfGmC{U}mUmCmUmUmCmUmGmGmUmU |
| 171 | STS12009V2 3L4-a | mA(ps)fA(ps)mCmCmAmGmAmAmGmAmAfGmCfAmGmGmU(p s)mG(ps)mA |
| 172 | STS 12009V2 3L4-b | GalNAc- mUmCmAmCmCmUmGmCmUmUmCmUmUmCmUmGmG(ps)m U(ps)mU |
| 173 | STS 12009V2 5L4-a | mA(ps)fA(ps)mCmCmAmGmAmAmGmAmAfGmCfAmGmGmU(p s)mG(ps)mA |
| 174 | STS 12009V2 5L4-b | GalNAc- mUmCmAmCmCmUmGmCfUmUmCmUmUmCmUmGmG(ps)mU (ps)mU |
| 175 | STS 12009V2 6L4-a | mA(ps)fA(ps)mCmCmAmGmAmAmGmAmAfGmCfAmGmGmU(p s)mG(ps)mA |
| 176 | STS 12009V2 6L4-b | GalNAc- mUmCmAmCmCmUfGmCmUmUmCmUmUmCmUmGmG(ps)mU (ps)mU |
| 177 | STS 12009V2 7L4-a | mA(ps)fA(ps)mCmCmAmGmAmAmGmAmAfGmCfAmGmGmU(p s)mG(ps)mA |
| 178 | STS 12009V2 7L4-b | GalNAc- mUmCmAmCmCmUfGfCfUmUmCmUmUmCmUmGmG(ps)mU(p s)mU |
| 179 | STS12009V4 1L4-a | mA(ps)fA(ps)mCmCmAmGmAmAmGmAmAfGmCfAmGmGmU(p s)mG(ps)mA |
| 180 | STS12009V4 1L4-b | GalNAc- fUmCfAmCfCmUfGmCfUmUfCmUfUmCfUmGfG(ps)mU(ps)fU |
| 181 | TMP70-a | mA(ps)fA(ps)mCfCmAfGmAfAmGfAmAfGmCfAmGfGmU(ps)fG(p s)mA |
| 182 | TMP70-b | fU(ps)mC(ps)fAmCfCmUfGmCfUmUfCmUfUmCfUmGfG(ps)mU(p s)fU |
| 183 | TMP119-A | mA(ps)fA(ps)mCfCmAfGmAfAmGfAmAfGmCfAmGfGmU(ps)fG(p s)mA |
| 184 | TMP119-B | mU(ps)mC(ps)mAmCmCmUfGfCfUmUmCmUmUmCmUmGmG(p s)mU(ps)mU |
| 185 | TMP120-A | mA(ps)fA(ps)mCmCmAmGmAmAmGmAmAmGmCfAmGmGmU( ps)mG(ps)mA |
| 186 | TMP120-B | mU(ps)mC(ps)mAmCmCmUfGfCfUmUmCmUmUmCmUmGmG(p s)mU(ps)mU |
| 187 | TMP121-A | mA(ps)fA(ps)mCfCmAfGmAfAmGfAmAfGmCfAmGfGmU(ps)fG(p s)mA |
| 188 | TMP121-B | mU(ps)mC(ps)mAmCmCmU[G][C][T]mUmCmUmUmCmUmGmG (ps)mU(ps)mU |
| 189 | TMP122-A | mA(ps)fA(ps)mCfCmAfGmAfAmGfAmAfGmCfAmGfGmU(ps)fG(p s)mA |
| 190 | TMP122-B | mU(ps)mC(ps)mAmCmCmU[G]mC[T]mUmCmUmUmCmUmGmG (ps)mU(ps)mU |
| 191 | TMP123-A | mA(ps)fA(ps)mCmCmAmGmAmAmGmAmAmGmCfAmGmGmU( ps)mG(ps)mA |
| 192 | TMP123-B | mU(ps)mC(ps)mAmCmCmU[G][C][T]mUmCmUmUmCmUmGmG (ps)mU(ps)mU |
| 193 | TMP124-A | mA(ps)[A](ps)mCmCmAmGmAmAmGmAmAmGmC[A]mGmGmU (ps)mG(ps)mA |
| 194 | TMP124-B | mU(ps)mC(ps)mAmCmCmU[G][C][T]mUmCmUmUmCmUmGmG (ps)mU(ps)mU |
| 195 | TMP125-A | mA(ps)[A](ps)mCmCmAmGmAmAmGmAmAmGmCfAmGmGmU( ps)mG(ps)mA |
| 196 | TMP125-B | mU(ps)mC(ps)mAmCmCmU[G][C][T]mUmCmUmUmCmUmGmG (ps)mU(ps)mU |
| 197 | TMP126-A | mA(ps)[A](ps)mCmCmAmGmAmAmGmAmAmGmCAmGmGmU( ps)mG(ps)mA |
| 198 | TMP126-B | mU(ps)mC(ps)mAmCmCmU[G][C][T]mUmCmUmUmCmUmGmG (ps)mU(ps)mU |
| 199 | ALD91-A | mAfAmUmGmUmUmUmUmCmCmUmGmC [T] mGmAmCmGmG |
| 200 | ALD91-B | fCmCfGmUfCmAfGmCfAmGfGmAfAmAfAmCfAmUfU |
| 201 | ALD92-A | mA [A] mUmGmUmUmUmUmCmCmUmGmC [T] mGmAmCmGmG |
| 202 | ALD92-B | fCmCfGmUfCmAfGmCfAmGfGmAfAmAfAmCfAmUfU |
| 203 | ALD93-A | mAfAmUfGmUfUmUfUmCfCmUfGmCfUmGfAmCfGmG |
| 204 | ALD93-B | mCmCmGmUmCmAfGmCfAmGmGmAmAmAmAmCmAmUmU |
| 205 | ALD94-A | mAfAmUfGmUfUmUfUmCfCmUfGmCfUmGfAmCfGmG |
| 206 | ALD94-B | mCmCmGmUmCmA[G]mCfAmGmGmAmAmAmAmCmAmUmU |
| 207 | ALD95-A | mAfAmUfGmUfUmUfUmCfCmUfGmCfUmGfAmCfGmG |
| 208 | ALD95-B | mCmCmGmUmCmAfGmC[A]mGmGmAmAmAmAmCmAmUmU |
| 209 | ALD96-A | mAfAmUfGmUfUmUfUmCfCmUfGmCfUmGfAmCfGmG |
| 210 | ALD96-B | mCmCmGmUmCmA [G] mC [A] mGmGmAmAmAmAmCmAmUmU |
| 211 | ALD97-A | mAfAmUfGmUfUmUfUmCfCmUfGmCfUmGfAmCfGmG |
| 212 | ALD97-B | mCmCmGmUmCmAfGfCfAmmGmGmAmAmAmAmCmAmUmU |
| 213 | ALD98-A | mAfAmUfGmUfUmUfUmCfCmUfGmCfUmGfAmCfGmG |
| 214 | ALD98-B | mCmCmGmUmCmA [G] [C] [A] mGmGmAmAmAmAmCmAmUmU |
| 215 | ALD99-A | mAfUmGfUmAfGmCfCmGfAmGfGmAfUmCfUmUfCmU |
| 216 | ALD99-B | fAmGfAmAfGmAfUmCfCmUfCmGfGmCfUmAfCmAfU |
| 217 | ALD100-A | mAfUmGmUmAmGmCmCmGmAmGmGmA [T] mCmUmUmCmU |
| 218 | ALD100-B | fAmGfAmAfGmAfUmCfCmUfCmGfGmCfUmAfCmAfU |
| 219 | ALD101-A | mA [T] mGmUmAmGmCmCmGmAmGmGmA [T] mCmUmUmCmU |
| 220 | ALD101-B | fAmGfAmAfGmAfUmCfCmUfCmGfGmCfUmAfCmAfU |
| 221 | ALD102-A | mAfUmGfUmAfGmCfCmGfAmGfGmAfUmCfUmUfCmU |
| 222 | ALD102-B | mAmGmAmAmGmAfUmCfCmUmCmGmGmCmUmAmCmAmU |
| 223 | ALD103-A | mAfUmGfUmAfGmCfCmGfAmGfGmAfUmCfUmUfCmU |
| 224 | ALD103-B | mAmGmAmAmGmA[T]mCfCmUmCmGmGmCmUmAmCmAmU |
| 225 | ALD104-A | mAfUmGfUmAfGmCfCmGfAmGfGmAfUmCfUmUfCmU |
| 226 | ALD104-B | mAmGmAmAmGmAmUmC [C] mUmCmGmGmCmUmAmCmAmU |
| 227 | ALD105-A | mAfUmGfUmAfGmCfCmGfAmGfGmAfUmCfUmUfCmU |
| 228 | ALD105-B | mAmGmAmAmGmA [U] mC [C] mUmCmGmGmCmUmAmCmAmU |
| 229 | ALD106-A | mAfUmGfUmAfGmCfCmGfAmGfGmAfUmCfUmUfCmU |
| 230 | ALD106-B | mAmGmAmAmGmAfUfCfCmUmCmGmGmCmUmAmCmAmU |
| 231 | ALD107-A | mAfUmGfUmAfGmCfCmGfAmGfGmAfUmCfUmUfCmU |
| 232 | ALD107-B | mAmGmAmAmGmA [T] [C] [C] mUmCmGmGmCmUmmAmCmAmU |
| 233 | DGT11-A | mU [T] mAmAmAmUmAmAmmCmCmCmAmCfAmGmAmCmAmC |
| 234 | DGT11-B | fGmUfGmUfCmUfGmUfGmGfGmUfUmAfUmUfUmAfA |
| 235 | DGT12-A | mUfUmAmAmAmUmAmAmCmCmCmAmC[A]mGmAmCmAmC |
| 236 | DGT12-B | fGmUfGmUfCmUfGmUfGmGfGmUfUmAfUmUfUmAfA |
| 237 | DGT13-A | mUfUmAmAmAmUmAmAmCmCmCmAmC[A]mGmAmCmAmC |
| 238 | DGT13-B | fGmUfGmUfCmUfGmUfGmGfGmUfUmAfUmUfUmAfA |
| 239 | DGT14-A | mU [T] mAmAmAmUmAmAmCmCmCmAmC [A] mGmAmCmAmC |
| 240 | DGT14-B | fGmUfGmUfCmUfGmUfGmGfGmUfUmAfUmUfUmAfA |
| 241 | DGT15-A | mUfUmAfAmAfUmAfAmCfCmCfAmCfAmGfAmCfAmC |
| 242 | DGT15-B | mGmUmGmUmCmUfGmUfGmGmGmUmUmAmUmUmUmAmA |
| 243 | DGT16-A | mUfUmAfAmAfUmAfAmCfCmCfAmCfAmGfAmCfAmC |
| 244 | DGT16-B | mGmUmGmUmCmUfGmU [G] mGmGmUmUmAmUmUmUmAmA |
| 245 | DGT17-A | mUfUmAfAmAfUmAfAmCfCmCfAmCfAmGfAmCfAmC |
| 246 | DGT17-B | mGmUmGmUmCmU [G] mU [G] mGmGmUmUmAmUmUmUmAmA |
| 247 | DGT18-A | mUfUmAfAmAfUmAfAmCfCmCfAmCfAmGfAmCfAmC |
| 248 | DGT18-B | mGmUmGmUmCmUfGfUfGmGmGmUmUmAmUmUmUmAmA |
| 249 | DGT19-A | mUfUmAfAmAfUmAfAmCfCmCfAmCfAmGfAmCfAmC |
| 250 | DGT19-B | mGmUmGmUmCmU [G] [T] [G] mGmGmUmUmAmUmUmUmAmA |
| 251 | ALD108-A | mA (ps) fU (ps) mGmUmAmGmCmCmGmAmGmGmAfUmCmUmU (ps) mC (ps) mU |
| 252 | ALD108-B | mA (ps) mG (ps) mAmAmGmAfUmCfCmUmCmGmGmCmUmAmC (ps) mA (ps) mU |
| 253 | ALD115-A | mA (ps) (MOE-U) (ps) mGmUmAmGmCmCmGmAmGmGmAfUmCmUmU (ps) mC (ps) mU |
| 254 | ALD115-B | mA (ps) mG (ps) mAmAmGmAfUmCfCmUmCmGmGmCmUmAmC (ps) mA (ps) mU |
| 255 | ALD116-A | mA (ps) fU (ps) mGmUmAmGmCmCmGmAmGmGmA (MOE-U) mCmUmU (ps) mC (ps) mU |
| 256 | ALD116-B | mA (ps) mG (ps) mAmAmGmAfUmCfCmUmCmGmGmCmUmAmC (ps) mA (ps) mU |
| 257 | ALD117-A | mA (ps) (MOE-U) (ps) mGmUmAmGmCmCmGmAmGmGmA (MOE-U) mCmUmU (ps) mC (ps) mU |
| 258 | ALD117-B | mA (ps) mG (ps) mAmAmGmAfUmCfCmUmCmGmGmCmUmAmC (ps) mA (ps) mU |
| 259 | ALD118-A | mA (ps) fU (ps) mGmUmAmGmCmCmGmAmGmGmAfUmCmUmU (ps) mC (ps) mU |
| 260 | ALD118-B | mA (ps) mG (ps) mAmAmGmA (MOE-U) mCfCmUmCmGmGmCmUmAmC (ps) mA (ps) mU |
| 261 | ALD119-A | mA (ps) fU (ps) mGmUmAmGmCmCmGmAmGmGmAfUmCmUmU (ps) mC (ps) mU |
| 262 | ALD119-B | mA (ps) mG (ps) mAmAmGmAfUmC (MOE-C) mUmCmGmGmCmUmAmC (ps) mA (ps) mU |
| 263 | ALD120-A | mA (ps) fU (ps) mGmUmAmGmCmCmGmAmGmGmAfUmCmUmU (ps) mC (ps) mU |
| 264 | ALD120-B | mA (ps) mG (ps) mAmAmGmA (MOE-U) mC (MOE-C) mUmCmGmGmCmUmAmC (ps) mA (ps) mU |
| 265 | CLC28-a | AUGCAAAAUACACUUCUAC |
| 266 | CLC28-b | GUAGAAGUGUAUUUUGCAU |
| 267 | HFE04-a | AUUGAUAGAACCAUCUUCA |
| 268 | HFE04-b | UGAAGAUGGUUCUAUCAAU |
| 269 | ALD01-a | AAUGUUUUCCUGCUGACGG |
| 270 | ALD01-b | CCGUCAGCAGGAAAACAUU |
| 271 | ALD72-a | AUGUAGCCGAGGAUCUUCU |
| 272 | ALD72-b | AGAAGAUCCUCGGCUACAU |
| 273 | DGT01-a | UUAAAUAACCCACAGACAC |
| 274 | DGT01-b | GUGUCUGUGGGUUAUUUAA |
| 275 | TMP01-a | AACCAGAAGAAGCAGGUGA |
| 276 | TMP01-b | UCACCUGCUUCUUCUGGUU |
| 277 | STS 12009V2 3L4-a | AACCAGAAGAAGCAGGUGA |
| 278 | STS 12009V2 3L4-b | UCACCUGCUUCUUCUGGUU |

### Key

A, U, C, G - RNA
mA, mU, mC, mG - 2'-OMe RNA
fA, fU, fC, fG - 2'-F RNA
(ps) - phosphorothioate
[A], [U], [C], [G] - 2'-H (DNA)
{A}, {U}, {C}, {G} - LNA
GalNAc - [ST23 (ps)]3 ST41 (ps)
(MOE-U), (MOE-C) - 2'methoxyethyl RNA

The following abbreviations may be used :

| | |
|---|---|
| ivN | Inverted nucleotide, either 3'-3' or 5'-5' |
| (ps2) | Phosphorodithioate |
| vinylphosphonate | Vinyl-(E)-phosphonate |
| FAM | 6-Carboxyfluorescein |
| TAMRA | 5-Carboxytetramethylrhodamine |
| BHQ1 | Black Hole Quencher 1 |
| (ps) | Phosphorothioate |
| GN | 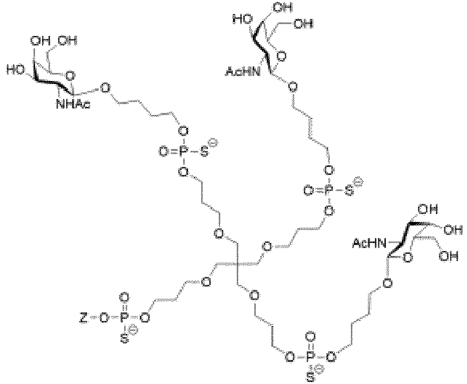 |
| GN2 | 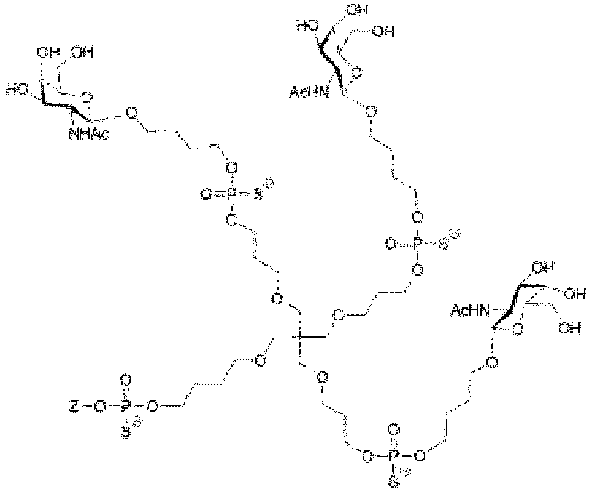 |
| GN3 | 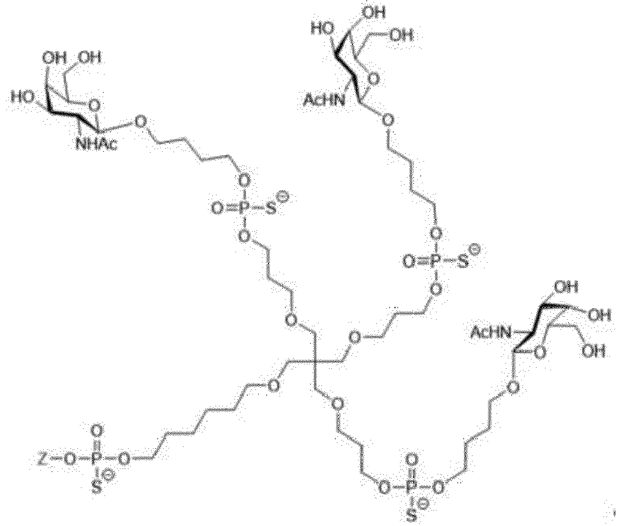 |
| GNo | Same as GN2 but with phosphodiesters instead of phosphorothioates |
| ST23 | 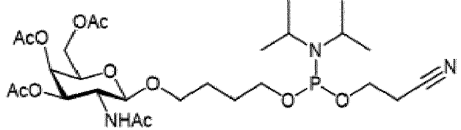 |
| ST41/C4XLT | 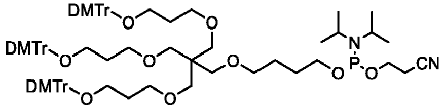 |
| | ST41 |
| ST43/C6XLT | 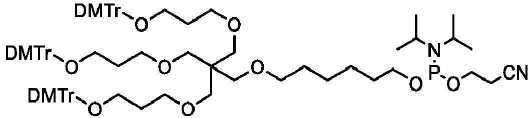 |
| Long trebler/ltrb/STKS | 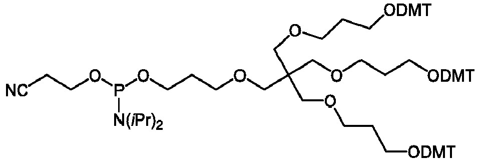 |
| Ser(GN) | 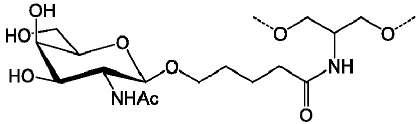 |
| GlyC3Am(GalNA c) | 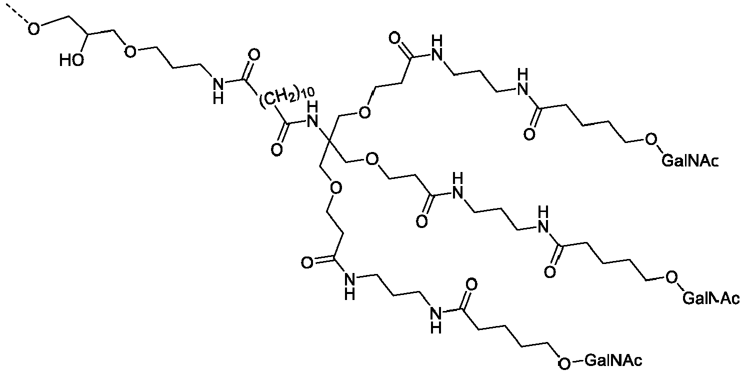 |
| GalNAc (only in when used in sequences) | GN2 (see above) |
| (MOE-U), (MOE-C) | 2'methoxyethyl RNA |
| {A}, {U}, {C}, {G} | LNA |
| [ST23 (ps)]3 ST41 (ps) | GN2 (see above) |
| [ST23 (ps)]3 ST43 (ps) | GN3 (see above) |
| ST23(ps) long trebler(ps) | GN (see above) |

### Statements of invention

1. Nucleic acid for inhibiting expression of a target gene in a cell, comprising at least one duplex region that comprises at least a portion of a first strand and at least a portion of a second strand that is at least partially complementary to the first strand, wherein said first strand is at least partially complementary to at least a portion of RNA transcribed from said target gene, wherein said first strand includes modified nucleotides or unmodified nucleotides at a plurality of positions in order to facilitate processing of the nucleic acid by RISC.
2. Nucleic acid of statement 1, wherein the nucleotides at positions 2 and 14 from the 5' end of the first strand are modified.
3. Nucleic acid of statement 1 or statement 2, wherein the nucleotides at positions 2 and 14 from the 5' end of the first strand are not modified with a 2' O-methyl modification.
4. Nucleic acid of any one of statements 1 to 3, wherein the nucleotides at positions 2 and 14 from the 5' end of the first strand are not modified with a modification selected from the group consisting of 2-O-(2-Methoxyethyl), 2'-O-allyl, 2'-O-DNP, 2'-CE, 2'-EA, 2'-AEM, 2'-APM and 2'-GE.
5. Nucleic acid of any one of statements 1 to 3, wherein the nucleotides at positions 2 and 14 from the 5' end of the first strand are modified with a modification selected from the group consisting of 2'F, 4'-S, 2'-FANA and UNA.
6. Nucleic acid of statement 1, wherein the nucleotides at positions 2 and 14 from the 5' end of the first strand are unmodified.
7. Nucleic acid of any one of statements 1 to 6, wherein in the second strand, nucleotides at position 2 and 14 from the 5' end of the second strand are modified with a 2' O-methyl modification or with a '-O-(2-Methoxyethyl) modification.
8. Nucleic acid of any one of statements 1 to 7, wherein the first strand and the second strand are separate strands
9. Nucleic acid of any one of statements 1 to 7, comprising a single strand that comprises the first strand and the second strand.
10. Nucleic acid according to any one of statements 1 to 9, wherein said first strand and/or said second strand are each from 17-35 nucleotides in length
11. Nucleic acid of any one of statements 1 to 10, wherein the at least one duplex region consists of 19-25 nucleotide base pairs.
12. Nucleic acid of any preceding statement, which
   a) is blunt ended at both ends; or
   b) has an overhang at one end and a blunt end at the other; or
   c) has an overhang at both ends.
13. Nucleic acid according to any preceding statement, wherein one or more nucleotides on the first and / or second strand are modified, to form modified nucleotides.
14. Nucleic acid of statement 13, wherein one or more of the odd numbered nucleotides of the first strand are modified.
15. Nucleic acid according to statement 14, wherein one or more of the even numbered nucleotides of the first strand are modified by at least a second modification, wherein the at least second modification is different from the modification of statement 14.
16. Nucleic acid of statement 15, wherein at least one of the one or more modified even numbered nucleotides is adjacent to at least one of the one or more modified odd numbered nucleotides.
17. Nucleic acid of any of statements 14 to 16, wherein a plurality of odd numbered nucleotides are modified.
18. Nucleic acid of statement 15 or 17, wherein a plurality of even numbered nucleotides are modified by a second modification.
19. Nucleic acid of any of statements 13 to 18, wherein the first strand comprises adjacent nucleotides that are modified by a common modification.
20. Nucleic acid of any of statements 14 to 19, wherein the first strand comprises adjacent nucleotides that are modified by a second modification that is different to the modification of statement 14.
21. Nucleic acid of any of statements 14 to 20, wherein one or more of the odd numbered nucleotides of the second strand are modified by a modification that is different to the modification of statement 14.
22. Nucleic acid according to any of statements 14 to 21, wherein one or more of the even numbered nucleotides of the second strand are modified by the modification of statement 14.
23. Nucleic acid of statement 21 or 22, wherein at least one of the one or more modified even numbered nucleotides of the second strand is adjacent to the one or more modified odd numbered nucleotides.
24. Nucleic acid of any of statements 21 to 23, wherein a plurality of odd numbered nucleotides of the second strand are modified by a common modification.
25. Nucleic acid of any of statements 21 to 24, wherein a plurality of even numbered nucleotides are modified by a modification according to statement 14.
26. Nucleic acid of any of statements 21 to 25, wherein a plurality of odd numbered nucleotides are modified by a second modification, wherein the second modification is different from the modification of statement 14.
27. Nucleic acid of any of statements 21 to 26, wherein the second strand comprises adjacent nucleotides that are modified by a common modification.
28. Nucleic acid of any of statements 21 to 27, wherein the second strand comprises adjacent nucleotides that are modified by a second modification that is different from the modification of statement 14.
29. Nucleic acid of statements 13 to 28, wherein each of the odd numbered nucleotides in the first strand and each of the even numbered nucleotides in the second strand are modified with a common modification.
30. Nucleic acid of statement 29, wherein each of the even numbered nucleotides are modified in the first strand with a second modification and each of the odd numbered nucleotides are modified in the second strand with a second modification, provided that positions 2 and 14 are not modified with a 2'OMe.
31. Nucleic acid according to any of statements 13 to 30, wherein the modified nucleotides of the first strand are shifted by at least one nucleotide relative to the unmodified or differently modified nucleotides of the second strand.
32. Nucleic acid according to any one of statements 13 to 31, wherein the first modification and the second modification are each and individually selected from the group consisting of 3'-terminal deoxy-thymine, 2'-O-methyl, 2'-deoxy-modification, a locked nucleotide, an abasic nucleotide, a 2'-amino-modification, a 2'-alkyl-modification, a morpholino modification, a phosphoramidate modification, a non-natural base comprising nucleotide, 5'-phosphorothioate group modification, a 5' phosphate or 5' phosphate mimic modification and a cholesteryl derivative or a dodecanoic acid bisdecylamide group modification.
33. Nucleic acid of any preceding statement wherein the modification nucleotide is any one of a locked nucleotide, an abasic nucleotide or a non-natural base comprising nucleotide.
34. Nucleic acid according to any one of statements 13 to 33, wherein the first modification is 2'-O-methyl.
35. Nucleic acid of any one of statements 13 to 34, wherein the second modification is 2'-F.
36. Nucleic acid according to any one of statements 1 to 35, conjugated with a ligand.
37. Nucleic acid according to any one of statements 1 to 36, wherein the linkage between the terminal one, two or three 3' and/or 5' nucleotides on the first and/or the second strand of the Nucleic acid comprises a phosphorothioate linkage.
38. Nucleic acid according to any one of statements 1 to 37, wherein both the 5' and 3' terminal ends of the first strand and the 3' end of the second strand comprise two phosphorothioate linkages.
39. Nucleic acid for inhibiting expression of a target gene in a cell, comprising at least one duplex region that comprises at least a portion of a first strand and at least a portion of a second strand that is at least partially complementary to the first strand, wherein said first strand is at least partially complementary to at least a portion of a RNA transcribed from said target gene, wherein said first strand includes modified nucleotides or modified nucleotides at a plurality of positions in order to facilitate processing of the nucleic acid by RISC, and wherein the nucleic acid is conjugated to a ligand.
40. Nucleic acid according to any of statements 36 to 39, wherein the ligand comprises one or more GalNac ligands or derivatives thereof.
41. Nucleic acid according to any of statements 36 to 40, wherein the ligand is conjugated to nucleic acid as defined in any preceding statements by a bivalent or trivalent branched linker.
42. Nucleic acid of statement 39 to 41, wherein the nucleotides are modified as defined in any preceding statements.
43. A nucleic acid of any of statements 36 to 42, wherein the ligand comprises the formula I:

   [S-X¹-p-X²]₃-A-X³- (I)

   wherein:
   S represents a saccharide, wherein the saccharide is N-acetyl galactosamine;
   X¹ represents C₃-C₆ alkylene or (-CH₂-CH₂-O)m(-CH₂)₂- wherein m is 1, 2, or 3;
   P is a phosphate or modified phosphate (preferably a thiophosphate);
   X² is alkylene or an alkylene ether of the formula (-CH₂)ₙ-O-CH₂- where n = 1- 6;
   A is a branching unit;
   X³ represents a bridging unit;
   wherein a nucleic acid according to the present invention is conjugated to X³ via a phosphate or modified phosphate (preferably a thiophosphate).
44. A conjugated nucleic acid having one of the following structures 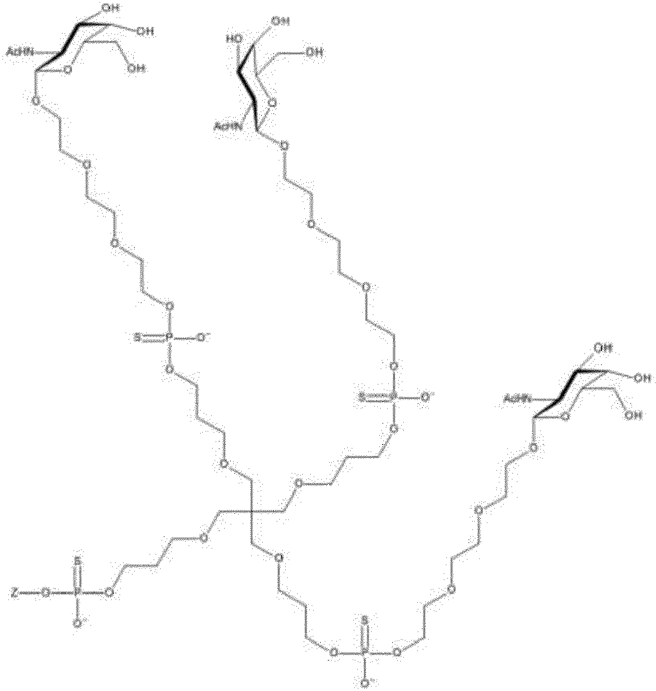 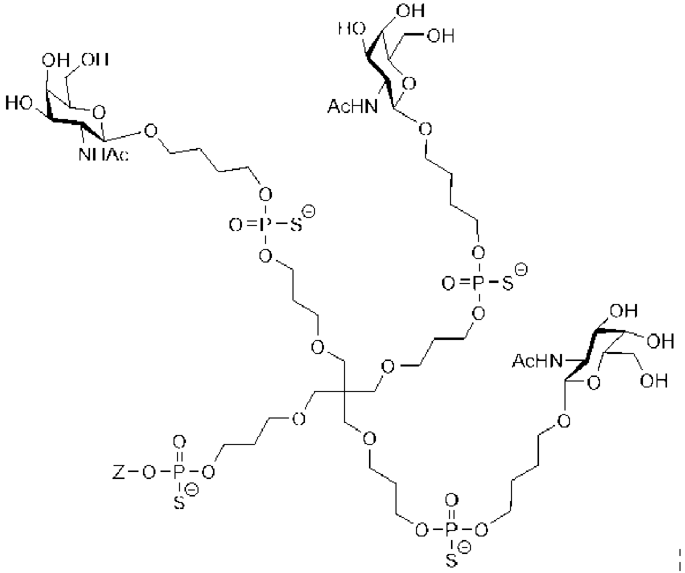 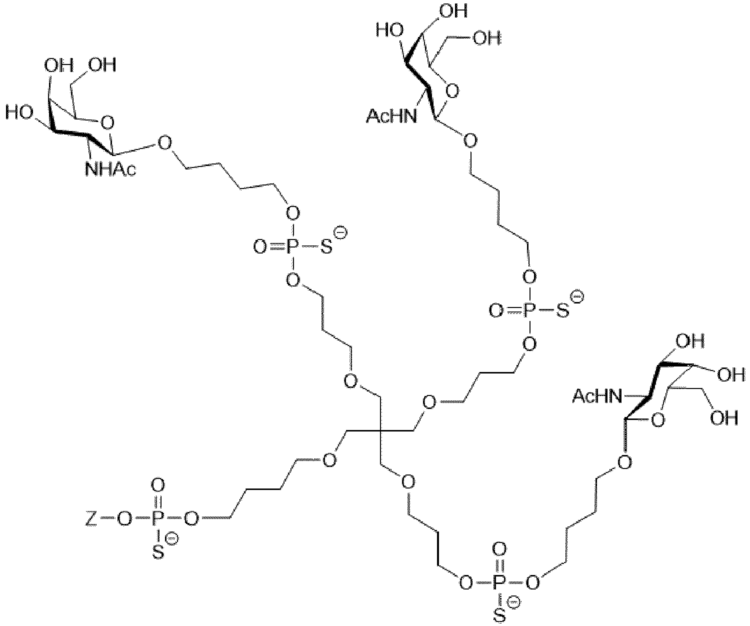 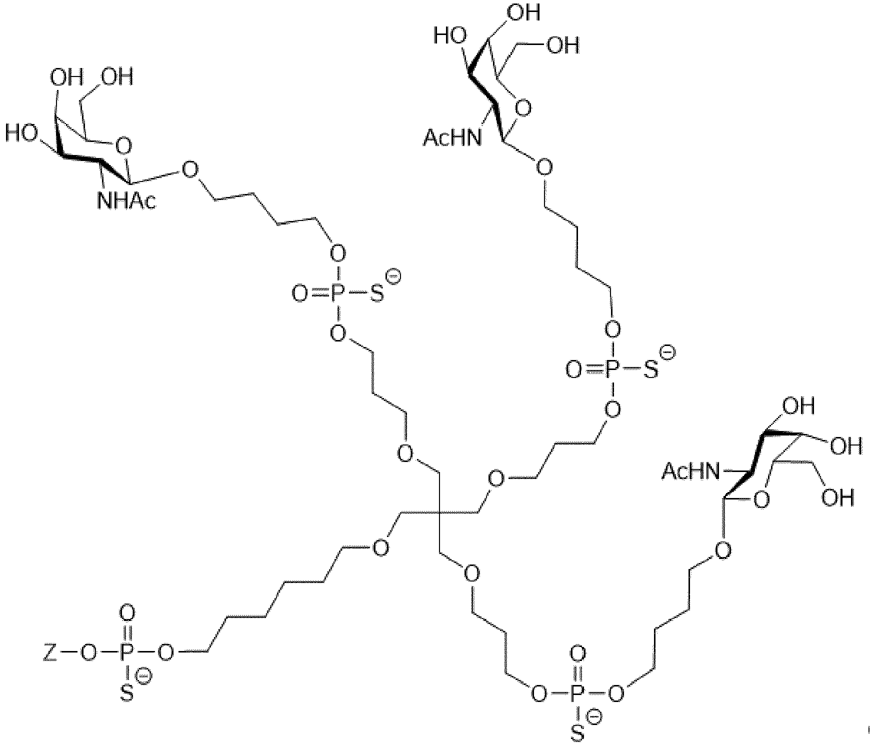 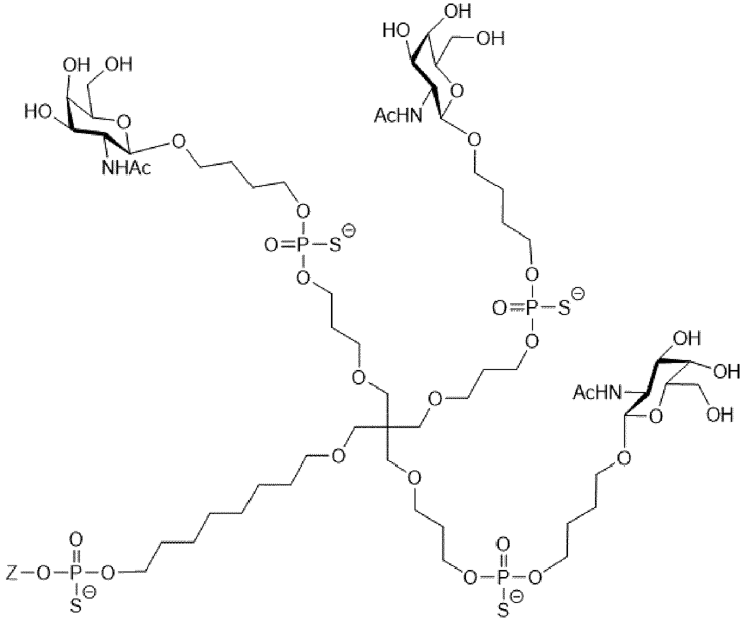 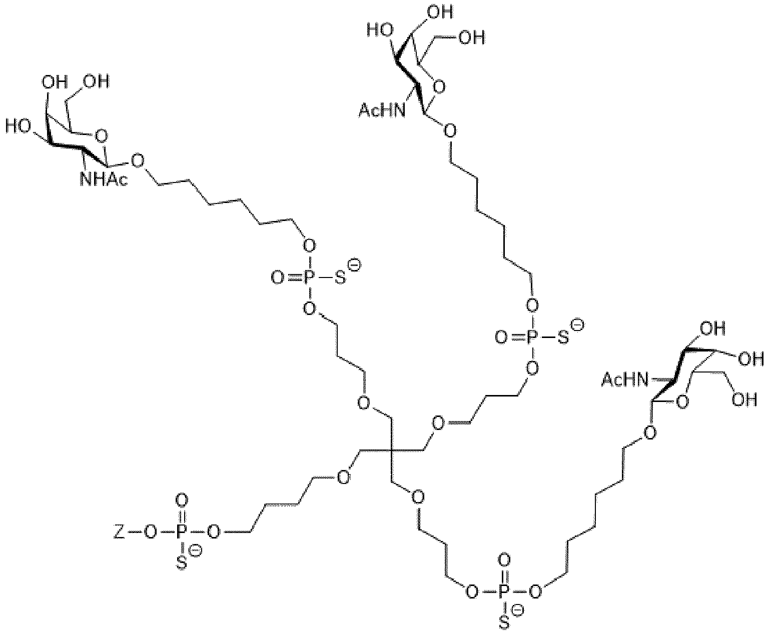 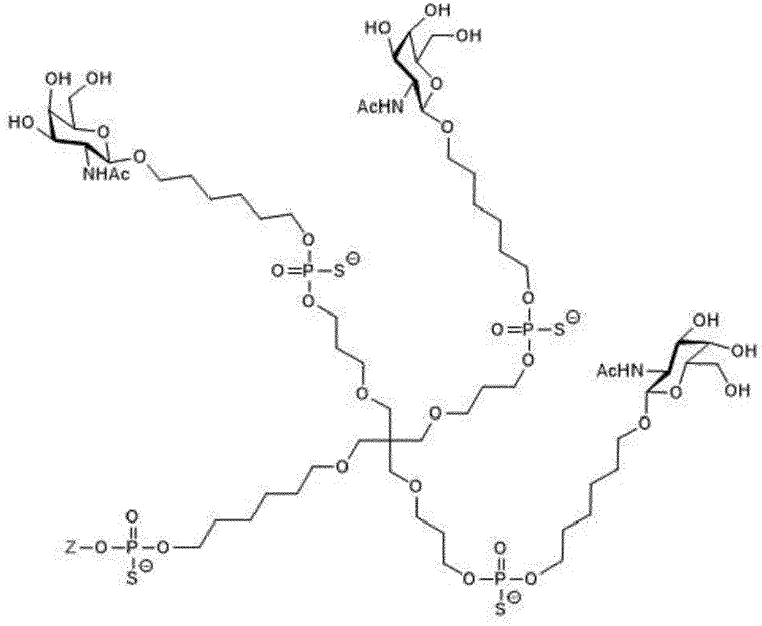 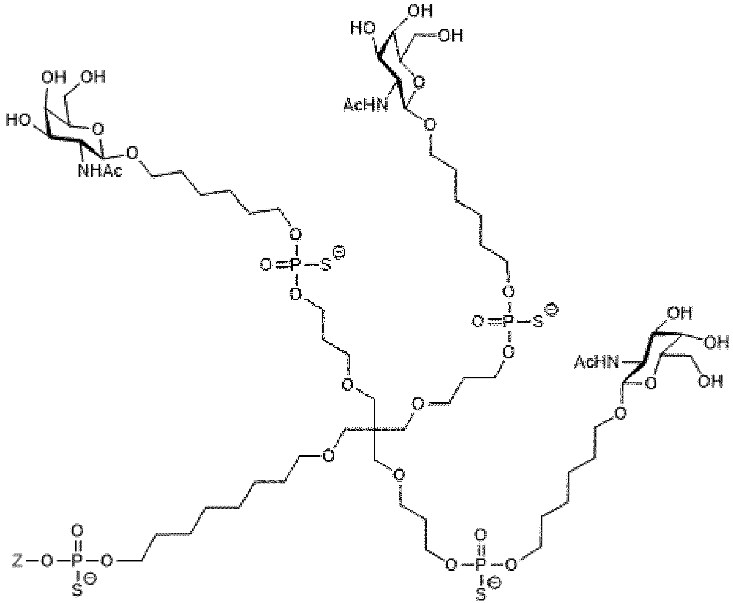 wherein Z is a nucleic acid according to any of claims 1 to 35.
45. A nucleic acid of any one of statements 39 to 44, wherein the ligand comprises 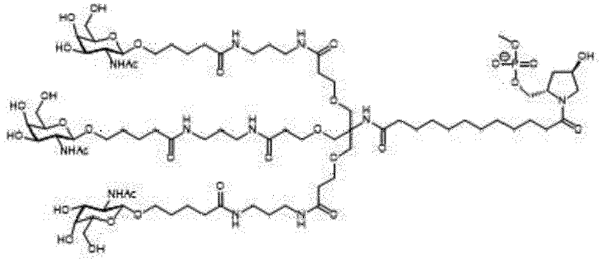
46. A nucleic acid or conjugated nucleic acid of any preceding statement, wherein the duplex comprises separate strands.
47. A nucleic acid or conjugated nucleic acid of any preceding statement, wherein the duplex comprises a single strand comprising a first strand and a second strand.
48. A composition comprising a nucleic acid or conjugated nucleic acid as defined in any of statements 1 to 47 and a formulation comprising:
   i) a cationic lipid, or a pharmaceutically acceptable salt thereof;
   ii) a steroid;
   iii) a phosphatidylethanolamine phospholipid;
   iv) a PEGylated lipid.
49. A composition according to statement 48 wherein in the formulation, the content of the cationic lipid component is from about 55 mol% to about 65 mol% of the overall lipid content of the lipid formulation, preferably about 59 mol% of the overall lipid content of the lipid formulation.
50. A composition
   in statement 48 or 49, wherein the formulation comprises;
   A cationic lipid having the structure; 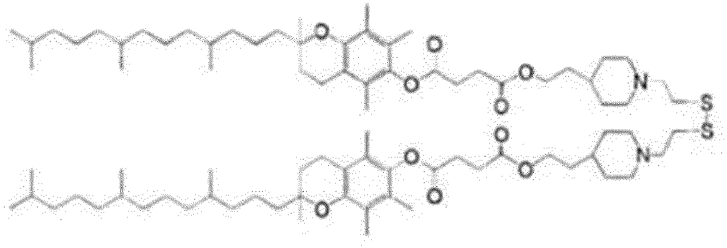 the steroid has the structure; 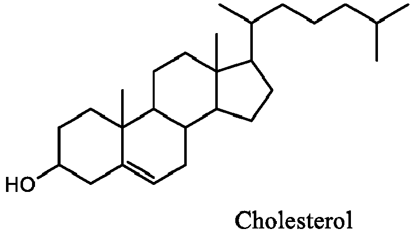 the a phosphatidylethanolamine phospholipid has the structure; 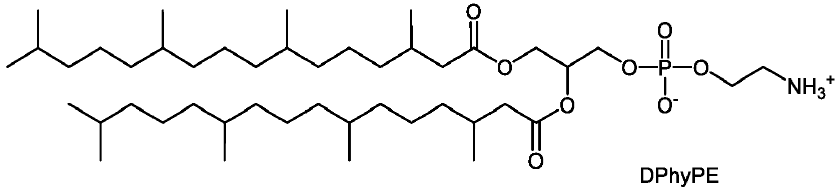 And the PEGylated lipid has the structure; 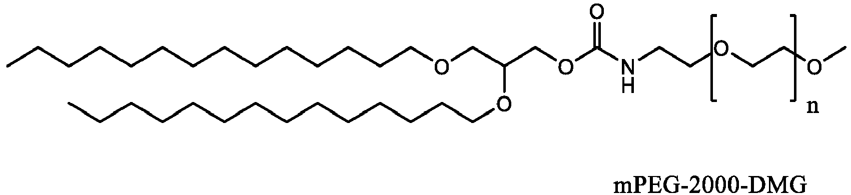
51. A composition comprising a nucleic acid or conjugated nucleic acid of any of statements 1 to 47 and a physiologically acceptable excipient.
52. A nucleic acid or conjugated nucleic acid according to any of statements 1 to 47 for use in the treatment of a disease or disorder.
53. Use of a nucleic acid or conjugated nucleic acid according to any of statements 1 to 47 in the manufacture of a medicament for treating a disease or disorder.
54. A method of treating a disease or disorder comprising administration of a composition comprising a nucleic acid or conjugated nucleic acid according to any of statements 1 to 47 to an individual in need of treatment.
55. The method of statement 54, wherein the nucleic acid or conjugated nucleic acid is administered to the subject subcutaneously or intravenously.
56. A process for making a nucleic acid or conjugated nucleic acid of any one of statements 1 to 47.

Other clauses of the invention include:
1. A conjugate for inhibiting expression of a target gene in a cell, said conjugate comprising a nucleic acid portion and ligand portions, said nucleic acid portion comprising at least one duplex region that comprises at least a portion of a first RNA strand and at least a portion of a second RNA strand that is at least partially complementary to the first strand, wherein said first strand is at least partially complementary to at least a portion of RNA transcribed from said target gene, said ligand portions comprising a serinol-derived linker moiety and a targeting ligand for *in vivo* targeting of cells and being conjugated exclusively to the 3' and/or 5' ends of one or both RNA strands, wherein the 5' end of the first RNA strand is not conjugated, wherein:
   (i) the second RNA strand is conjugated at the 5' end to the targeting ligand, and wherein (a) the second RNA strand is also conjugated at the 3' end to the targeting ligand and the 3' end of the first RNA strand is not conjugated; or (b) the first RNA strand is conjugated at the 3' end to the targeting ligand and the 3' end of the second RNA strand is not conjugated; or (c) both the second RNA strand and the first RNA strand are also conjugated at the 3' ends to the targeting ligand; or
   (ii) both the second RNA strand and the first RNA strand are conjugated at the 3' ends to the targeting ligand and the 5' end of the second RNA strand is not conjugated.
2. The conjugate according to clause 1 wherein the second RNA strand is conjugated at the 5' end to the targeting ligand, the second RNA strand is also conjugated at the 3' end to the targeting ligand and the 3' end of the first RNA strand is not conjugated.
3. The conjugate according to clause 1 wherein the second RNA strand is conjugated at the 5' end to the targeting ligand, the first RNA strand is conjugated at the 3' end to the targeting ligand and the 3' end of the second RNA strand is not conjugated.
4. The conjugate according to clause 1 wherein the second RNA strand is conjugated at the 5' end to the targeting ligand and both the second RNA strand and the first RNA strand are also conjugated at the 3' ends to the targeting ligand.
5. The conjugate according to clause 1 wherein both the second RNA strand and the first RNA strand are conjugated at the 3' ends to the targeting ligand and the 5' end of the second RNA strand is not conjugated.
6. The conjugate according to any one of clauses 1 to 5 wherein the ligands are monomeric ligands.
7. The conjugate according to any one of clauses 1 to 6 wherein the ligands are selected from GalNAc and galactose moieties, especially GalNAc moieties.
8. The conjugate according to any one of clauses 1 to 7 wherein the conjugated RNA strands are conjugated to a targeting ligand via a serinol-derived linker moiety including a further linker wherein the further linker is or comprises a saturated, unbranched or branched C₁₋₁₅ alkyl chain, wherein optionally one or more carbons (for example 1, 2 or 3 carbons, suitably 1 or 2, in particular 1) is/are replaced by a heteroatom selected from O, N, S(O)ₚ wherein p is 0, 1 or 2, (for example a CH2 group is replaced with O, or with NH, or with S, or with SO₂ or a -CH3 group at the terminus of the chain or on a branch is replaced with OH or with NH2) wherein said chain is optionally substituted by one or more oxo groups (for example 1 to 3, such as 1 group).
9. The conjugate according to clause 8 wherein the further linker comprises a saturated, unbranched C₁₋₁₅ alkyl chain wherein one or more carbons (for example 1, 2 or 3 carbons, suitably 1 or 2, in particular 1) is/are replaced by an oxygen atom.
10. The conjugate according to clause 9 wherein the further linker comprises a PEG-chain.
11. The conjugate according to clause 8 wherein the further linker comprises a saturated, unbranched C₁₋₁₅ alkyl chain.
12. The conjugate according to clause 11 wherein the further linker comprises a saturated, unbranched C₁₋₆ alkyl chain.
13. The conjugate according to clause 12 wherein the further linker comprises a saturated, unbranched C₄ or C₆ alkyl chain, e.g. a C₄ alkyl chain.
14. The conjugate according to clause 1 wherein the first RNA strand is a compound of formula (I): 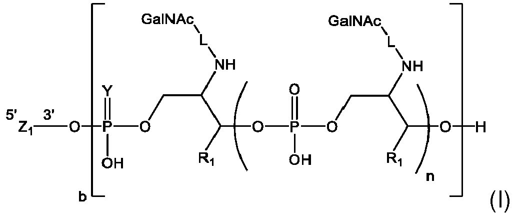 wherein b is 0 or 1; and
   the second RNA strand is a compound of formula (II): 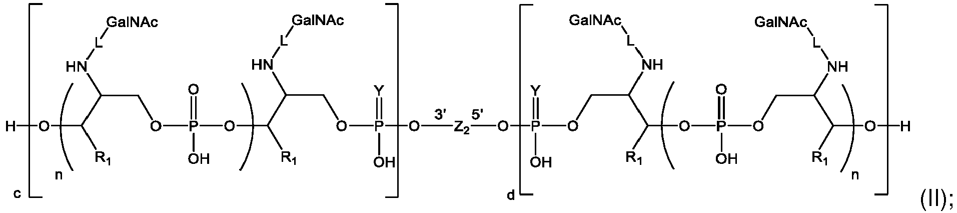 wherein c and d are independently 0 or 1;
   wherein:
   Z₁ and Z₂ are the RNA portions of the first and second RNA strands respectively;
   Y is O or S;
   R₁ is H or methyl;
   n is 0, 1, 2 or 3; and
   L is the same or different in formulae (I) and (II) and is selected from the group consisting of:

      -(CH₂)_{q},

      wherein q = 2-12;

      -(CH₂)ᵣC(O)-,

      wherein r = 2-12;

      -(CH₂-CH₂-O)ₛ-CH₂-C(O)-,

      wherein s = 1-5;

      -(CH₂)ₜ-CO-NH-(CH₂)ₜ-NH-C(O)-,

      wherein t is independently is 1-5;

      -(CH₂)ᵤ-CO-NH-(CH2)ᵤ-C(O)-,

      wherein u is independently is 1-5; and

      -(CH₂)ᵥ-NH-C(O)-,

      wherein v is 2-12; and
   wherein the terminal C(O) (if present) is attached to the NH group;
   and wherein b + c + d is 2 or 3.
15. The conjugate according to clause 14 wherein b is 0, c is 1 and d is 1.
16. The conjugate according to clause 14 wherein b is 1, c is 0 and d is 1.
17. The conjugate according to clause 14 wherein b is 1, c is 1 and d is 0.
18. The conjugate according to clause 14 wherein b is 1, c is 1 and d is 1.
19. The conjugate according to any one of clauses 14-18 wherein Y is O.
20. The conjugate according to any one of clauses 14-18 wherein Y is S.
21. The conjugate according to any one of clauses 14-20 wherein R₁ is H.
22. The conjugate according to any one of clauses 14-20 wherein R₁ is methyl.
23. The conjugate according to any one of clauses 14-22 wherein n is 0.
24. The conjugate according to any one of clauses 14-23 wherein L is -(CH₂)ᵣ-C(O)-, wherein r = 2-12.
25. The conjugate according to clause 24 wherein r = 2-6.
26. The conjugate according to clause 25 wherein r = 4 or 6 e.g. 4.
27. The conjugate according to any one of clauses 1-26 wherein the targeted cells are hepatocytes.
28. The conjugate according to any one of clauses 1-27 wherein the nucleic acid portion comprises two RNA strands of 15-30 ribonucleotides, suitably 20-25 ribonucleotides.
29. The conjugate according to any one of clauses 1-28 wherein the first RNA strand has one to six (e.g. one to three) phosphorothioate internucleotide linkages at each end.
30. The conjugate according to any one of clauses 1-29 wherein the second RNA strand has one to six (e.g. one to three) phosphorothioate internucleotide linkages at each end.
31. A method of making the conjugate, in any one of clauses 1-30, the method comprising adding together the components of the conjugate to form the conjugate.
32. A pharmaceutical composition comprising the conjugate according to any one of clauses 1-30 together with a pharmaceutically acceptable diluent or carrier.
33. The conjugate according to any one of clauses 1-30 or the pharmaceutical composition according to clause 32 for use in medicine.
34. The conjugate or composition, for use as in clause 33, wherein the use for treating liver disease, genetic disease, hemophilia and bleeding disorder, liver fibrosis, non alcoholic steatohepatitis (NASH), non alcoholic fatty liver disease (NAFLD), viral hepatitis, rare diseases (e.g. acromegaly), metabolic diseases (e.g. hypercholesterolemia, dyslipidemia, hypertriglyceridemia), cardiovascular diseases, obesity, hemochromatosis, thalassemia, liver injury, alcoholic liver diseases, alcohol dependence and/or anemia of chronic disease.
35. A method of inhibiting (in vitro or in vivo) the expression of a target gene in a mammalian cell, the method comprising contacting the mammalian cell with the conjugate as defined in any one of clauses 1 to 30, or a composition as in clause 32.
36. A method of inducing RNAi in a subject, the method comprising administering to the subject an effective amount of the conjugate as in any one of clauses 1 to 30, or a composition as in clause 32.
37. A method in clause 35 or clause 36 for use in the treatment of liver disease, genetic disease, hemophilia and bleeding disorder, liver fibrosis, non-alcoholic steatohepatitis (NASH), non-alcoholic fatty liver disease (NAFLD), viral hepatitis, rare diseases (e.g. acromegaly), metabolic diseases (e.g. hypercholesterolemia, dyslipidemia, hypertriglyceridemia), cardiovascular diseases, obesity, hemochromatosis, thalassemia, liver injury, alcoholic liver diseases, alcohol dependence and/or anemia of chronic disease in patient in need thereof.

### Statements to the invention

1. A conjugate for inhibiting expression of a target gene in a cell, said conjugate comprising a nucleic acid portion and ligand portions, said nucleic acid portion comprising at least one duplex region that comprises at least a portion of a first RNA strand and at least a portion of a second RNA strand that is at least partially complementary to the first strand, wherein said first strand is at least partially complementary to at least a portion of RNA transcribed from said target gene, said ligand portions comprising a serinol-derived linker moiety and a targeting ligand for in vivo targeting of cells and being conjugated exclusively to the 3' and/or 5' ends of one or both RNA strands, wherein the 5' end of the first RNA strand is not conjugated, wherein:
   (i) the second RNA strand is conjugated at the 5' end to the targeting ligand, and wherein (a) the second RNA strand is also conjugated at the 3' end to the targeting ligand and the 3' end of the first RNA strand is not conjugated; or (b) the first RNA strand is conjugated at the 3' end to the targeting ligand and the 3' end of the second RNA strand is not conjugated; or (c) both the second RNA strand and the first RNA strand are also conjugated at the 3' ends to the targeting ligand; or
   (ii) both the second RNA strand and the first RNA strand are conjugated at the 3' ends to the targeting ligand and the 5' end of the second RNA strand is not conjugated; and
   (iii) wherein said first strand includes modified nucleotides at a plurality of positions, and wherein the nucleotides at positions 2 and 14 from the 5' end of the first strand are not modified with a 2' O-methyl modification.
2. A nucleic acid of statement 1 wherein the nucleotides at positions 2 and 14 from the 5' end of the first strand are modified.
3 A nucleic acid according to statement 2, wherein the nucleotides at positions 2 and 14 from the 5' end of the first strand are not modified with a 2' O-methyl modification, and the nucleotide on the second strand which corresponds to position 13 of the first strand is not modified with a 2' O-methyl modification.
4 A nucleic acid according to statement 2, wherein the nucleotides at positions 2 and 14 from the 5' end of the first strand are not modified with a 2' O-methyl modification, and the nucleotide on the second strand which corresponds to position 11 of the first strand is not modified with a 2' O-methyl modification.
5 A nucleic acid according to statement 2 -4 wherein the nucleotides at positions 2 and 14 from the 5' end of the first strand are not modified with a 2' O-methyl modification, and the nucleotides on the second strand which corresponds to position 11 and 13 of the first strand are not modified with a 2' O-methyl modification.
6 A nucleic acid of any preceding statement wherein the nucleotides on the second strand corresponding to positions 11 and/or 13 from the 5' end of the first strand are modified.
7 A nucleic acid according to statements 2-6, wherein the nucleotides at positions 2 and 14 from the 5' end of the first strand are not modified with a 2' O-methyl modification, and the nucleotides on the second strand which correspond to position 11, or 13, or 11 and 13, or 11-13 of the first strand are modified with a 2' fluoro modification.
8 A nucleic acid according to any one of statements 2-7, wherein the nucleotides at positions 2 and 14 from the 5' end of the first strand are modified with a 2' fluoro modification, and the nucleotides on the second strand which correspond to position 11, or 13, or 11 and 13, or 11-13 of the first strand are not modified with a 2' O-methyl modification.
9 A nucleic acid according to any of statements 2-8 wherein the nucleotides at positions 2 and 14 from the 5' end of the first strand are modified with a 2' fluoro modification, and the nucleotides on the second strand which correspond to position 11, or 13, or 11 and 13, or 11-13 of the first strand are modified with a 2' fluoro modification.
10 A nucleic acid according to any preceding statement wherein greater than 50% of the nucleotides of the first and/or second strand comprise a 2' O-methyl modification, such as greater than 55%, 60%, 65%, 70%, 75%, 80%, or 85%, or more, of the first and/or second strand comprise a 2' O-methyl modification, preferably measured as a percentage of the total nucleotides of both the first and second strands.
11 A nucleic acid according to any preceding statement comprising no more than 20%, (such as no more than 15% or no more than 10%) of 2' fluoro modifications on the first and/or second strand, as a percentage of the total nucleotides of both strands.
12 A nucleic acid according to any preceding statement wherein the terminal nucleotide at the 3' end of at least one of the first strand and the second strand is an inverted nucleotide and is attached to the adjacent nucleotide via the 3' carbon of the terminal nucleotide and the 3' carbon of the adjacent nucleotide and/ or the terminal nucleotide at the 5' end of at least one of the first strand and the second strand is an inverted nucleotide and is attached to the adjacent nucleotide via the 5' carbon of the terminal nucleotide and the 5' carbon of the adjacent nucleotide, or wherein the nucleic acid comprises a phosphorodithioate linkage.
13. A composition comprising a conjugated nucleic acid of any of statements 1 - 12 and a physiologically acceptable excipient.
14. A nucleic acid or conjugated nucleic acid according to any of statements 1 to 13 or a composition of statement 14 for use in the treatment of a disease or disorder.
15. Use of a nucleic acid or conjugated nucleic acid according to any of statements 1 to 13 or a composition of statement 14 in the manufacture of a medicament for treating a disease or disorder.
16. A method of treating a disease or disorder comprising administration of a composition comprising a nucleic acid or conjugated nucleic acid according to any of statements 1 to 14 to an individual in need of treatment.

## Claims

1. A conjugate for inhibiting expression of a target gene in a cell, said conjugate comprising a nucleic acid portion and ligand portions, said nucleic acid portion comprising at least one duplex region that comprises at least a portion of a first RNA strand and at least a portion of a second RNA strand that is at least partially complementary to the first strand, wherein said first strand is at least partially complementary to at least a portion of RNA transcribed from said target gene, said ligand portions comprising a serinol-derived linker moiety and a targeting ligand for in vivo targeting of cells and being conjugated exclusively to the 3' and/or 5' ends of one or both RNA strands, wherein the 5' end of the first RNA strand is not conjugated, wherein:
(i) the second RNA strand is conjugated at the 5' end to the targeting ligand, and wherein (a) the second RNA strand is also conjugated at the 3' end to the targeting ligand and the 3' end of the first RNA strand is not conjugated; or (b) the first RNA strand is conjugated at the 3' end to the targeting ligand and the 3' end of the second RNA strand is not conjugated; or (c) both the second RNA strand and the first RNA strand are also conjugated at the 3' ends to the targeting ligand; or
(ii) both the second RNA strand and the first RNA strand are conjugated at the 3' ends to the targeting ligand and the 5' end of the second RNA strand is not conjugated; and
(iii) wherein said first strand includes modified nucleotides at a plurality of positions, and wherein the nucleotides at positions 2 and 14 from the 5' end of the first strand are not modified with a 2' O-methyl modification.

2. A nucleic acid of statement 1 wherein the nucleotides at positions 2 and 14 from the 5' end of the first strand are modified.

3. A nucleic acid according to statement 2, wherein the nucleotides at positions 2 and 14 from the 5' end of the first strand are not modified with a 2' O-methyl modification, and the nucleotide on the second strand which corresponds to position 13 of the first strand is not modified with a 2' O-methyl modification.

4. A nucleic acid according to statement 2, wherein the nucleotides at positions 2 and 14 from the 5' end of the first strand are not modified with a 2' O-methyl modification, and the nucleotide on the second strand which corresponds to position 11 of the first strand is not modified with a 2' O-methyl modification.

5. A nucleic acid according to statement 2 -4 wherein the nucleotides at positions 2 and 14 from the 5' end of the first strand are not modified with a 2' O-methyl modification, and the nucleotides on the second strand which corresponds to position 11 and 13 of the first strand are not modified with a 2' O-methyl modification.

6. A nucleic acid of any preceding statement wherein the nucleotides on the second strand corresponding to positions 11 and/or 13 from the 5' end of the first strand are modified.

7. A nucleic acid according to statements 2-6, wherein the nucleotides at positions 2 and 14 from the 5' end of the first strand are not modified with a 2' O-methyl modification, and the nucleotides on the second strand which correspond to position 11, or 13, or 11 and 13, or 11-13 of the first strand are modified with a 2' fluoro modification.

8. A nucleic acid according to any one of statements 2-7, wherein the nucleotides at positions 2 and 14 from the 5' end of the first strand are modified with a 2' fluoro modification, and the nucleotides on the second strand which correspond to position 11, or 13, or 11 and 13, or 11-13 of the first strand are not modified with a 2' O-methyl modification.

9. A nucleic acid according to any of statements 2-8 wherein the nucleotides at positions 2 and 14 from the 5' end of the first strand are modified with a 2' fluoro modification, and the nucleotides on the second strand which correspond to position 11, or 13, or 11 and 13, or 11-13 of the first strand are modified with a 2' fluoro modification.

10. A nucleic acid according to any preceding statement wherein greater than 50% of the nucleotides of the first and/or second strand comprise a 2' O-methyl modification, such as greater than 55%, 60%, 65%, 70%, 75%, 80%, or 85%, or more, of the first and/or second strand comprise a 2' O-methyl modification, preferably measured as a percentage of the total nucleotides of both the first and second strands.

11. A nucleic acid according to any preceding statement comprising no more than 20%, (such as no more than 15% or no more than 10%) of 2' fluoro modifications on the first and/or second strand, as a percentage of the total nucleotides of both strands.

12. A nucleic acid according to any preceding statement wherein the terminal nucleotide at the 3' end of at least one of the first strand and the second strand is an inverted nucleotide and is attached to the adjacent nucleotide via the 3' carbon of the terminal nucleotide and the 3' carbon of the adjacent nucleotide and/ or the terminal nucleotide at the 5' end of at least one of the first strand and the second strand is an inverted nucleotide and is attached to the adjacent nucleotide via the 5' carbon of the terminal nucleotide and the 5' carbon of the adjacent nucleotide, or wherein the nucleic acid comprises a phosphorodithioate linkage.

13. A composition comprising a conjugated nucleic acid of any of statements 1 - 12 and a physiologically acceptable excipient.

14. A nucleic acid or conjugated nucleic acid according to any of statements 1 to 13 or a composition of statement 14 for use in the treatment of a disease or disorder.

15. Use of a nucleic acid or conjugated nucleic acid according to any of statements 1 to 13 or a composition of statement 14 in the manufacture of a medicament for treating a disease or disorder.
